# EUROPEAN PATENT APPLICATION

(11) **EP 4 843 632 A2**
(43) Date of publication of application: **25.11.2026**
(21) Application number: 26207933.8
(22) Date of filing: 12.11.2019
(51) Int. Cl.: A61P 37/06

(54) **METHODS FOR INDUCING IMMUNE TOLERANCE**

(30) Priority: 12.11.2018 US 201862758785 P
(62) Document number(s) of the earlier application(s) in accordance with Art. 76 EPC: 19820919.9 / 3 880 174
(71) Applicant: Translate Bio, Inc., Waltham, MA 02451 (US)
(72) Inventor: COBAUGH, Christian, LEXINGTON, 02421 (US)
(74) Representative: Lavoix

(57) **Abstract**

The invention is based on the discovery that unmodified mRNA encapsulated in a liposome that is preferentially directed to the liver is particularly effective at inducing immune tolerance in a subject and avoids the need for co-administering an immune regulator (either separately or in form of an mRNA encoding the immune regulator). The invention therefore provides methods for inducing immune tolerance to one or more peptides, polypeptides or proteins in a subject in need thereof, wherein said method comprises administering to the subject one or more mRNAs, each mRNA comprising a 5'UTR, a coding region and a 3'UTR, wherein the one or more coding regions of the one or more mRNAs encode the one or more peptides, polypeptides or proteins, wherein said one or more mRNAs are encapsulated in one or more liposomes, wherein upon administration the one or more liposomes are preferentially delivered to the liver of the subject, wherein the nucleotides of the one or more mRNAs are unmodified.

## Description

### BACKGROUND

A hallmark of autoimmune diseases is the breakdown of the immune response recognition of "self". The lack of immune tolerance towards so-called "self-antigens" causes autoimmune diseases, such as rheumatoid arthritis, type 1 diabetes and multiple sclerosis (Keeler, G.D. (2017) Cellular Immunology, https://doi.org/10.1016/j.cellimm.2017.12.002). The human immune system produces both T cells and B cells that are reactive to self-antigens, but autoreactive T cells are usually selected against in the thymus and autoreactive B cells are typically kept in a state of anergy. This process of selecting against autoreactive T cells may involve regulatory T-cells (Tregs). In autoimmune diseases the self-reactive immune cells are not suppressed and attack the body, often causing irreparable damage as the disease progresses. For example, the destruction of β-cells in the pancreas in type I diabetes is caused by an autoimmune response to the β-cells by autoreactive CD4+ T helper cells and CD8+ T cells as well as autoantibody-producing B cells (Bluestone et al. (2010) Nature 464, 1293-1300). To prevent the destructive effects of an autoimmune response, or limit the damage wrought by it, it is desirable to re-establish immune tolerance to self-antigens. Over the last decade, a body of research has accumulated that suggests that induction of tolerance is indeed possible under the right set of circumstances.

Protein replacement therapy has been successfully employed to treat numerous diseases, including patients with type I diabetes. Many of the diseases requiring protein replacement therapy are due to genetic defects. Patients with a genetic defect in a protein-encoding gene may produce only defective versions of the encoded protein or not express the protein at all. As a consequence, their immune systems have not been trained to recognise the functional version of the protein as a self-antigen. When protein replacement therapy is initiated in these patients, they will mount an immune response against the replacement protein (Martino et al. (2009) PLoS One 4 (8) e6379). As a result, the immune system forms neutralising antibodies against the therapeutic protein, which blocks or inhibits its functionality. For example, the debilitating blood disorder haemophilia A is treated with intravenous Factor VIII replacement therapy. Approximately 30% of patients with severe haemophilia and 5% of patients with milder forms of the disease produce neutralising antibodies, termed "inhibitors", against the replacement Factor VIII, thereby blocking the protein's function (Sherman et al. (2017) Frontiers in Immunology 8, Art. 1604 and Reipert et al.(2006) British Journal of Haematology 136, 12-25). The replacement Factor VIII protein is seen as non-self-antigen by the immune system, which triggers a T cell and B cell mediated immune response.

Much of what has been learnt about the establishment of immune tolerance in recent years has come from observations in animal experiments that tested gene therapy vectors for protein replacement therapy. Attempts to treat haemophilia A and other protein deficiencies with gene therapy vectors has resulted in the surprising discovery that such treatments can induce immune tolerance to the replacement protein encoded by the viral vectors used for its delivery. A number of viral vectors have been shown to induce immune tolerance, including adenoviral, adeno-associated viral (AAV) and lentiviral vectors. A key component of therapeutic success of these viral vectors appears to be their ability to target expression of the replacement protein to the liver.

The liver is an integral part of the body's immune system. It manages a large amount of foreign antigens that reaches it *via* the blood from the digestive tract. The high volume of antigens leads to a cellular environment that favours tolerance over an immune response (LoDuca et al. (2009) Current Gene Therapy 9, 104-114). A unique balance exists in the liver between immunosuppressive and inflammatory responses to antigens, which has been termed the 'liver tolerance effect'. Gene therapy can exploit the tolerogenic nature of the liver to induce systemic immunological tolerance to transgene products. It has been demonstrated that hepatic gene transfer can achieve immune tolerance to an exogenous protein encoded by the viral vector by inducing Tregs that are specific to the exogenous protein (Sherman et al. (2017) Frontiers in Immunology 8, Art. 1604).

Tregs are known to play a crucial role in the induction and maintenance of immune tolerance. Tregs are a unique subset of CD4+ T cells which express Forkhead box P3 (FoxP3) and help maintain immune homeostasis. Tregs can control the immune response through a number of mechanisms including direct and indirect suppression of antigen presenting cells, B lymphocytes, and T effector cells. Tregs can help to prevent inflammatory damage to tissues and can suppress self-reactive T-cells (Arruda and Samelson-Jones (2016) Journal of Thrombosis and Haemostasis, 14: 1121-1134).

Hepatic gene transfer using viral vectors has been clinically tested with a number of diseases. However, the viral capsid of these gene therapy vectors are identical or nearly identical to the capsid of the wild-type virus. Therefore the human immune system produces neutralising antibodies against these vectors. The host's immune system is activated in a similar way as when challenged with a natural infection with a virus, which can reduce the cell transduction efficiency of viral vectors. For example, the T cell-mediated immune response to AAV occurs in a dose-dependent fashion and, above a certain threshold, the immune response leads to hepatotoxicity and loss of transgene expression (Colella et al. (2018) Molecular Therapy: Methods & Clinical Development 8, 87-104). In addition, viral vectors can integrate randomly into the genome of transfected cells, which may lead to both loss- and gain-of-function mutations that can alter cell functionality and homeostasis and in extreme cases can cause neoplasia.

There is therefore a need for new immune tolerance induction therapies. It has recently proposed that mRNA could be used as an alternative vector for inducing immune tolerance. mRNA itself is unstable when exposed to bodily fluids and has also been found to be immunogenic. It is widely published that nucleobase modifications enhance the properties of mRNA by reducing the immunogenicity and increasing the stability of the RNA molecule. For example, WO2018/189193 teaches that modified nucleotides, and modified uridine bases in particular, are required to make mRNA non-immunogenic. These modified bases are suggested to be needed in order to suppress RNA-mediated activation of innate immune receptors. The data presented in WO2018/189193 show that modification of mRNA with pseudouridine and, in particular 1-methylpseudouridine, is essential if mRNA is used as a vector for inducing immune tolerance.

It has also been suggested that immune modulators, such as cytokines, are essential to provide the cellular microenvironment that is required to achieve immune tolerance to a peptide, polypeptide or protein. For example, plasmid DNA has been used as an alternative vector in immune tolerance therapy. For example, WO2018/083111 describes experiments with DNA plasmids that encoded the antigen of interest along with the cytokines TGF-β and IL-10. WO2016/036902 teaches that mRNA-based compositions for inducing immune tolerance should comprise phosphatidylserine. Phosphatidylserine has been suggested to inhibit the expression of MHC and other molecules associated with the maturation of dendritic cells, to prevent the secretion of IL-12p70 by these cells, and consequently block their ability to activate CD4 and CD8 T cells.

Going against this emerging paradigm, the inventors disclose herein that an mRNA encoding a peptide, polypeptide or protein which has been prepared with unmodified nucleotides can be used on its own to induce tolerance to the encoded peptide, polypeptide or protein. Specifically, the delivery of an unmodified mRNA in $s that preferentially target the liver is sufficient to induce antigen-specific immunologic tolerance to the encoded peptide, polypeptide or protein, without a requirement for any additional immune modulators such as cytokines or phosphatidylserine. This may be achieved at least in part because expression of the peptide, polypeptide or protein for which immune tolerance is desired is by and large restricted to hepatocytes and liver sinusoidal endothelial cells when using the liposomal mRNA composition of the invention. Liver sinusoidal endothelial cells in particular appear to be an important component in the induction of immune tolerance.

### SUMMARY OF THE INVENTION

The present invention provides, among other things, methods and compositions for use inducing immune tolerance in a subject.

In one embodiment, the present invention provides a method for inducing immune tolerance to one or more peptides, polypeptides or proteins in a subject in need thereof, wherein said method comprises administering to the subject one or more mRNAs, each mRNA comprising a 5'UTR, a coding region and a 3'UTR, wherein the one or more coding regions of the one or more mRNAs encode the one or more peptides, polypeptides or proteins, wherein said one or more mRNAs are encapsulated in one or more liposomes, wherein upon administration the one or more liposomes are preferentially delivered to the liver of the subject, and wherein the nucleotides of the one or more mRNAs are unmodified.

The present invention also provides an mRNA comprising a 5'UTR, a coding region and a 3'UTR, wherein the coding region of the mRNA encodes a peptide, polypeptide or protein, for use in inducing immune tolerance to the peptide, polypeptide or protein in a subject in need thereof, wherein the mRNA is encapsulate in a liposome, wherein the liposome is preferentially delivered to the liver of the subject and wherein the nucleotides of the mRNAs are unmodified.

In some embodiments, the one or more mRNAs encoding the one or more peptides, polypeptides or proteins are the only therapeutic agents for inducing immune tolerance that are administered to the subject.

In some embodiments, an mRNA in accordance with the invention comprises a nucleic acid sequence that prevents its expression and/or induces its degradation in a haematopoietic cell. The haematopoietic cell may be an antigen-presenting cell. In some embodiments, the nucleic acid sequence is in the 3' UTR of the mRNA. In some embodiments, the nucleic acid sequence comprises one or more binding sites for miR-142-3p and/or miR-142-5p.

In some embodiments, the methods of the invention do not involve the administration of an immune regulator. In a specific embodiment, the compositions of the invention do not include an immune regulator. The immune regulator may be a cytokine or phosphatidylserine.

In some embodiments, a liposome in accordance with the invention comprises one or more cationic lipids, one or more non-cationic lipids, one or more cholesterol-based lipids and one or more PEG-modified lipids. In some embodiments, the one or more cationic lipids are selected from the group consisting of DOTAP (1,2-dioleyl-3-trimethylammonium propane), DODAP (1,2-dioleyl-3-dimethylammonium propane) , DOTMA (1,2-di-0-octadecenyl-3-trimethylammonium propane), DLinKC2DMA, DLin-KC2-DM, C12-200, cKK-E12 (3,6-bi s(4-(bis(2-hydroxydodecyl)amino)butyl)piperazine-2, 5 -dione), HGT5000, HGT5001 , HGT4003, ICE, OF-02 and combinations thereof. In some embodiments, the one or more non-cationic lipids are selected from DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine), DOPG (1,2-dioleoyl-*sn*-glycero-3-phospho-(1'-*rac*-glycerol)) or combinations thereof. In some embodiments, the one or more cholesterol-based lipids are cholesterol or PEGylated cholesterol. In some embodiments, the one or more PEG-modified lipids comprise a poly(ethylene) glycol chain of up to 5 kDa in length covalently attached to a lipid with alkyl chain(s) of C₆-C₂₀ length.

In some embodiments, the cationic lipid constitutes about 30%, 40 %, 50%, or 60% of the liposome by molar ratio. In some embodiments, the ratio of cationic lipids:non-cationic lipids:cholesterol lipids:PEGylated lipids is approximately 40:30:20:10 by molar ratio. In some embodiments, the ratio of cationic lipids:non-cationic lipids:cholesterol lipids:PEGylated lipids is approximately 40:30:25:5 by molar ratio. In some embodiments, the ratio of cationic lipids:non-cationic lipids:cholesterol lipids:PEGylated lipids is approximately 40:32:25:3 by molar ratio. In some embodiments, the ratio of cationic lipids:non-cationic lipids:cholesterol lipids:PEGylated lipids is approximately 50:25:20:5 by molar ratio.

In some embodiments, a liposome in accordance with the invention comprises cKK-E12, C12-200, HGT4003, HGT5001, HGT5000, DLinKC2DMA, DODAP or DODMA as the cationic lipid, DOPE as the non-cationic lipid, cholesterol as the neutral lipid, and DMG-PEG2K as the PEG-modified lipid. In some embodiments, a liposome in accordance with the invention comprises cKK-E12, DOPE, cholesterol and DMG-PEG2K.

In some embodiments, a liposome in accordance with the invention comprises a cholesterol-derived cationic lipid, a non-cationic lipid, and a PEG-modified lipid. In some embodiments, a liposome in accordance with the invention comprises ICE, DOPE and DMG-PEG2K.

In some embodiments, liposomes in accordance with the invention have a size of about 80 nm to 100 nm, optionally wherein the liposome has a size of about 100 nm or less than 100 nm.

In some embodiments, the 5'UTR of an mRNA in accordance with the invention comprises a nucleic acid sequence for liver-specific expression. In some embodiments, the nucleic acid sequence for liver-specific expression is a sequence from the 5' UTR of FGA (Fibrinogen alpha chain) mRNA, complement factor 3 (C3) mRNA or cytochrome p4502E1 (CYP2E1) mRNA. In some embodiments, an mRNA in accordance with the invention does not comprise a binding site for a liver-specific miRNA. In some embodiments, a liver-specific miRNA is one or more of miR-122, miR-29, miR-33a/b, miR-34a, miR-92a, miR-92, miR-103, miR-107, miR-143, miR-335 and miR-483.

In some embodiments, a subject in need of inducing immune tolerance to one or more peptides, polypeptides or proteins suffers from an autoimmune response mounted against or triggered by the one or more peptides, polypeptides or proteins. In some embodiments, the one or more peptides, polypeptides or proteins are or are derived from a self-antigen listed in Table 1.

In some embodiments, a method for inducing immune tolerance to one or more peptides, polypeptides or proteins in accordance with the invention reduces the levels of autoreactive CD4+ T helper cells and/or CD8+ T cells specific for the one or more peptides, polypeptides or proteins. In some embodiments, a method for inducing immune tolerance to one or more peptides, polypeptides or proteins in accordance with the invention reduces the levels of B cells that produce autoantibodies specific for the one or more peptides, polypeptides or proteins. In some embodiments, a method for inducing immune tolerance to one or more peptides, polypeptides or proteins in accordance with the invention increases the levels of T regulatory cells (Tregs), in particular CD4+CD25+FOXP3+ Tregs, that are specific for the one or more peptides, polypeptides or proteins.

In some embodiments, the subject in need of inducing immune tolerance to one or more peptides, polypeptides or proteins suffers from an autoimmune disease selected from type I diabetes, celiac disease, multiple sclerosis, rheumatoid arthritis, systemic lupus erythematosus, primary biliary cirrhosis, myasthenia gravis, neuromyelitis optica, or Graves' disease. In a specific embodiment, the autoimmune disease is type I diabetes and the one or more peptides, polypeptides or proteins for which immune tolerance is induced in accordance with the invention are or are derived from proinsulin. In some embodiments, administering one or more mRNAs encoding the one or more peptides, polypeptides or proteins derived from proinsulin in accordance with the invention reduces and/or eliminates the autoimmune response to the subject's β-cells. In another specific embodiment, the autoimmune disease is celiac disease and the one or more peptides, polypeptides or proteins are or are derived from tTG or ACT1.

In other embodiments, the subject in need of inducing immune tolerance to one or more peptides, polypeptides or proteins suffers from a protein deficiency and the one or more peptides, polypeptides or proteins are or are derived from a replacement protein that is or will be administered to the subject to treat the protein deficiency. In some embodiments, the subject has been treated with and produces antibodies against the replacement protein. In some embodiments, the protein deficiency and the corresponding replacement protein are selected from Table 2. In some embodiments, the protein deficiency is selected from haemophilia A or B, a lysosomal storage disorder, a metabolic disorder and an α-antitrypsin deficiency. In some embodiments, the protein deficiency is a metabolic disorder. In some embodiments, the metabolic disorder and the corresponding replacement protein are selected from Table 3.

In another specific embodiments, the protein deficiency is haemophilia A and the one or more peptides, polypeptides or proteins for which immune tolerance is induced in accordance with the invention are or are derived from Factor VIII.

In other embodiments, the subject in need of inducing immune tolerance to one or more peptides, polypeptides or proteins suffers from an allergy triggered by the one or more peptides, polypeptides or proteins. In some embodiments, administering one or more mRNAs encoding the one or more peptides, polypeptides or proteins in accordance with the invention reduces or eliminates the subject's allergic response to the one or more peptides, polypeptides or proteins. In some embodiments, the one or more peptides, polypeptides or proteins for which immune tolerance is induced in accordance with the invention are or are derived from an allergen listed in Table 4.

### BRIEF DESCRIPTION OF THE DRAWING

The drawings are for illustration purposes only, not for limitation.

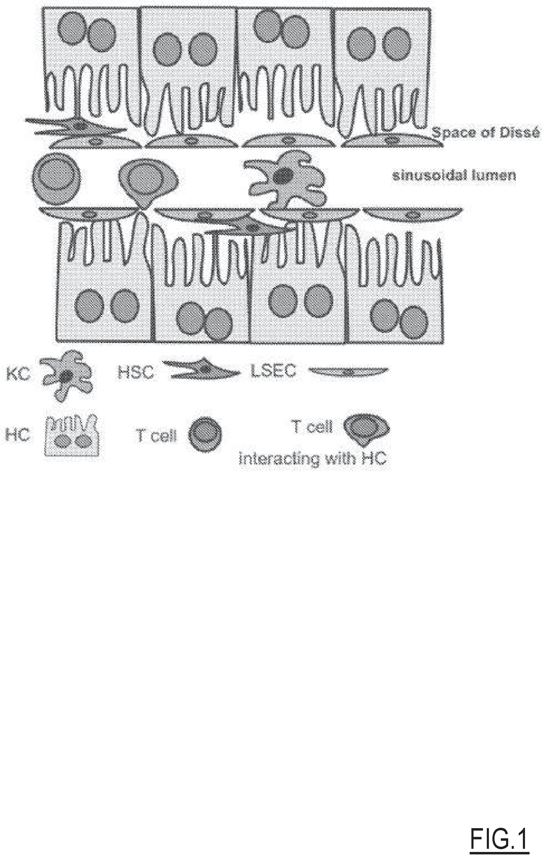

**Figure 1** is a schematic representation of the microanatomy of the liver sinusoids and their cellular composition (based on Figure 1 of Horst et al. (2016) Cellular & Molecular Immunology 13, 277-292). The cells shown include Kupffer cells (KCs), liver sinusoidal endothelial cells (LSECs), hepatic stellate cells (HSCs), and hepatic sinusoidal cell (HC).

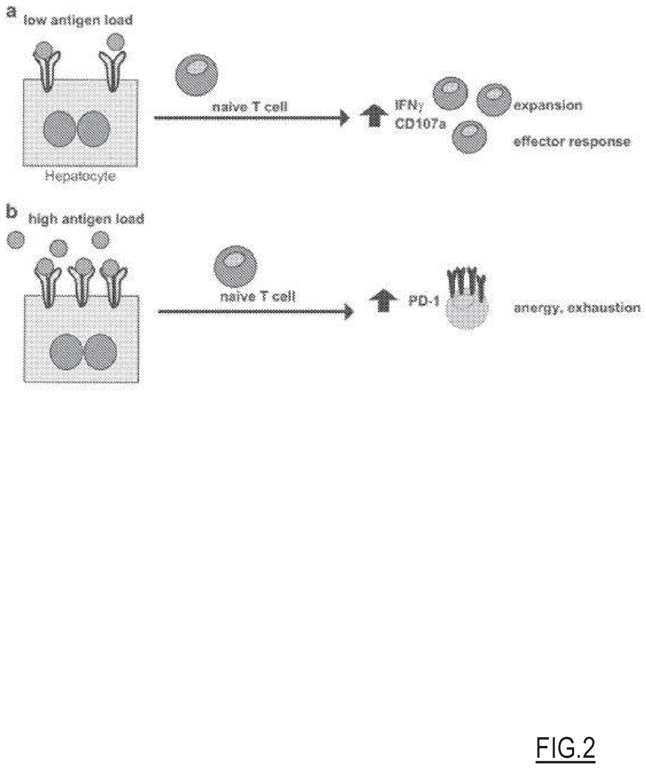

**Figure 2** shows liver-mediated T-cell priming and hepatocyte-T-cell interactions depend on antigen load (adapted from Horst et al. (2016) Cellular & Molecular Immunology 13, 277-292).

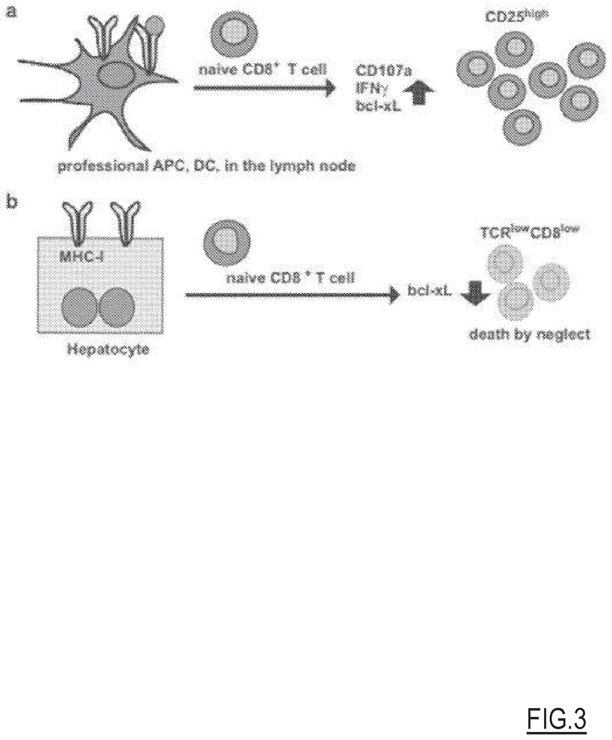

**Figure 3** shows differences in the outcome of T-cell priming between conventional professional antigen-presenting cells (APCs) of hematopoietic origin, such as dendritic cells (DC), in the lymph nodes and nonconventional APCs, such as hepatocytes in the liver (adapted from Horst et al. (2016) Cellular & Molecular Immunology 13, 277-292).

### DEFINITIONS

In order for the present invention to be more readily understood, certain terms are first defined below. Additional definitions for the following terms and other terms are set forth throughout the specification. The publications and other reference materials referenced herein to describe the background of the invention and to provide additional detail regarding its practice are hereby incorporated by reference.

Alkyl: As used herein, "alkyl" refers to a radical of a straight-chain or branched saturated hydrocarbon group having from 1 to 15 carbon atoms ("C1-15 alkyl"). In some embodiments, an alkyl group has 1 to 3 carbon atoms ("C1-3 alkyl"). Examples of C1-3 alkyl groups include methyl (C1), ethyl (C2), n-propyl (C3), and isopropyl (C3). In some embodiments, an alkyl group has 8 to 12 carbon atoms ("C8-12 alkyl"). Examples of C8-12 alkyl groups include, without limitation, n-octyl (C8), n-nonyl (C9), n-decyl (C10), n-undecyl (C11), n-dodecyl (C12) and the like. The prefix "n-" (normal) refers to unbranched alkyl groups. For example, n-C8 alkyl refers to (CH2)7CH3, n-C10 alkyl refers to (CH2)9CH3, etc.

Amino acid: As used herein, term "amino acid," in its broadest sense, refers to any compound and/or substance that can be incorporated into a polypeptide chain. In some embodiments, an amino acid has the general structure H2N-C(H)(R)-COOH. In some embodiments, an amino acid is a naturally occurring amino acid. In some embodiments, an amino acid is a synthetic amino acid; in some embodiments, an amino acid is a d-amino acid; in some embodiments, an amino acid is an l-amino acid. "Standard amino acid" refers to any of the twenty standard l-amino acids commonly found in naturally occurring peptides. "Nonstandard amino acid" refers to any amino acid, other than the standard amino acids, regardless of whether it is prepared synthetically or obtained from a natural source. As used herein, "synthetic amino acid" encompasses chemically modified amino acids, including but not limited to salts, amino acid derivatives (such as amides), and/or substitutions. Amino acids, including carboxy- and/or amino-terminal amino acids in peptides, can be modified by methylation, amidation, acetylation, protecting groups, and/or substitution with other chemical groups that can change the peptide's circulating half-life without adversely affecting their activity. Amino acids may participate in a disulfide bond. Amino acids may comprise one or posttranslational modifications, such as association with one or more chemical entities (e.g., methyl groups, acetate groups, acetyl groups, phosphate groups, formyl moieties, isoprenoid groups, sulfate groups, polyethylene glycol moieties, lipid moieties, carbohydrate moieties, biotin moieties, etc.). The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

Animal: As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (e.g., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically-engineered animal, and/or a clone.

Approximately or about: As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value). Typically, the term "approximately" or "about" refers to a range of values that within 10%, or more typically 1%, of the stated reference value.

Biologically active: As used herein, the phrase "biologically active" refers to a characteristic of any agent that has activity in a biological system, and particularly in an organism. For instance, an agent that, when administered to an organism, has a biological effect on that organism, is considered to be biologically active.

Codon-optimized: As used herein, the term describes a nucleic acid in which one or more of the nucleotides present in a naturally occurring nucleic acid sequence (also referred to as 'wild-type' sequence) has been substituted with an alternative nucleotide to optimize protein expression without changing the amino acid sequence of the polypeptide encoded by the naturally occurring nucleic acid sequence. For example, the codon AAA may be altered to become AAG without changing the identity of the encoded amino acid (lysine). In some embodiments, the nucleic acids of the invention are codon optimized to increase protein expression of the protein encoded by the nucleic acid.

Delivery: As used herein, the term "delivery" encompasses both local and systemic delivery. For example, delivery of mRNA encompasses situations in which an mRNA is delivered to a target tissue and the encoded protein is expressed and retained within the target tissue (also referred to as "local distribution" or "local delivery"), and situations in which an mRNA is delivered to a target tissue and the encoded protein is expressed and secreted into patient's circulation system (e.g., serum) and systematically distributed and taken up by other tissues (also referred to as "systemic distribution" or "systemic delivery).

Dosing interval: As used herein dosing interval in the context of a method for treating a disease is the frequency of administering a therapeutic composition in a subject (mammal) in need thereof, for example an mRNA composition, at an effective dose of the mRNA, such that one or more symptoms associated with the disease is reduced; or one or more biomarkers associated with the disease is reduced, at least for the period of the dosing interval. Dosing frequency and dosing interval may be used interchangeably in the current disclosure.

Expression: As used herein, "expression" of a nucleic acid sequence refers to translation of an mRNA into a polypeptide, assemble multiple polypeptides into an intact protein (e.g., enzyme) and/or post-translational modification of a polypeptide or fully assembled protein (e.g., enzyme). In this application, the terms "expression" and "production," and grammatical equivalent, are used inter-changeably.

Effective dose: As used herein, an effective dose is a dose of the mRNA in the pharmaceutical composition which when administered to the subject in need thereof, hereby a mammalian subject, according to the methods of the invention, is effective to bring about an expected outcome in the subject, for example reduce a symptom associated with the disease.

Functional: As used herein, a "functional" biological molecule is a biological molecule in a form in which it exhibits a property and/or activity by which it is characterized.

Half-life: As used herein, the term "half-life" is the time required for a quantity such as nucleic acid or protein concentration or activity to fall to half of its value as measured at the beginning of a time period.

Immune regulator: As used herein, the term "immune modulator" refers to a molecule that modulates the function of a cell of the immune system. The immune cell can be either a T-cell, such as a naive CD4+ cell, or a professional antigen-presenting cell of hematopoietic origin, such as a macrophage and/or a dendritic cell. Examples of an immune modulator in accordance with the present disclosure are cytokines that induce or enhance a Treg phenotype, such as TGF-beta (including the inactive latent form and the processed form), IL-27, IL-35 and/or IL37, IL-2, IL-10, IL-19, IL-20, IL-22, IL-24, IL-26, including any of the extended IL-10 superfamily; or phospholipids, such as phosphatidylserine. The presence of IL-10 and TGF-beta leads to an increase in expansion of Foxp3+ induced Tregs, which have enhanced CTLA-4 expression and suppressive capability that are comparable to that of natural Tregs. The synergistic effects of IL-2 and TGF-β can induce naive CD4+ cells to become CD25+Foxp3+ suppressor cells that express the characteristic markers of natural Treg cells. Another example of an immune modulator is a molecule that down-modulates the function of macrophages and/or dendritic cells. Suitable molecules with this function include phospholipids, in particular phosphatidylserine. Phosphatidylserine-liposomes have been shown to inhibit immune responses through down-modulation of macrophages and dendritic cells.

Improve, increase, or reduce: As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.

In Vitro: As used herein, the term "in vitro" refers to events that occur in an artificial environment, e.g., in a test tube or reaction vessel, in cell culture, etc., rather than within a multicellular organism.

In Vivo: As used herein, the term "in vivo" refers to events that occur within a multicellular organism, such as a human and a non-human animal. In the context of cell-based systems, the term may be used to refer to events that occur within a living cell (as opposed to, for example, in vitro systems).

Isolated: As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. Isolated substances and/or entities may be separated from about 10%, about 20%, about 30%, about 40%, about 50%, about 60%, about 70%, about 80%, about 90%, about 91%, about 92%, about 93%, about 94%, about 95%, about 96%, about 97%, about 98%, about 99%, or more than about 99% of the other components with which they were initially associated. In some embodiments, isolated agents are about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components. As used herein, calculation of percent purity of isolated substances and/or entities should not include excipients (e.g., buffer, solvent, water, etc.).

Local distribution or delivery: As used herein, the terms "local distribution," "local delivery," or grammatical equivalent, refer to tissue specific delivery or distribution. Typically, local distribution or delivery requires a protein (e.g., enzyme) encoded by mRNAs be translated and expressed intracellularly or with limited secretion that avoids entering the patient's circulation system.

messenger RNA (mRNA): As used herein, the term "messenger RNA (mRNA)" refers to a polyribonucleotide that encodes at least one polypeptide. mRNA may contain one or more coding and non-coding regions. mRNA can be purified from natural sources, produced using recombinant expression systems and optionally purified, in vitro transcribed, chemically synthesized, etc. An mRNA sequence is presented in the 5' to 3' direction unless otherwise indicated. Typically, the mRNA of the present invention is synthesized from adenosine, guanosine, cytidine and uridine nucleotides that bear no modifications. Such mRNA is referred to herein as mRNA with unmodified nucleotides or 'unmodified mRNA' for short. Typically, this means that the mRNA of the present invention does not comprise any of the following nucleoside analogs: 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine. An mRNA suitable for practising the claimed invention commonly does not comprise nucleosides comprising chemically modified bases; biologically modified bases (e.g., methylated bases); intercalated bases; modified sugars (e.g., 2'-fluororibose, ribose, 2'-deoxyribose, arabinose, and hexose); and/or modified phosphate groups (e.g., phosphorothioates and 5'-N-phosphoramidite linkages).

Nucleic acid: As used herein, the term "nucleic acid," in its broadest sense, refers to any compound and/or substance that is or can be incorporated into a polynucleotide chain. In some embodiments, a nucleic acid is a compound and/or substance that is or can be incorporated into a polynucleotide chain via a phosphodiester linkage. In some embodiments, "nucleic acid" refers to individual nucleic acid residues (e.g., nucleotides and/or nucleosides). In some embodiments, "nucleic acid" refers to a polynucleotide chain comprising individual nucleic acid residues. In some embodiments, "nucleic acid" encompasses RNA as well as single and/or double-stranded DNA and/or cDNA.

Patient: As used herein, the term "patient" or "subject" refers to any organism to which a provided composition may be administered, e.g., for experimental, diagnostic, prophylactic, cosmetic, and/or therapeutic purposes. Typical patients include animals (e.g., mammals such as mice, rats, rabbits, non-human primates, and/or humans). In some embodiments, a patient is a human. A human includes pre- and post-natal forms.

Pharmaceutically acceptable: The term "pharmaceutically acceptable" as used herein, refers to substances that, within the scope of sound medical judgment, are suitable for use in contact with the tissues of human beings and animals without excessive toxicity, irritation, allergic response, or other problem or complication, commensurate with a reasonable benefit/risk ratio.

Pharmaceutically acceptable salt: Pharmaceutically acceptable salts are well known in the art. For example, S. M. Berge et al., describes pharmaceutically acceptable salts in detail in J. Pharmaceutical Sciences (1977) 66:1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2- naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N+(C1-4 alkyl)4 salts. Representative alkali or alkaline earth metal salts include sodium, lithium, potassium, calcium, magnesium, and the like. Further pharmaceutically acceptable salts include, when appropriate, nontoxic ammonium. quaternary ammonium, and amine cations formed using counterions such as halide, hydroxide, carboxylate, sulfate, phosphate, nitrate, sulfonate and aryl sulfonate. Further pharmaceutically acceptable salts include salts formed from the quarternization of an amine using an appropriate electrophile, e.g., an alkyl halide, to form a quarternized alkylated amino salt.

Systemic distribution or delivery: As used herein, the terms "systemic distribution," "systemic delivery," or grammatical equivalent, refer to a delivery or distribution mechanism or approach that affect the entire body or an entire organism. Typically, systemic distribution or delivery is accomplished via body's circulation system, e.g., blood stream. Compared to the definition of "local distribution or delivery."

Subject: As used herein, the term "subject" refers to a human or any non-human animal (e.g., mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

Substantially: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

Target tissues: As used herein, the term "target tissues" refers to any tissue that is affected by a disease to be treated. In some embodiments, target tissues include those tissues that display disease-associated pathology, symptom, or feature.

Therapeutically effective amount: As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one unit dose.

Treating: As used herein, the term "treat," "treatment," or "treating" refers to any method used to partially or completely alleviate, ameliorate, relieve, inhibit, prevent, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease.

### DETAILED DESCRIPTION

### Therapeutic uses

The invention is based on the discovery that unmodified mRNA encapsulated in a liposome that is preferentially directed to the liver is particularly effective at inducing immune tolerance in a subject and avoids the need for co-administering an immune regulator (either separately or in form of an mRNA encoding the immune regulator).

The invention therefore provides methods for inducing immune tolerance to one or more peptides, polypeptides or proteins in a subject in need thereof, wherein said method comprises administering to the subject one or more mRNAs, each mRNA comprising a 5'UTR, a coding region and a 3'UTR, wherein the one or more coding regions of the one or more mRNAs encode the one or more peptides, polypeptides or proteins, wherein said one or more mRNAs are encapsulated in one or more liposomes, wherein upon administration the one or more liposomes are preferentially delivered to the liver of the subject, wherein the nucleotides of the one or more mRNAs are unmodified.

The present invention also provides one or more mRNAs, each mRNA comprising a 5'UTR, a coding region and a 3'UTR, wherein the one or more coding regions of the one or more mRNAs encode the one or more peptides, polypeptides or proteins, for use in a method of inducing immune tolerance to the peptide, polypeptide or protein in a subject in need thereof, wherein said one or more mRNAs are encapsulated in one or more liposomes, wherein upon administration the one or more liposomes are preferentially delivered to the liver of the subject, wherein the nucleotides of the one or more mRNAs are unmodified.

Establishing immune tolerance to a particular antigen, including self and foreign antigens is desirable for treating or preventing autoimmune diseases, combating inhibitors in protein replacement therapy and in treating allergies.

### Autoimmune disease

Autoimmune diseases are characterised by the dysregulation of the immune system to recognise self-antigens. The human immune system normally produces both T cells and B cells that are reactive with self-antigens, but these cells are usually inactivated by regulatory T-cells (Tregs) in healthy individuals. In contrast, in patients suffering from autoimmune diseases, these self-reactive immune cells are not inactivated and attack the body causing irreparable damage. For example, the destruction of β-cells in the pancreas in type I diabetes is caused by an autoimmune response to the β-cells by autoreactive CD4+ T helper cells and CD8+ T cells and autoantibody-producing B cells (Reipert et al.(2006) British Journal of Haematology 136, 12-25).

The inventors have discovered that one or more mRNAs encoding one or more peptides, polypeptides or proteins can be used to establish immune tolerance to the one or more peptides, polypeptides or proteins in a subject with an autoimmune disease mounted against or triggered by the one more peptides, polypeptides or proteins. This has been achieved *inter alia* by encapsulating the mRNA in a liposome that preferentially delivers the mRNA to the liver.

In certain embodiments, the invention provides a method for inducing immune tolerance to one or more peptides, polypeptides or proteins in a subject that suffers from an autoimmune response mounted against or triggered by the one or more peptides, polypeptides or proteins, wherein said method comprises administering to the subject one or more mRNAs encoding the one or more peptides, polypeptides or proteins. In other embodiments of the invention, one or more mRNAs encoding one or more peptides, polypeptides or proteins are provided for use in a method of inducing immune tolerance to one or more peptides, polypeptides or proteins in a subject suffering from an autoimmune response mounted against or triggered by the one or more peptides, polypeptides or proteins.

In some embodiments, the one or more peptides, polypeptides or proteins are or are derived from a self-antigen. In some embodiments, the one or more peptides, polypeptides or proteins are or are derived from a self-antigen listed in Table 1.

**Table 1 - Self-antigens that are involved in autoimmune disease**

| **Autoimmune disease** | **Self-antigen** |
|---|---|
| | Carboxypeptidase H |
| | Chromogranin A |
| | Glutamate decarboxylase |
| | Imogen-38 |
| | Insulin |
| Type I diabetes | Insulinoma antigen-2 and 2β |
| | Islet-specific glucose-6-phosphatase catalytic subunit related protein (IGRP) |
| | Proinsulin |
| | Islet cell autoantibodies |
| | 65Kda glutamic acid decarboxylase |
| | Phosphatase related IA-2 |
| Celiac disease | tissue transglutaminase (tTG) |
| | ACT1 |
| Multiple sclerosis | Kir1.4 |
| | α-enolase |
| | Aquaporin-4 |
| | β-arrestin |
| | Myelin basic protein |
| | Myelin oligodendrocytic glycoprotein |
| | Proteolipid protein |
| | S100-β |
| Rheumatoid arthritis | Citrullinated protein |
| | Collagen II |
| | Heat shock proteins |
| | Human cartilage glycoprotein 39 |
| Systemic lupus erythematosus | Double-stranded DNA |
| | La antigen |
| | Nucleosomal histones and ribonucleoproteins (snRNP) |
| | Phospholipid-β-2 glycoprotein I complex |
| | Poly(ADP-ribose) polymerase |
| | Sm antigens of U-1 small ribonucleoprotein complex |
| Primary biliary cirrhosis | pyruvate dehydrogenase E2 |
| | branched-chain ketoacid dehydrogenase |
| | dihydrolipoamide acetyltransferase (PDC-E2) |
| | dihydrolipoamide succinyltransferase (OGDC) |
| | dihydrolipoamide S-acetyltransferase |
| Myasthenia gravis | α-chain AChR |
| Neuromyelitisoptica | AQP4 |
| Graves' disease | TSHR |

In certain embodiments, the subject suffers from an autoimmune disease selected from type I diabetes, celiac disease, multiple sclerosis, rheumatoid arthritis, systemic lupus erythematosus, primary biliary cirrhosis, myasthenia gravis, neuromyelitis optica, or Graves' disease. In preferred embodiments, the subject suffers from type I diabetes.

In certain embodiments, the invention provides a method for inducing immune tolerance to one or more peptides, polypeptides or proteins in a subject, wherein the subject suffers from primary biliary cirrhosis and wherein the one or more peptides, polypeptides or proteins are or are derived from PDC E2/DLAT, BCKDC and/or OGDC. In certain embodiments, the invention provides a method for inducing immune tolerance to one or more peptides, polypeptides or proteins in a subject, wherein the subject suffers from myasthenia gravis and wherein the one or more peptides, polypeptides or proteins are or are derived from α-chain AChR. In certain embodiments, the invention provides a method for inducing immune tolerance to one or more peptides, polypeptides or proteins in a subject, wherein the subject suffers from neuromyelitis optica avis and wherein the one or more peptides, polypeptides or proteins are or are derived from AQP4. In certain embodiments, the invention provides a method for inducing immune tolerance to one or more peptides, polypeptides or proteins in a subject, wherein the subject suffers from multiple sclerosis and wherein the one or more peptides, polypeptides or proteins are or are derived from Kir1.4, MBP and/or MOG. In certain embodiments, the invention provides a method for inducing immune tolerance to one or more peptides, polypeptides or proteins in a subject, wherein the subject suffers from Graves' disease and wherein the one or more peptides, polypeptides or proteins are or are derived from TSHR.

In certain embodiments, a method for inducing immune tolerance to one or more peptides, polypeptides or proteins in accordance with the invention reduces the levels of autoreactive CD4+ T helper cells and/or CD8+ T cells specific for the one or more peptides, polypeptides or proteins. In certain embodiments, a method for inducing immune tolerance to one or more peptides, polypeptides or proteins in accordance with the invention reduces the levels of B cells that produce autoantibodies specific for the one or more peptides, polypeptides or proteins. In certain embodiments, a method for inducing immune tolerance to one or more peptides, polypeptides or proteins in accordance with the invention increases the levels of T regulatory cells (Tregs), in particular CD4+CD25+FOXP3+ Tregs, that are specific for the one or more peptides, polypeptides or proteins.

In certain embodiments, a method for inducing immune tolerance in accordance with the invention restores self-tolerance in a subject with an autoimmune disease. In certain embodiments, a method for inducing immune tolerance in accordance with the invention ameliorates the symptoms of the autoimmune disease. In certain embodiments, a method for inducing immune tolerance in accordance with the invention prevents the progression of an autoimmune disease in a subject. In certain embodiments, a method for inducing immune tolerance in accordance with the invention prevents a subject from developing the autoimmune disease.

The invention also provides compositions comprising mRNA encapsulated in a liposome for use in inducing immune tolerance to a self-antigen in a subject suffering from an autoimmune disease.

The invention also provides an mRNA encapsulated in a liposome for use in inducing immune tolerance in a subject suffering from an autoimmune response, wherein the autoimmune response is mounted against or triggered by a peptide, polypeptide or protein.

Often a plurality of autoantigens are associated with a single autoimmune disease. Therefore the invention provides methods for inducing immune tolerance in a subject suffering from an autoimmune disease, wherein the method comprises administering a plurality of mRNAs each mRNA encoding one or more of the plurality of self-antigens. In certain embodiments, the invention provides a method for inducing immune tolerance to two or more peptides, polypeptides or proteins in a subject in need thereof, wherein the method comprises administering to the subject one or more mRNAs encoding the two or more peptides, polypeptides or proteins.

### Type I diabetes

Type 1 diabetes is a disease that arises following the autoimmune destruction of insulin-producing pancreatic β cells. The disease is often diagnosed in children and adolescents and requires lifetime exogenous insulin replacement therapy. The symptoms of type 1 diabetes are polydipsia (excessive thirst), polyphagia (excessive eating), polyuria (frequent urination) and hyperglycemia. Patients generally present symptoms between the ages of 5-7 years old or at or near puberty (Atkinson (2012) Perspectives in Medicine 2:a007641).

In certain embodiments, the invention provides a method for inducing immune tolerance to one or more peptides, polypeptides or proteins in a subject, wherein the subject suffers from type I diabetes and wherein the one or more peptides, polypeptides or proteins are or are derived from a polypeptide or protein that is known to be involved in triggering type I diabetes. The present invention also provides one or more mRNAs for use in a method of inducing immune tolerance to one or more peptides, polypeptides or proteins in a subject, wherein the subject suffers from type I diabetes and wherein the one or more peptides, polypeptides or proteins are or are derived from a polypeptide or protein that is known to be involved in triggering type I diabetes. In a preferred embodiment, the one or more peptides, polypeptides or proteins are or are derived from proinsulin. Other proteins that are known to be involved in triggering type I diabetes include, but are not limited to, Carboxypeptidase H, Chromogranin A, Glutamate decarboxylase, Imogen-38, Insulin, Insulinoma antigen-2 and 2β, Islet-specific glucose-6-phosphatase catalytic subunit related protein (IGRP), Proinsulin, Islet cell autoantibodies, 65Kda glutamic acid decarboxylase and/or Phosphatase related IA-2.

In certain embodiments, the invention provides a method for inducing immune tolerance to one or more peptides, polypeptides or proteins in a subject, wherein the subject is suffering from early onset type I diabetes, wherein the method comprises administering to the subject one or more mRNAs, wherein the one or more mRNAs encode one or more peptides, polypeptides or proteins which are or are derived from a polypeptide or protein known to be involved in triggering type I diabetes (e.g., proinsulin). In certain embodiments, the invention provides a method for inducing immune tolerance to one or more peptides, polypeptides or proteins in a subject, wherein the subject is prediabetic, wherein the method comprises administering to the subject one or more mRNAs, wherein the one or more mRNAs encode one or more peptides, polypeptides or proteins which are or are derived from a polypeptide or protein known to be involved in triggering type I diabetes (e.g., proinsulin).

In certain embodiments, the methods of the invention treat or prevent type I diabetes in a subject in need thereof. In certain embodiments, the methods of the invention reduce and/or eliminate the autoimmune response to β-cells in the subject. In certain embodiments, the methods of the invention prevent the destruction of β-cells in the pancreas of the subject. In certain embodiments, the methods of the invention prevent the expansion of autoreactive T-cells in the subject. In certain embodiments, the methods of the invention reduce the levels of autoreactive CD4+ T helper cells and/or CD8+ T cells. In certain embodiments, the methods of the invention reduce the number of autoantibody-producing B cells. In certain embodiments, the methods of the invention increase the levels of autoantigen specific T regulatory cells (Tregs). In a specific embodiment, these Tregs are CD4+CD25+FOXP3+ Tregs.

In certain embodiments, the subject suffering from has functional β-cells before treatment. In certain embodiments, the subject has partially functioning β-cells before treatment. In certain embodiments, the subject has no functional β-cells before treatment. In certain embodiments, the subject does not require exogenous insulin replacement therapy before treatment. In certain embodiments, the subject requires exogenous insulin replacement therapy before treatment.

In some embodiments, the subject requires reduced levels of exogenous insulin replacement therapy after treatment. In other embodiments, the subject does not require exogenous insulin replacement therapy after treatment.

In certain embodiments, the subject is under 18 years old. In preferred embodiments, the subject between the ages of 5-7 years old. In certain embodiments, the subject is at or near puberty.

The invention also provides one or more mRNA encapsulated in a liposome, wherein the one or more mRNAs encode one or more peptides, polypeptides or proteins which are or are derived from a polypeptide or protein known to be involved in triggering type I diabetes (e.g., proinsulin) for use in inducing immune tolerance to the one or more peptides, polypeptides or proteins in a subject in need thereof. In certain embodiments, the liposome preferentially delivers the mRNA to the liver. In certain embodiments, the mRNA encodes proinsulin. In certain embodiments, the subject has type I diabetes. In certain embodiments, the subject has a genetic propensity to develop type I diabetes. In certain embodiments, the subject is prediabetic. In other embodiments, the subject has early onset type I diabetes.

### Celiac disease

Celiac disease is a serious hereditary autoimmune disorder that affects the small intestine. When patients with celiac disease eat gluten (a protein found in wheat, rye and barley), their body mounts an immune response that attacks the small intestine damaging the villi. This damage reduces the ability of the small intestine to absorb nutrients. In addition, it triggers an autoimmune response to tTG and/or ACT1.

There is a tendency for patients suffering with celiac disease to also suffer from other autoimmune diseases. For example, the association between celiac disease and type 1 diabetes is well established with around 4.5-11% of adult and paediatric patients suffering from both immune diseases (Denham and Hill (2013) Curr Allergy Asthma Rep 13, 347-353).

In certain embodiments, the invention provides a method for inducing immune tolerance to tTG and/or ACT1 in a subject suffering from celiac disease, wherein the method comprises administering to the subject one or more mRNAs, wherein one or more mRNAs encode one or more peptides, polypeptides or proteins which are or are derived from tTG and/or ACT1.

In certain embodiments, the invention provides a method for inducing immune tolerance to (i) tTG and/or and ACT1, and (ii) a polypeptide or protein known to be involved in triggering type I diabetes (e.g., proinsulin) in a subject, wherein the subject has celiac disease and type I diabetes, wherein the method comprises administering to the subject two or more mRNAs, where the first mRNA encodes one or more peptides, polypeptides or proteins which are or are derived from tTG and/or ACT1 and the second mRNA encodes one or more peptides, polypeptides or proteins which are or are derived from a polypeptide or protein known to be involved in triggering type I diabetes (e.g., proinsulin).

In certain embodiments, the subject is under 18 years old. In certain embodiments, the subject is over 18 years old.

### Protein replacement therapy

Protein replacement therapy is used to treat diseases where a particular protein is defective or absent in patient, typically due to a genetic defect in the gene encoding the protein. In some patients the administration of exogenous replacement protein can activate an immune response, resulting in the production of antibodies (also termed inhibitors) directed against the exogenous replacement protein. These inhibitors can block the protein function and prevent the therapy from being effective. Diseases that are treatable by protein replacement therapies include haemophilias A and B, lysosomal storage disorders, metabolic disorders, hepatitis and α- antitrypsin deficiency. A list of disease treatable by protein replacement therapies is provided in Table 2 below:

**Table 2 - Examples of protein replacement therapies for patients suffering from a protein deficiency**

| **Replacement protein** | **Protein deficiency** |
|---|---|
| Factor VIIa | Factor VII deficiency |
| Factor VIII | Hemophilia A |
| Factor IX | Hemophilia B |
| Factor X | Factor X deficiency |
| Factor XI | Factor XI deficiency |
| Factor XIII | Factor XIII deficiency |
| vWF | Von Willebrand disease |
| Protein C | Protein C deficiency |
| Antithrombin III | Antithrombin deficiency |
| Fibrinogen | Fibrinogen deficiency |
| C1-esterase inhibitor | Hereditary angioedema |
| α-1 proteinase inhibitor | α-PI deficiency |
| Glucocerebrosidase | Gaucher disease |
| α-L-iduronidase | Mucopolysaccharidosis I |
| Iduronate sulfatase | Mucopolysaccharidosis II |
| *N*-acetylgalactosamine-4-sulfatase | Mucopolysaccharidosis VI |
| *N*-acetylgalactosamine-6-sulfatase | Mucopolysaccharidosis IVA |
| Heparan sulfate sulfatase | Mucopolysaccharidosis IIIA |
| α-galactosidase A | Fabry disease |
| α-glucosidase | Pompe disease |
| Acid sphingomyelinase | Niemann-Pick type B disease |
| α-mannosidase | α-mannosidosis |
| Arylsulfatase A | Metachromatic leukodystrophy |
| Lysosomal acid lipase (LAL) | LAL deficiency |
| Sucrose-isomaltase | Sucrase-isomaltase deficiency |
| Adenosine deaminase (ADA) | ADA deficiency |
| Insulin-like growth factor 1 (IGF-1) | Primary IGF-1 deficiency |
| Alkaline phosphatase | Hypophosphatasia |
| Porphobilinogen deaminase | Acute intermittent porphyria |
| Phenylalanine ammonia lyase | Phenylketonuria |

Metabolic disorders can be treated with replacement exogenous enzyme. Examples of therapeutic enzymes that are used to treat metabolic disorders are summarised in the table below (Kang and Stevens (2009) Human mutation 30 (12) 1591-1610).

**Table 3 - Examples of exogenous replacement enzymes that can treat the protein deficiency in a subject**

| **Metabolic disorder** | **Replacement protein** |
|---|---|
| Gaucher | glucocerebrosidase |
| Fabry | a-galactosidase |
| Pompe | Acid a-glucosidase |
| Hurler and Hurler-Scheie forms of mucopolysaccharidosis I | a-L-iduronidase |
| Hunter Disease | Iduronate-2- sulfatase |
| Mucopolysaccharidosis VI | N-acetylgalactosamine4-sulfatase |
| Metachromatic leukodystrophy | Arylsulfatase A |
| Niemann-Pick | Acid sphingomyelinase |
| Hypophosphatasia | Tissue-nonspecific alkaline phosphatase fusion protein |
| Acute intermittent porphyria | Porphobilinogen deaminase |
| Phenylketonuria | Phenylalanine ammonia lyase |

The inventors realised that mRNA encoding a peptide, polypeptide or protein that is or is derived from therapeutic protein used in protein replacement therapy can be particularly helpful at restoring immune tolerance to the therapeutic protein, specifically in circumstances where the subject produces or is prone to produce antibodies against the therapeutic replacement protein. This has been achieved *inter alia* by encapsulating the mRNA in a liposome that preferentially delivers the mRNA to the liver.

In certain embodiments, the invention provides a method for inducing immune tolerance to one or more peptides, polypeptides or proteins in a subject, wherein the subject suffers from a protein deficiency and the one or more peptides, polypeptides or proteins are or are derived from a replacement protein that is or will be administered to the subject to treat the protein deficiency, wherein the method comprises administering to the subject one or more mRNAs, wherein the one or more mRNAs encode one or more peptides, polypeptides or proteins which are or are derived from the replacement protein. In certain embodiments, the subject has been treated with and produces antibodies against the replacement protein.

In certain embodiments, the protein deficiency is selected from haemophilia A or B, a lysosomal storage disorder, a metabolic disorder and an α-antitrypsin deficiency. In certain embodiments, the invention provides a method for inducing immune tolerance to one or more peptides, polypeptides or proteins in a subject, wherein the subject suffers from a lysosomal storage disorder and the one or more peptides, polypeptides or proteins are or are derived from a replacement protein that is or will be administered to the subject to treat the lysosomal storage disorder. In certain embodiments, the invention provides a method for inducing immune tolerance to one or more peptides, polypeptides or proteins in a subject, wherein the subject suffers from a metabolic disorder and the one or more peptides, polypeptides or proteins are or are derived from a replacement protein that is or will be administered to the subject to treat the metabolic disorder. In certain embodiments, the invention provides a method for inducing immune tolerance to one or more peptides, polypeptides or proteins in a subject, wherein the subject suffers from an α-antitrypsin deficiency and the one or more peptides, polypeptides or proteins are or are derived from a replacement protein that is or will be administered to the subject to treat an α-antitrypsin deficiency.

In certain embodiments, the replacement protein is an enzyme. In certain embodiments, the one or more mRNAs encode an enzyme. In certain embodiments, the enzyme is glucocerebrosidase, α-galactosidase, acid α-glucosidase, α-L-iduronidase, iduronate-2- sulfatase, N-acetylgalactosamine-4-sulfatase, arylsulfatase A, acid sphingomyelinase, tissue-nonspecific alkaline phosphatase fusion protein, porphobilinogen deaminase and phenylalanine ammonia lyase.

### Haemophilia

Haemophilia is a debilitating blood disorder that prevents the blood from clotting leading to severe bleeding. The major treatment for the disease is intravenous Factor VIII replacement therapy. Factor VIII is a glycoprotein which upon activation catalyses a critical step in the coagulation cascade. However, approximately 30% of patients with severe haemophilia and 5% of patients with milder forms of the disease produce neutralising antibodies, termed "inhibitors", against the replacement Factor VIII blocking the proteins function, reducing the protein's therapeutic capacity (Bluestone et al. (2010) Nature 464, 1293-1300 and Martino et al. (2009) PLoS One 4 (8) e6379).

Inhibitors are usually observed in young paediatric patients during the first 5 days of exposure to Factor VII, however inhibitors have also been reported patients over 50 years old. Inhibitor formation is callused by B-cell activation, which is dependent on CD4+ T helper cells. The current methodology to eliminate inhibitors is immune tolerance induction, which involves a high daily dose of Factor VIII (Bluestone et al. (2010) Nature 464, 1293-1300). However, these protocols take a long time (9-48 months) and can cause anaphylaxis and liver failure.

The inventors have surprisingly found that mRNA encoding Factor VIII can be effectively used to induce immune tolerance to Factor VIII. This has been achieved *inter alia* by encapsulating the mRNA in a liposome that preferentially delivers the mRNA to the liver.

Therefore, in certain embodiments, the invention provides a method for inducing immune tolerance to Factor VIII in a subject, wherein the subject suffers from haemophilia A and replacement Factor VIII is or will be administered to the subject to treat haemophilia A, wherein the method comprises administering to the subject an mRNA encoding a peptide, polypeptide or protein which is or is derived from Factor VIII. In certain embodiments, the subject has been treated with and produces antibodies against Factor VIII.

In other embodiments, the invention provides one or more mRNAs encoding one or more peptides, polypeptides or proteins which are or are derived from Factor VIII for use in a method inducing immune tolerance to Factor VIII in a subject suffering from haemophilia A.

In certain embodiments, the subject is concurrently receiving protein replacement therapy. In certain embodiments, the subject is under 18 years old. In other embodiments, the subject is over 50 years old.

### Allergies

Allergies are an increasing burden on healthcare system in the developed world. Food allergies affect 6% of adults and 8% if children and their prevalence is increasing. The only long-term curative treatment for food allergies is allergen-specific immunotherapy, which involves the administration of increasing doses of the causative allergen with the aim of inducing immune tolerance (Akdis and Akdis (2014) The Journal of Clinical Investigation 124 (11) 4678-4680). Examples of food allergies that can be treated in this way are peanut and sesame allergies. Allergen-specific immunotherapy induces peripheral T cell tolerance and promotes the formation of regulatory T-cells, including CD4+CD25+FOXP3+ Tregs.

The inventors have discovered that an mRNA encoding an allergen can be particularly helpful at restoring immune tolerance to the allergen and at reducing or eliminating allergy symptoms. This has been achieved *inter alia* by encapsulating the mRNA in a liposome that preferentially delivers the mRNA to the liver.

In certain embodiments, the invention provides a method for inducing immune tolerance to one or more peptides, polypeptides or proteins in a subject, wherein the subject suffers from an allergy triggered by the one or more peptides, polypeptides or proteins. In certain embodiments, the method reduces or eliminates the subject's allergic response to the one or more peptides, polypeptides or proteins.

The invention is broadly applicable to any type of allergy for which the peptide, polypeptide or protein that triggers the allergic reaction is known or can be identified. In certain embodiments, the one or more peptides, polypeptides or proteins are or are derived from food allergen. In certain embodiments, the food allergen can be derived from peanut, cow's milk, egg, wheat and other grains that contain gluten (for example barley, rye, and oats); hazelnut, soybean, fish, shellfish, sesame, or tree nuts (for example almonds, pine nuts, brazil nuts, walnuts and pecans).

In a specific embodiment, the invention provides a method for inducing immune tolerance to one or more peptides, polypeptides or proteins in a subject, wherein the subject suffers from a food allergy triggered by the one or more peptides, polypeptides or proteins, wherein the method comprises administering to the subject one or more mRNAs encoding one or more peptides, polypeptides or proteins encapsulated in one or more liposomes. In certain embodiments, the method reduces or eliminates the subject's allergic response to the one or more peptides, polypeptides or proteins.

Examples of known food allergens are provided in the Table 4. Therefore in certain embodiments, the one or more peptides, polypeptides or proteins are or are derived from an allergen listed in Table 4.

**Table 4 - A table of known plant and animal allergens. The systematic allergen nomenclature used is approved by the World Health Organisation and the International Union of Immunological Societies**

| **Plants** | **Peptide Allergen** | **Animals** | **Peptide Allergen** |
|---|---|---|---|
| *Triticum aestivum* (Wheat) | Tri a 12 | *Bos domesticus Bos taurus* (domestic cattle) | Bos d 2 |
| | Tri a 14 | | Bos d 3 |
| | Tri a 15 | | Bos d 4 |
| | Tri a 17 | | Bos d 5 |
| | Tri a 19 | | Bos d 6 |
| | Tri a 20 | | Bos d 7 |
| | Tri a 21 | | Bos d 8 |
| | Tri a 25 | | Bos d 9 |
| | Tri a 26 | | Bos d 10 |
| | Tri a 27 | | Bos d 11 |
| | Tri a 28 | | Bos d 12 |
| | Tri a 29 | *Gallus gallus domesticus* (Chicken) | Gal d 1 |
| | Tri a 30 | | Gal d 2 |
| | Tri a 31 | | Gal d 3 |
| | Tri a 32 | | Gal d 4 |
| | Tri a 33 | | Gal d 5 |
| | Tri a 34 | | Gal d 6 |
| | Tri a 35 | | Gal d 7 |
| | Tri a 36 | | Gal d 8 |
| | Tri a 37 | | Gal d 9 |
| | Tri a 39 | *Penaeus monodon* (Black Tiger Shrimp) | Pen m 1 |
| | Tri a 40 | | Pen m 2 |
| | Tri a 41 | | Pen m 3 |
| | Tri a 42 | | Pen m 4 |
| | Tri a 43 | | Pen m 6 |
| | Tri a 44 | *Artemia franciscana* (Brine shrimp) | Art fr 5 |
| | Tri a 45 | *Crangon crangon* (North Sea shrimp) | Cra c 1 |
| *Triticum turgidum ssp durum* (Durum Wheat) | Tri tu 14 | | Cra c 2 |
| *Hordeum vulgare* (Barley) | Hor v 5 | | Cra c 4 |
| | Hor v 12 | | Cra c 5 |
| | Hor v 15 | | Cra c 6 |
| | Hor v 16 | | Cra c 8 |
| | Hor v 17 | *Litopenaeus vannamei* (White shrimp) | Lit v 1 |
| | Hor v 20 | | Lit v 2 |
| *Secale cereal* (Rye) | Sec c 1 | | Lit v 3 |
| | Sec c 5 | | Lit v 4 |
| | Sec c 20 | *Macrobrachium rosenbergii* (giant freshwater prawn) | Mac r 1 |
| | Sec c 38 | *Melicertus latisulcatus* (King Prawn) | Mel I 1 |
| *Zea mays* (Maize) | Zea m 1 | *Metapenaeus ensis* (Shrimp) | Met e 1 |
| | Zea m 8 | *Pandalus borealis* (Northern shrimp) | Pan b 1 |
| | Zea m 12 | *Panulirus stimpsoni* (Spiny lobster) | Pan s 1 |
| | Zea m 14 | *Penaeus aztecus* (Brown shrimp | Pen a 1 |
| | Zea m 25 | *Penaeus indicus* (Shrimp) | Pen i 1 |
| *Glycine max* (Soybean) | Gly m 1 | *Pontastacus leptodactylus* (Narrow-clawed crayfish) | Pon 1 4 |
| | Gly m 2 | | Pon 1 7 |
| | Gly m 3 | *Crassostrea gigas* (Pacific Oyster) | Cra g 1 |
| | Gly m 4 | *Clupea harengus* (Atlantic herring) | Clu h 1 |
| | Gly m 5 | *Cyprinus carpio* (Common carp) | Cyp c 1 |
| | Gly m 6 | *Gadus callarias* (Baltic cod) | Gad c 1 |
| | Gly m 7 | *Gadus morhua* (Atlantic cod) | Gad m 1 |
| | Gly m 8 | | Gad m 2 |
| | Gly m Bd 30K | | Gad m 3 |
| *Sesamum indicum* (Sesame) | Ses i 1 | *Lepidorhombus whiffiagonis* (Turbot) | Lep w 1 |
| | Ses i 2 | *Oncorhynchus mykiss* (Rainbow trout) | Onc m 1 |
| | Ses i 3 | *Oreochromis mossambicus* (tilapia) | Ore m 4 |
| | Ses i 4 | *Salmo salar* (Atlantic salmon) | Sal s 1 |
| | Ses i 5 | | Sal s 2 |
| | Ses i 6 | | Sal s 3 |
| | Ses i 7 | *Sardinops sagax* (Pacific pilchard) | Sar sa 1 |
| *Arachis hypogaea* (Peanut) | Ara h 1 | *Sebastes marinus* (Ocean perch) | Seb m 1 |
| | Ara h 2 | *Thunnus albacares* (Yellowfin tuna) | Thu a 1 |
| | Ara h 3 | | Thu a 2 |
| | Ara h 5 | | Thu a 3 |
| | Ara h 6 | *Xiphias gladius* (Swordfish) | Xip g 1 |
| | Ara h 7 | | |
| | Ara h 8 | | |
| | Ara h 9 | | |
| | Ara h 10 | | |
| | Ara h 11 | | |
| | Ara h 12 | | |
| | Ara h 13 | | |
| | Ara h 14 | | |
| | Ara h 15 | | |
| | Ara h 16 | | |
| | Ara h 17 | | |
| *Corylus avellana* (Hazelnut) | Cor a 1 | | |
| | Cor a 2 | | |
| | Cor a 8 | | |
| | Cor a 6 | | |
| | Cor a 9 | | |
| | Cor a 10 | | |
| | Cor a 11 | | |
| | Cor a 12 | | |
| | Cor a 13 | | |
| | Cor a 14 | | |
| *Juglans regia* (Walnut) | Jug r 1 | | |
| | Jug r 2 | | |
| | Jug r 3 | | |
| | Jug r 4 | | |
| | Jug r 5 | | |
| | Jug r 6 | | |
| | Jug r 7 | | |
| | Jug r 8 | | |
| *Carya illinoinensis* (Pecan) | Car i 1 | | |
| | Car i 2 | | |
| | Car I 4 | | |
| *Prunus dulcis* (Almond) | Pru du 3 | | |
| | Pru du 4 | | |
| | Pru du 5 | | |
| | Pru du 6 | | |
| *Anacardium occidentale* (Cashew) | Ana o 1 | | |
| | Ana o 2 | | |
| | Ana o 3 | | |
| *Pistacia vera* (Pistachio) | Pis v 1 | | |
| | Pis v 2 | | |
| | Pis v 3 | | |
| | Pis v 4 | | |
| | Pis v 5 | | |
| *Bertholletia excelsa* (Brazil nut) | Ber e 1 | | |
| | Ber e 2 | | |

The invention is more broadly applicable to any type of allergy for which the peptide, polypeptide or protein that triggers the allergic reaction is known or can be identified. Therefore, the invention further provides a method for inducing immune tolerance to one or more peptides, polypeptides or proteins in a subject, wherein the subject suffers from an allergy triggered by the one or more peptides, polypeptides or proteins, wherein the method comprises administering to the subject one or more mRNAs encapsulated in one or more liposomes. In certain embodiments, the method reduces or eliminates the subject's allergic response to the one or more peptides, polypeptides or proteins.

### Liposomes

According to the present invention, the one or more mRNAs encode the one or more peptides, polypeptides or proteins are encapsulated in one or more liposomes. In some embodiments, mRNAs, each encoding a different peptide, polypeptide or protein, may be delivered in separate liposomes. In other embodiments, mRNAs, each encoding a different peptide, polypeptide or protein, may be delivered in a single liposome. Typically, all liposomes in a given formulation will have the same lipid composition. In some embodiments, all liposomes in a given formulation that encapsulate mRNAs that encode the same protein have the same lipid composition, but liposome that encapsulate mRNAs that encode a different protein may have different a different lipid composition

As used herein, liposomes are usually characterized as microscopic vesicles having an interior aqua space sequestered from an outer medium by a membrane of one or more bilayers. Bilayer membranes of liposomes are typically formed by amphiphilic molecules, such as lipids of synthetic or natural origin that comprise spatially separated hydrophilic and hydrophobic domains (Lasic, Trends Biotechnol., 16: 307-321, 1998). Bilayer membranes of the liposomes can also be formed by amphophilic polymers and surfactants (e.g., polymerosomes, niosomes, etc.). In the context of the present invention, a liposome typically serves to transport a desired mRNA to a target cell or tissue, typically the liver. A typical liposome in accordance with the invention comprises one or more cationic lipids, one or more non-cationic lipids, one or more cholesterol-based lipids and one or more PEG-modified lipids.

### Cationic Lipids

As used herein, the phrase "cationic lipids" refers to any of a number of lipid species that have a net positive charge at a selected pH, such as physiological pH.

Several cationic lipids have been described in the literature, many of which are commercially available. Suitable cationic lipids for use in the compositions and methods of the invention include the cationic lipids as described in International Patent Publication WO 2010/144740, which is incorporated herein by reference.

In certain embodiments, the compositions and methods of the present invention include a cationic lipid, (6Z,9Z,28Z,31Z)-heptatriaconta-6,9,28,31-tetraen-19-yl 4-(dimethylamino) butanoate, having a compound structure of: 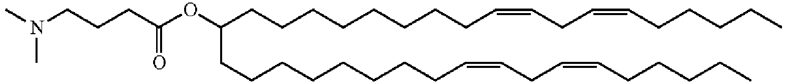 and pharmaceutically acceptable salts thereof.

Other suitable cationic lipids for use in the compositions and methods of the present invention include ionizable cationic lipids as described in International Patent Publication WO 2013/149140, which is incorporated herein by reference. In some embodiments, the compositions and methods of the present invention include a cationic lipid of one of the following formulas: 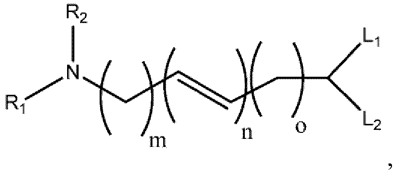 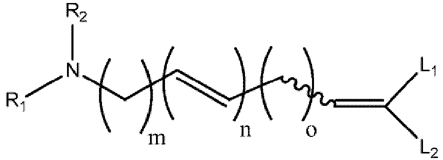 or a pharmaceutically acceptable salt thereof, wherein R₁ and R₂ are each independently selected from the group consisting of hydrogen, an optionally substituted, variably saturated or unsaturated C₁-C₂₀ alkyl and an optionally substituted, variably saturated or unsaturated C₆-C₂₀ acyl; wherein L₁ and L₂ are each independently selected from the group consisting of hydrogen, an optionally substituted C₁-C₃₀ alkyl, an optionally substituted variably unsaturated C₁-C₃₀ alkenyl, and an optionally substituted C₁-C₃₀ alkynyl; wherein m and o are each independently selected from the group consisting of zero and any positive integer (e.g., where m is three); and wherein n is zero or any positive integer (e.g., where n is one). In certain embodiments, the compositions and methods of the present invention include the cationic lipid (15Z, 18Z)-N,N-dimethyl-6-(9Z,12Z)-octadeca-9,12-dien-1-yl) tetracosa-15,18-dien-1-amine ("HGT5000"), having a compound structure of: 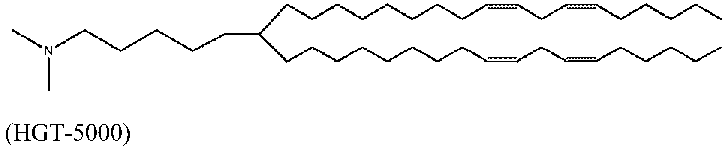 and pharmaceutically acceptable salts thereof. In certain embodiments, the compositions and methods of the present invention include the cationic lipid (15Z, 18Z)-N,N-dimethyl-6-((9Z,12Z)-octadeca-9,12-dien-1-yl) tetracosa-4,15,18-trien-1 -amine ("HGT5001"), having a compound structure of: 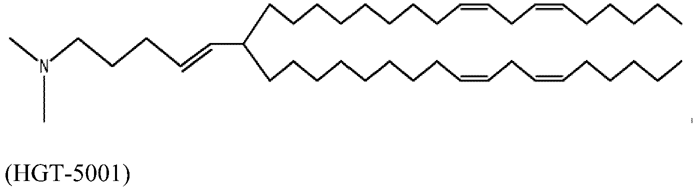 and pharmaceutically acceptable salts thereof. In certain embodiments, the compositions and methods of the present invention include the cationic lipid and (15Z,18Z)-N,N-dimethyl-6-((9Z,12Z)-octadeca-9,12-dien-1-yl) tetracosa-5,15,18-trien- 1 -amine ("HGT5002"), having a compound structure of: 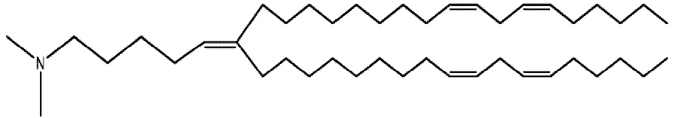 (HGT-5002) and pharmaceutically acceptable salts thereof.

Other suitable cationic lipids for use in the compositions and methods of the invention include cationic lipids described as aminoalcohol lipidoids in International Patent Publication WO 2010/053572, which is incorporated herein by reference. In certain embodiments, the compositions and methods of the present invention include a cationic lipid having a compound structure of: 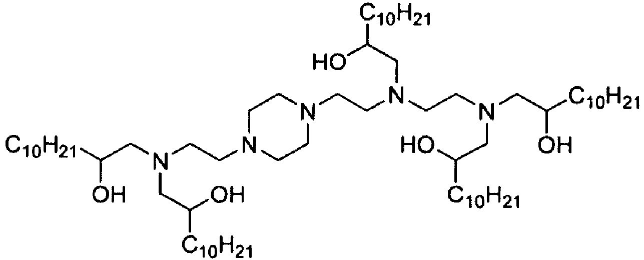 and pharmaceutically acceptable salts thereof.

Other suitable cationic lipids for use in the compositions and methods of the invention include the cationic lipids as described in International Patent Publication WO 2016/118725, which is incorporated herein by reference. In certain embodiments, the compositions and methods of the present invention include a cationic lipid having a compound structure of: 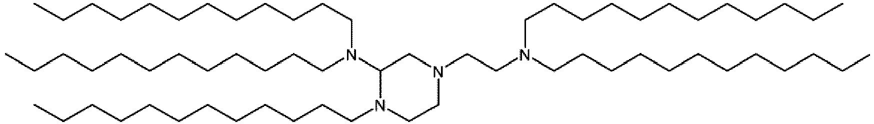 and pharmaceutically acceptable salts thereof.

Other suitable cationic lipids for use in the compositions and methods of the invention include the cationic lipids as described in International Patent Publication WO 2016/118724, which is incorporated herein by reference. In certain embodiments, the compositions and methods of the present invention include a cationic lipid having a compound structure of: 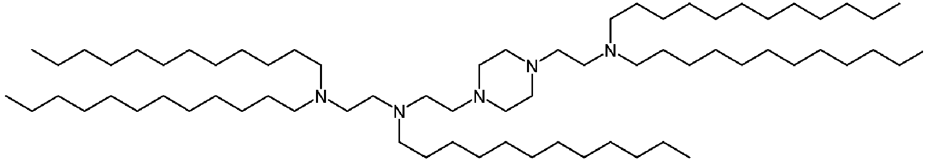 and pharmaceutically acceptable salts thereof.

Other suitable cationic lipids for use in the compositions and methods of the invention include a cationic lipid having the formula of 14,25-ditridecyl 15,18,21,24-tetraaza-octatriacontane, and pharmaceutically acceptable salts thereof.

Other suitable cationic lipids for use in the compositions and methods of the invention include the cationic lipids as described in International Patent Publications WO 2013/063468 and WO 2016/205691, each of which are incorporated herein by reference. In some embodiments, the compositions and methods of the present invention include a cationic lipid of the following formula: 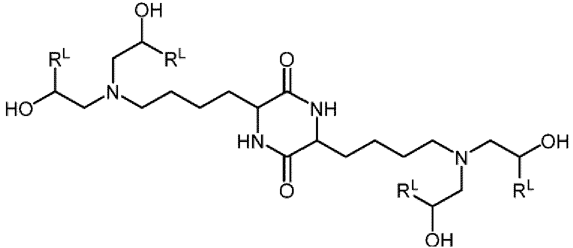 or pharmaceutically acceptable salts thereof, wherein each instance of R^{L} is independently optionally substituted C₆-C₄₀ alkenyl. In certain embodiments, the compositions and methods of the present invention include a cationic lipid having a compound structure of: 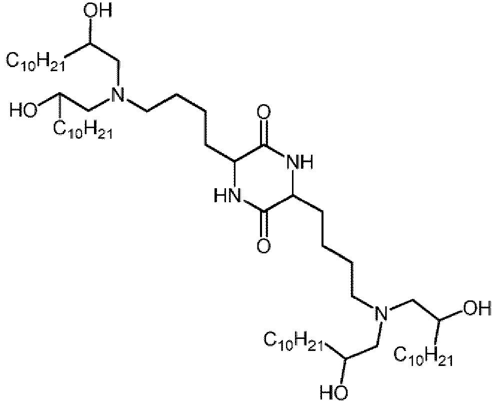 and pharmaceutically acceptable salts thereof. In certain embodiments, the compositions and methods of the present invention include a cationic lipid having a compound structure of: 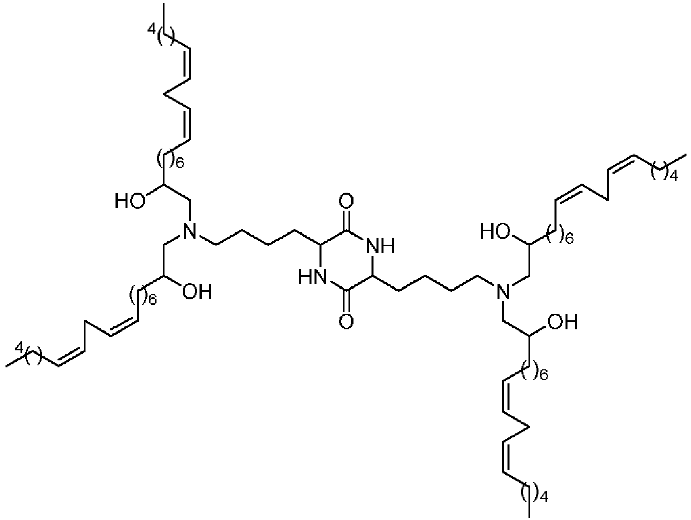 and pharmaceutically acceptable salts thereof. In certain embodiments, the compositions and methods of the present invention include a cationic lipid having a compound structure of: 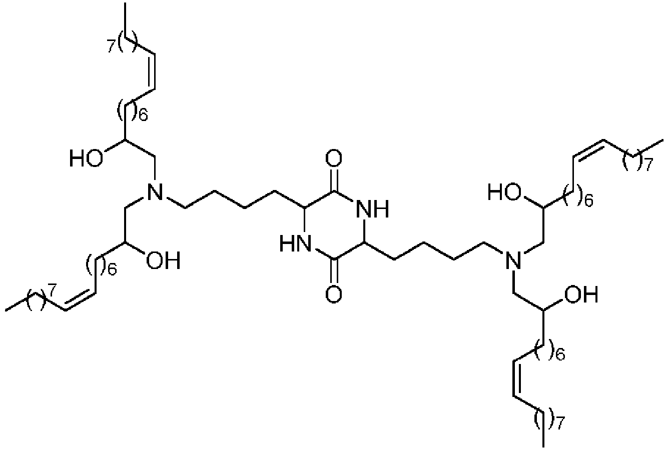 and pharmaceutically acceptable salts thereof. In certain embodiments, the compositions and methods of the present invention include a cationic lipid having a compound structure of: 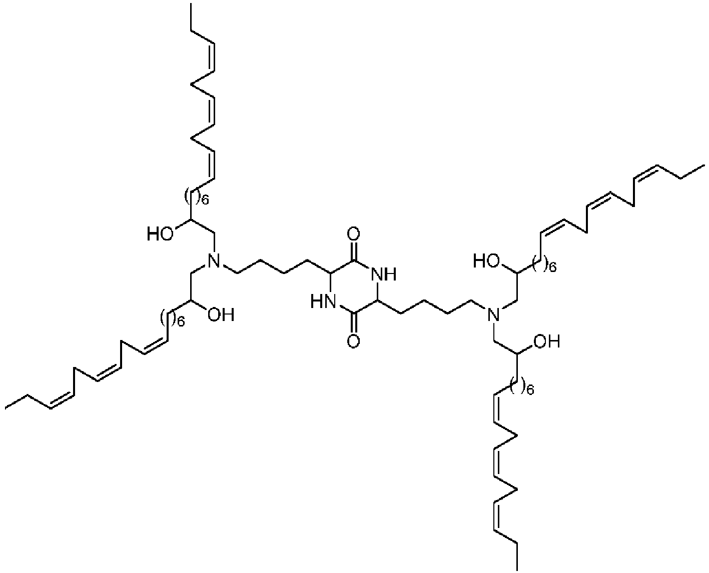 and pharmaceutically acceptable salts thereof.

Other suitable cationic lipids for use in the compositions and methods of the invention include the cationic lipids as described in International Patent Publication WO 2015/184256, which is incorporated herein by reference. In some embodiments, the compositions and methods of the present invention include a cationic lipid of the following formula: 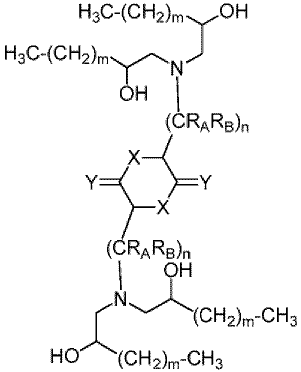 or a pharmaceutically acceptable salt thereof, wherein each X independently is O or S; each Y independently is O or S; each m independently is 0 to 20; each n independently is 1 to 6; each R_{A} is independently hydrogen, optionally substituted C1-50 alkyl, optionally substituted C2-50 alkenyl, optionally substituted C2-50 alkynyl, optionally substituted C3-10 carbocyclyl, optionally substituted 3-14 membered heterocyclyl, optionally substituted C6-14 aryl, optionally substituted 5-14 membered heteroaryl or halogen; and each R_{B} is independently hydrogen, optionally substituted C1-50 alkyl, optionally substituted C2-50 alkenyl, optionally substituted C2-50 alkynyl, optionally substituted C3-10 carbocyclyl, optionally substituted 3-14 membered heterocyclyl, optionally substituted C6-14 aryl, optionally substituted 5-14 membered heteroaryl or halogen. In certain embodiments, the compositions and methods of the present invention include a cationic lipid, "Target 23", having a compound structure of: 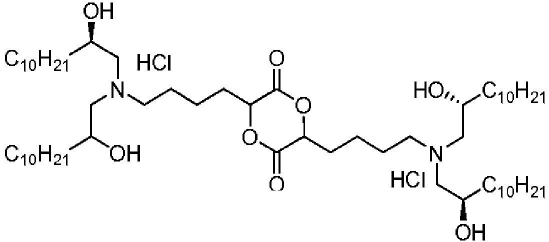 (Target 23) and pharmaceutically acceptable salts thereof.

Other suitable cationic lipids for use in the compositions and methods of the invention include the cationic lipids as described in International Patent Publication WO 2016/004202, which is incorporated herein by reference. In some embodiments, the compositions and methods of the present invention include a cationic lipid having the compound structure: 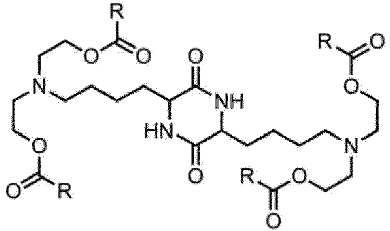 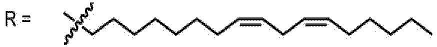 or a pharmaceutically acceptable salt thereof. In some embodiments, the compositions and methods of the present invention include a cationic lipid having the compound structure: 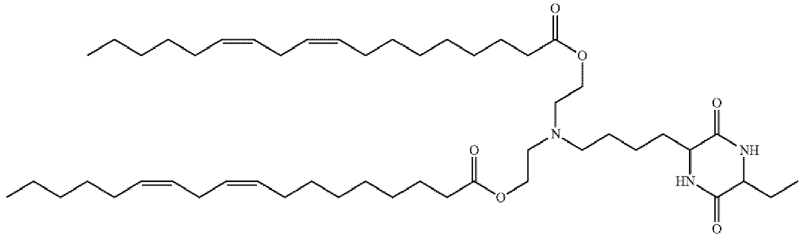 or a pharmaceutically acceptable salt thereof. In some embodiments, the compositions and methods of the present invention include a cationic lipid having the compound structure: 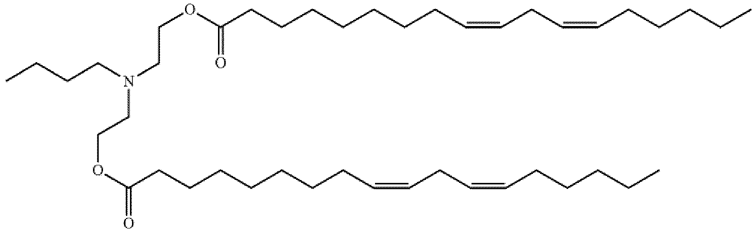 or a pharmaceutically acceptable salt thereof.

Other suitable cationic lipids for use in the compositions and methods of the present invention include the cationic lipids as described in J. McClellan, M. C. King, Cell 2010, 141, 210-217 and in Whitehead et al., Nature Communications (2014) 5:4277, which is incorporated herein by reference. In certain embodiments, the cationic lipids of the compositions and methods of the present invention include a cationic lipid having a compound structure of: 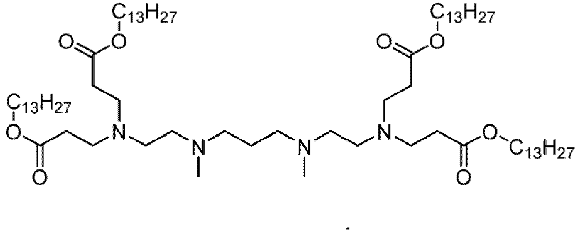 and pharmaceutically acceptable salts thereof.

Other suitable cationic lipids for use in the compositions and methods of the invention include the cationic lipids as described in International Patent Publication WO 2015/199952, which is incorporated herein by reference. In some embodiments, the compositions and methods of the present invention include a cationic lipid having the compound structure: 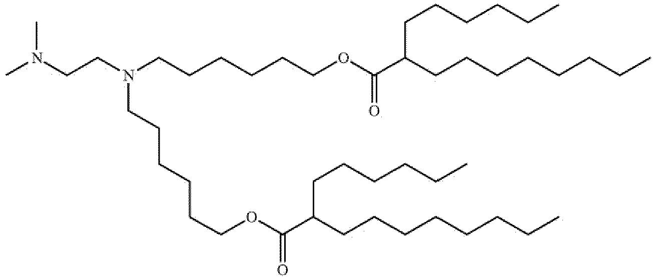 and pharmaceutically acceptable salts thereof. In some embodiments, the compositions and methods of the present invention include a cationic lipid having the compound structure: 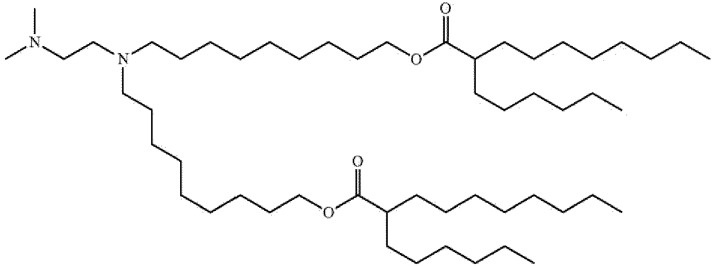 and pharmaceutically acceptable salts thereof. In some embodiments, the compositions and methods of the present invention include a cationic lipid having the compound structure: 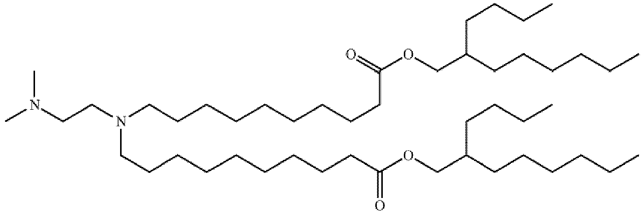 and pharmaceutically acceptable salts thereof. In some embodiments, the compositions and methods of the present invention include a cationic lipid having the compound structure: 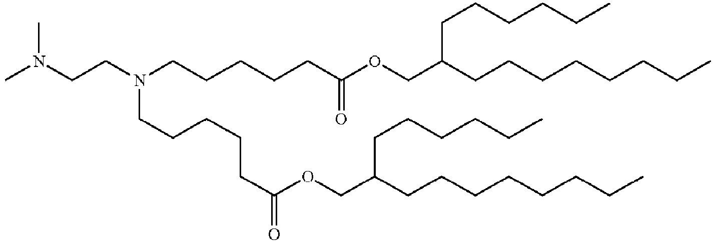 and pharmaceutically acceptable salts thereof. In some embodiments, the compositions and methods of the present invention include a cationic lipid having the compound structure: 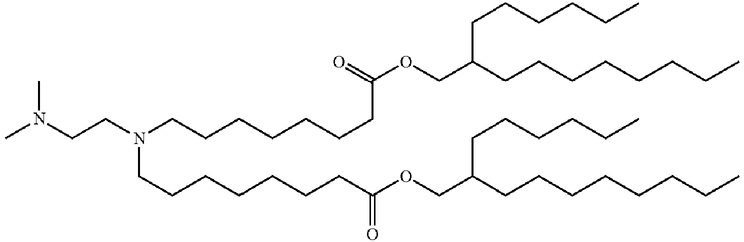 and pharmaceutically acceptable salts thereof. In some embodiments, the compositions and methods of the present invention include a cationic lipid having the compound structure: 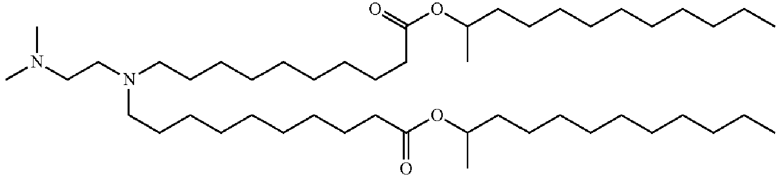 and pharmaceutically acceptable salts thereof. In some embodiments, the compositions and methods of the present invention include a cationic lipid having the compound structure: 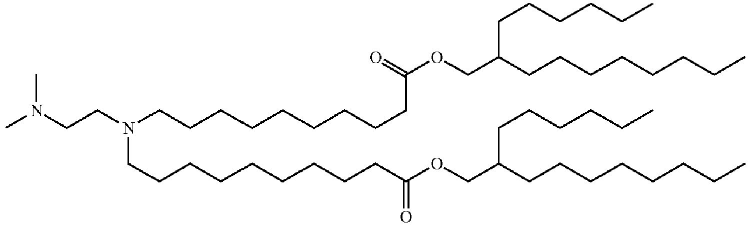 and pharmaceutically acceptable salts thereof. In some embodiments, the compositions and methods of the present invention include a cationic lipid having the compound structure: 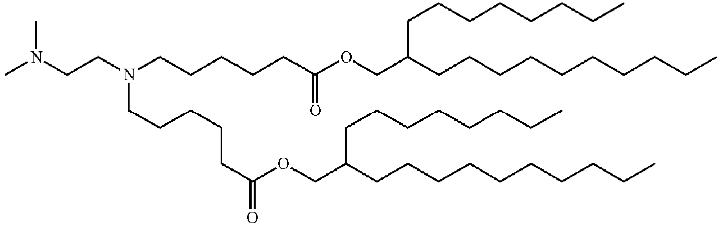 and pharmaceutically acceptable salts thereof. In some embodiments, the compositions and methods of the present invention include a cationic lipid having the compound structure: 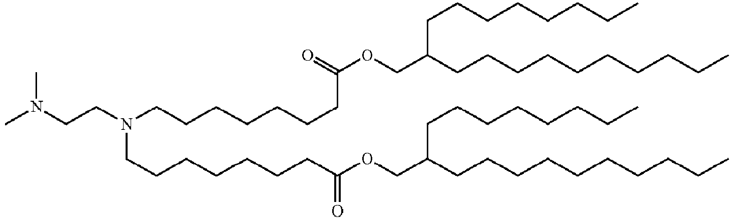 and pharmaceutically acceptable salts thereof. In some embodiments, the compositions and methods of the present invention include a cationic lipid having the compound structure: 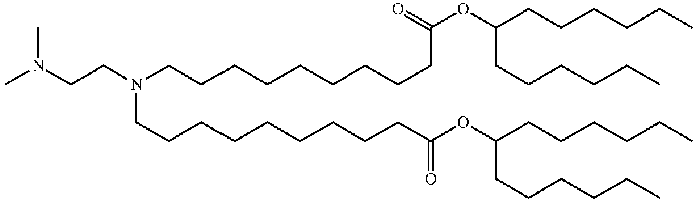 and pharmaceutically acceptable salts thereof. In some embodiments, the compositions and methods of the present invention include a cationic lipid having the compound structure: 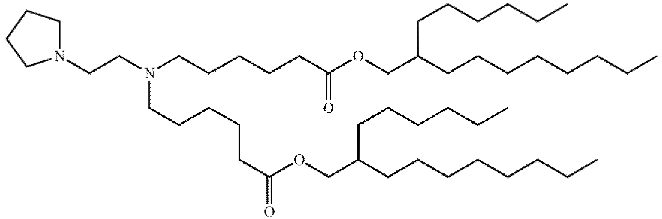 and pharmaceutically acceptable salts thereof. In some embodiments, the compositions and methods of the present invention include a cationic lipid having the compound structure: 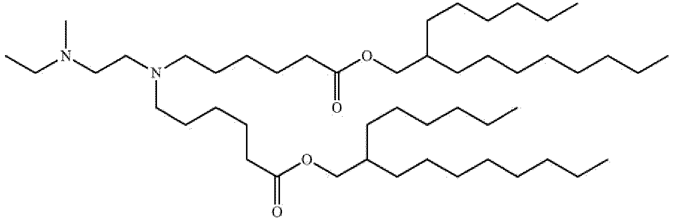 and pharmaceutically acceptable salts thereof. In some embodiments, the compositions and methods of the present invention include a cationic lipid having the compound structure: 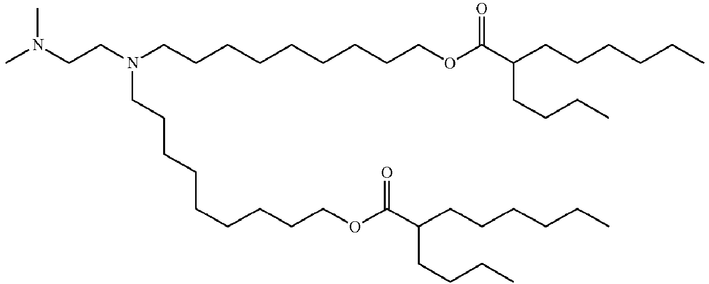 and pharmaceutically acceptable salts thereof.

Other suitable cationic lipids for use in the compositions and methods of the invention include the cationic lipids as described in International Patent Publication WO 2017/004143, which is incorporated herein by reference. In some embodiments, the compositions and methods of the present invention include a cationic lipid having the compound structure: 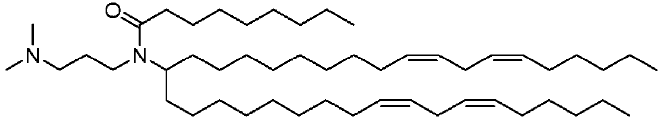 and pharmaceutically acceptable salts thereof. In some embodiments, the compositions and methods of the present invention include a cationic lipid having the compound structure: 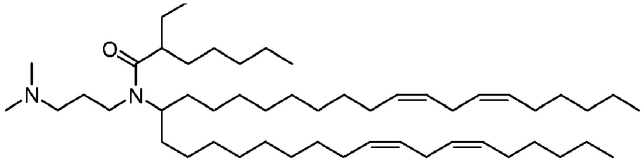 and pharmaceutically acceptable salts thereof. In some embodiments, the compositions and methods of the present invention include a cationic lipid having the compound structure: 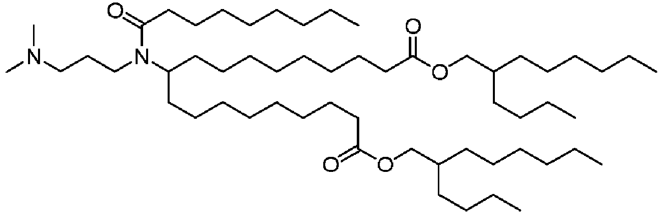 and pharmaceutically acceptable salts thereof. In some embodiments, the compositions and methods of the present invention include a cationic lipid having the compound structure: 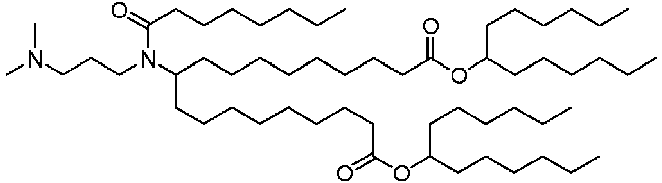 and pharmaceutically acceptable salts thereof. In some embodiments, the compositions and methods of the present invention include a cationic lipid having the compound structure: 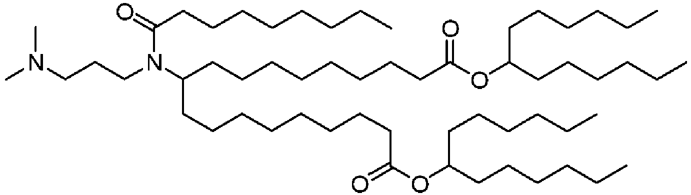 and pharmaceutically acceptable salts thereof. In some embodiments, the compositions and methods of the present invention include a cationic lipid having the compound structure: 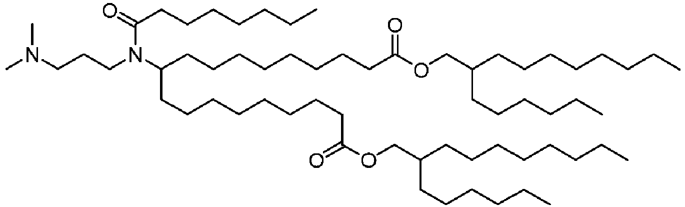 and pharmaceutically acceptable salts thereof. In some embodiments, the compositions and methods of the present invention include a cationic lipid having the compound structure: 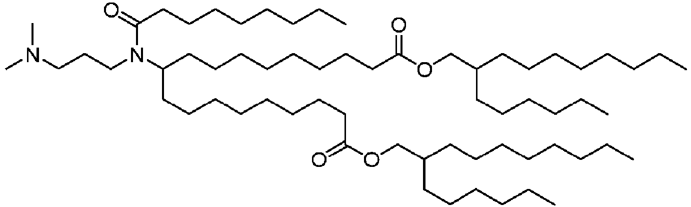 and pharmaceutically acceptable salts thereof. In some embodiments, the compositions and methods of the present invention include a cationic lipid having the compound structure: 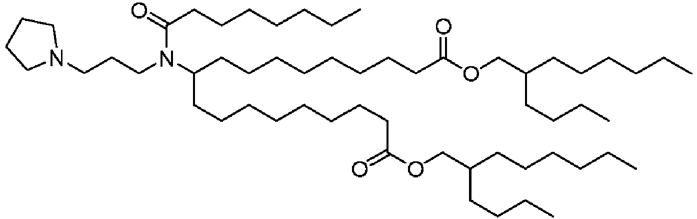 and pharmaceutically acceptable salts thereof. In some embodiments, the compositions and methods of the present invention include a cationic lipid having the compound structure: 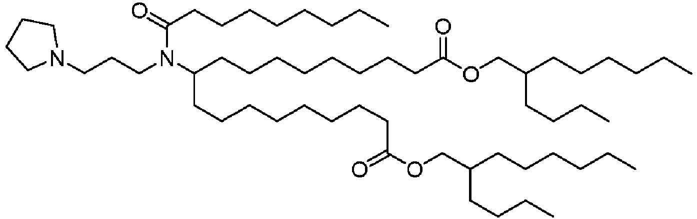 and pharmaceutically acceptable salts thereof. In some embodiments, the compositions and methods of the present invention include a cationic lipid having the compound structure: 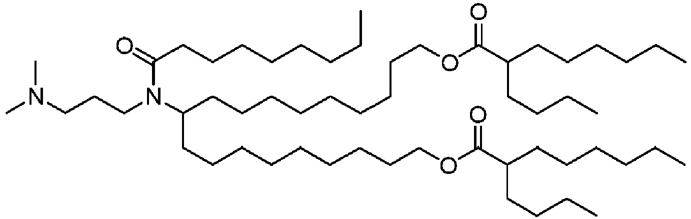 and pharmaceutically acceptable salts thereof. In some embodiments, the compositions and methods of the present invention include a cationic lipid having the compound structure: 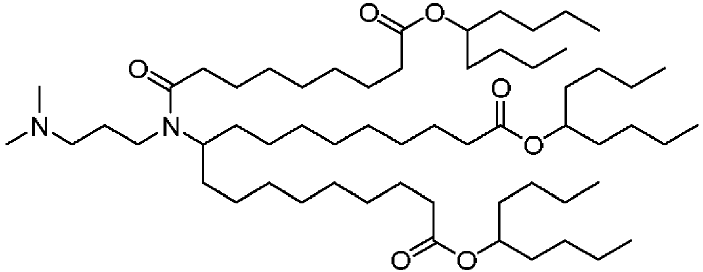 and pharmaceutically acceptable salts thereof. In some embodiments, the compositions and methods of the present invention include a cationic lipid having the compound structure: 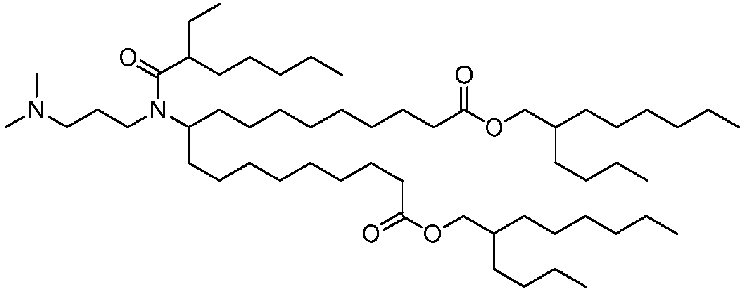 and pharmaceutically acceptable salts thereof. In some embodiments, the compositions and methods of the present invention include a cationic lipid having the compound structure: 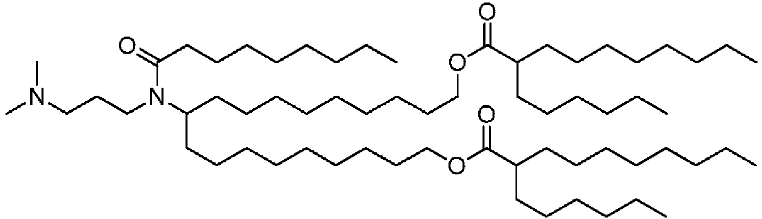 and pharmaceutically acceptable salts thereof. In some embodiments, the compositions and methods of the present invention include a cationic lipid having the compound structure: 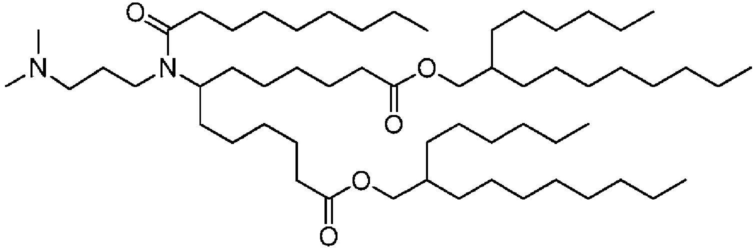 and pharmaceutically acceptable salts thereof. In some embodiments, the compositions and methods of the present invention include a cationic lipid having the compound structure: 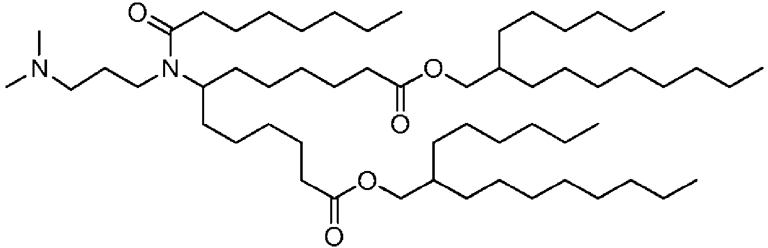 and pharmaceutically acceptable salts thereof. In some embodiments, the compositions and methods of the present invention include a cationic lipid having the compound structure: 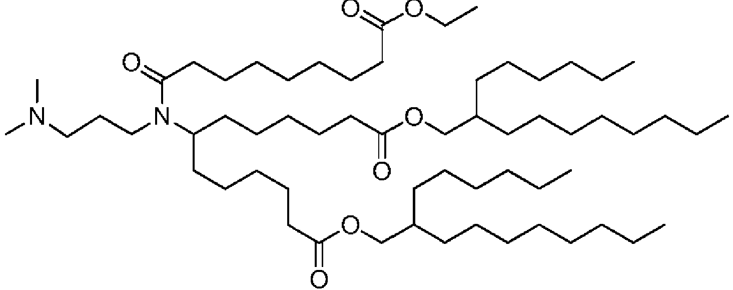 and pharmaceutically acceptable salts thereof. In some embodiments, the compositions and methods of the present invention include a cationic lipid having the compound structure: 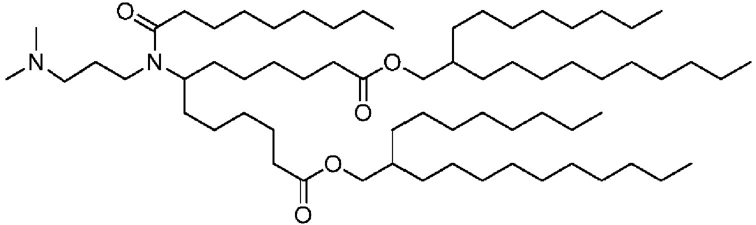 and pharmaceutically acceptable salts thereof.

Other suitable cationic lipids for use in the compositions and methods of the invention include the cationic lipids as described in International Patent Publication WO 2017/075531, which is incorporated herein by reference. In some embodiments, the compositions and methods of the present invention include a cationic lipid of the following formula: 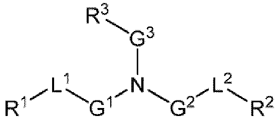 or a pharmaceutically acceptable salt thereof, wherein one of L¹ or L² is -O(C=O)-, -(C=O)O-,-C(=O)-, -O-, -S(O)ₓ, -S-S-, -C(=O)S-, -SC(=O)-, -NR^{a}C(=O)-, -C(=O)NR^{a}-, NR^{a}C(=O)NR^{a}-,-OC(=O)NR^{a}-, or -NR^{a}C(=O)O-; and the other of L¹ or L² is -O(C=O)-, -(C=O)O-, -C(=O)-, -O-,-S(O)ₓ, -S-S-, -C(=O)S-, SC(=O)-, -NR^{a}C(=O)-, -C(=O)NR^{a}-, ,NR^{a}C(=O)NR^{a}-, -OC(=O)NR^{a}- or-NR^{a}C(=O)O- or a direct bond; G¹ and G² are each independently unsubstituted C₁-C₁₂ alkylene or C₁-C₁₂ alkenylene; G³ is C₁-C₂₄ alkylene, C₁-C₂₄ alkenylene, C₃-C₈ cycloalkylene, C₃-C₈ cycloalkenylene; R^{a} is H or C₁-C₁₂ alkyl; R¹ and R² are each independently C₆-C₂₄ alkyl or C₆-C₂₄ alkenyl; R³ is H, OR⁵, CN, -C(=O)OR⁴, -OC(=O)R⁴ or -NR⁵ C(=O)R⁴; R⁴ is C₁-C₁₂ alkyl; R⁵ is H or C₁-C₆ alkyl; and x is 0, 1 or 2.

Other suitable cationic lipids for use in the compositions and methods of the invention include the cationic lipids as described in International Patent Publication WO 2017/117528, which is incorporated herein by reference. In some embodiments, the compositions and methods of the present invention include a cationic lipid having the compound structure: 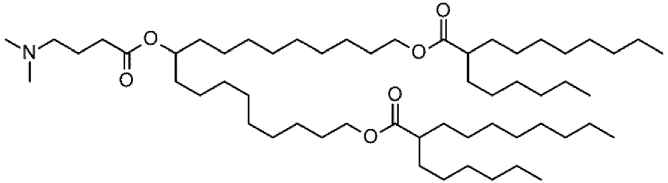 and pharmaceutically acceptable salts thereof. In some embodiments, the compositions and methods of the present invention include a cationic lipid having the compound structure: 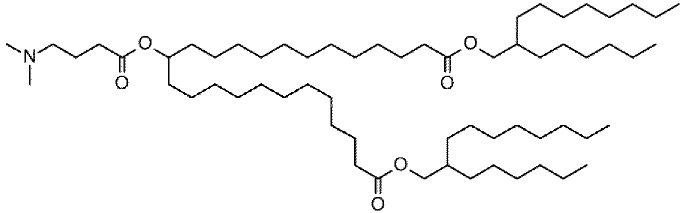 and pharmaceutically acceptable salts thereof. In some embodiments, the compositions and methods of the present invention include a cationic lipid having the compound structure: 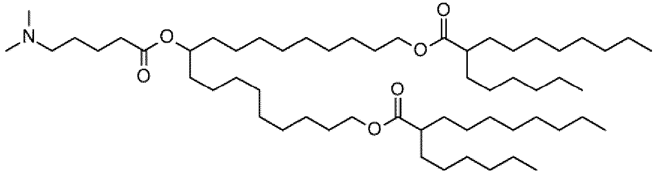 and pharmaceutically acceptable salts thereof.

Other suitable cationic lipids for use in the compositions and methods of the invention include the cationic lipids as described in International Patent Publication WO 2017/049245, which is incorporated herein by reference. In some embodiments, the cationic lipids of the compositions and methods of the present invention include a compound of one of the following formulas: 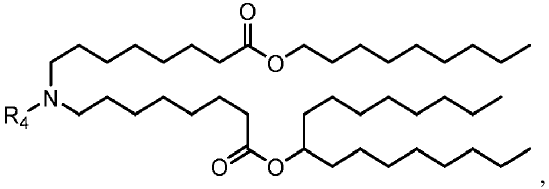 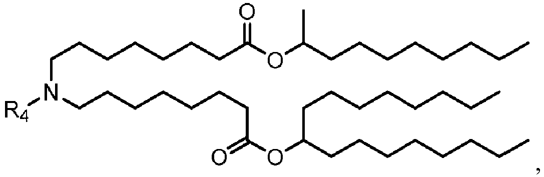 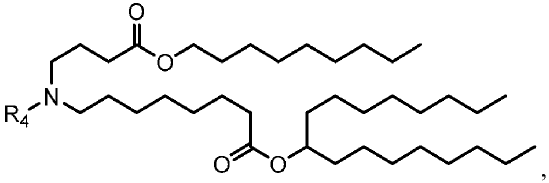 and 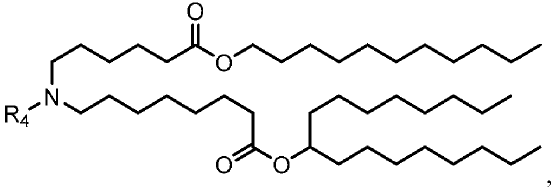 and pharmaceutically acceptable salts thereof. For any one of these four formulas, R₄ is independently selected from -(CH₂)ₙQ and -(CH₂) ₙCHQR; Q is selected from the group consisting of -OR, -OH, -O(CH₂)ₙN(R)₂, -OC(O)R, -CX₃, -CN, -N(R)C(O)R, -N(H)C(O)R, -N(R)S(O)₂R, - N(H)S(O)₂R, -N(R)C(O)N(R)₂, -N(H)C(O)N(R)₂, -N(H)C(O)N(H)(R), -N(R)C(S)N(R)₂, - N(H)C(S)N(R)₂, -N(H)C(S)N(H)(R), and a heterocycle; and n is 1, 2, or 3. In certain embodiments, the compositions and methods of the present invention include a cationic lipid having a compound structure of: 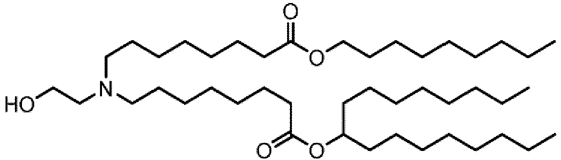 and pharmaceutically acceptable salts thereof. In certain embodiments, the compositions and methods of the present invention include a cationic lipid having a compound structure of: 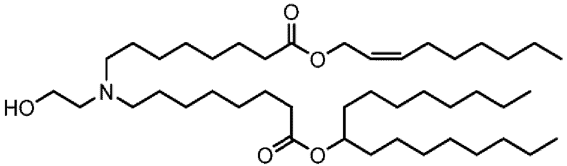 and pharmaceutically acceptable salts thereof. In certain embodiments, the compositions and methods of the present invention include a cationic lipid having a compound structure of: 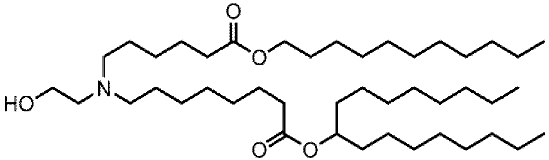 and pharmaceutically acceptable salts thereof. In certain embodiments, the compositions and methods of the present invention include a cationic lipid having a compound structure of: 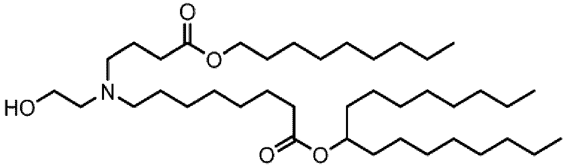 and pharmaceutically acceptable salts thereof.

Other suitable cationic lipids for use in the compositions and methods of the invention include the cationic lipids as described in International Patent Publication WO 2017/173054 and WO 2015/095340, each of which is incorporated herein by reference. In certain embodiments, the compositions and methods of the present invention include a cationic lipid having a compound structure of: 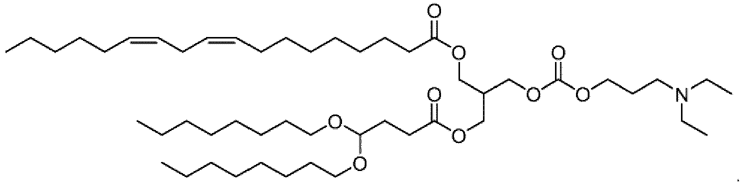 and pharmaceutically acceptable salts thereof. In certain embodiments, the compositions and methods of the present invention include a cationic lipid having a compound structure of: 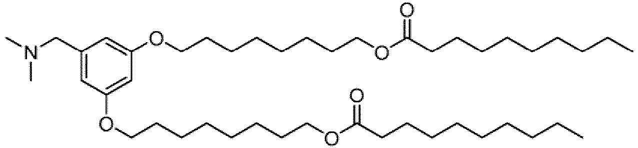 and pharmaceutically acceptable salts thereof. In certain embodiments, the compositions and methods of the present invention include a cationic lipid having a compound structure of: 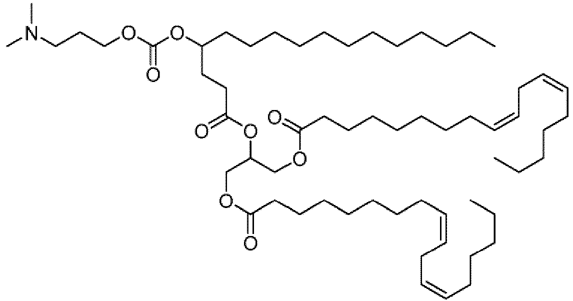 and pharmaceutically acceptable salts thereof. In certain embodiments, the compositions and methods of the present invention include a cationic lipid having a compound structure of: 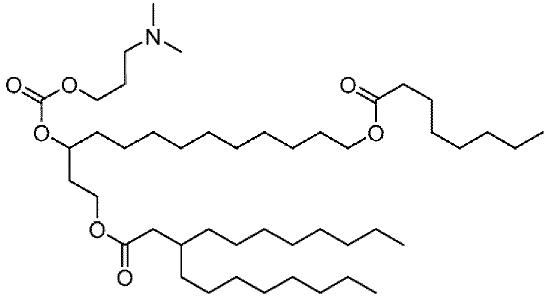 and pharmaceutically acceptable salts thereof.

Other suitable cationic lipids for use in the compositions and methods of the present invention include cleavable cationic lipids as described in International Patent Publication WO 2012/170889, which is incorporated herein by reference. In some embodiments, the compositions and methods of the present invention include a cationic lipid of the following formula: 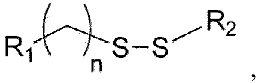 wherein R₁ is selected from the group consisting of imidazole, guanidinium, amino, imine, enamine, an optionally-substituted alkyl amino (e.g., an alkyl amino such as dimethylamino) and pyridyl; wherein R₂ is selected from the group consisting of one of the following two formulas: 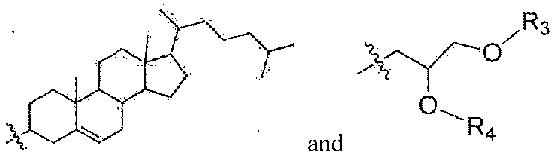 and wherein R₃ and R₄ are each independently selected from the group consisting of an optionally substituted, variably saturated or unsaturated C₆-C₂₀ alkyl and an optionally substituted, variably saturated or unsaturated C₆-C₂₀ acyl; and wherein n is zero or any positive integer (e.g., one, two, three, four, five, six, seven, eight, nine, ten, eleven, twelve, thirteen, fourteen, fifteen, sixteen, seventeen, eighteen, nineteen, twenty or more). In certain embodiments, the compositions and methods of the present invention include a cationic lipid, "HGT4001", having a compound structure of: 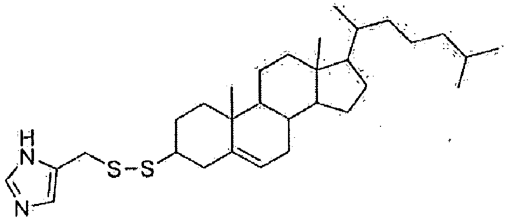 (HGT4001) and pharmaceutically acceptable salts thereof. In certain embodiments, the compositions and methods of the present invention include a cationic lipid, "HGT4002", having a compound structure of: 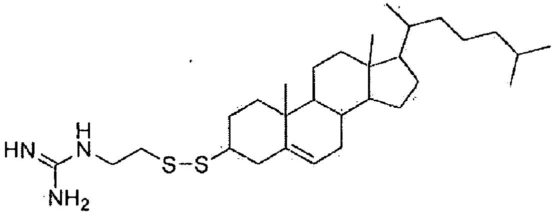 (HGT4002) and pharmaceutically acceptable salts thereof. In certain embodiments, the compositions and methods of the present invention include a cationic lipid, "HGT4003", having a compound structure of: 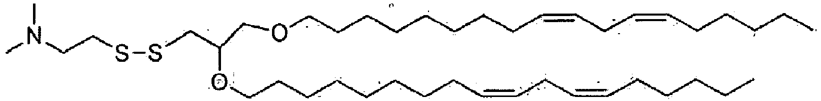 (HGT4003) and pharmaceutically acceptable salts thereof. In certain embodiments, the compositions and methods of the present invention include a cationic lipid, "HGT4004", having a compound structure of: 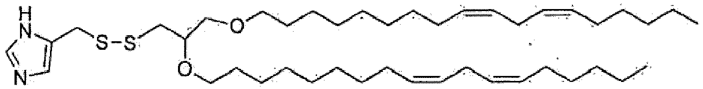 (HGT4004) and pharmaceutically acceptable salts thereof. In certain embodiments, the compositions and methods of the present invention include a cationic lipid "HGT4005", having a compound structure of: 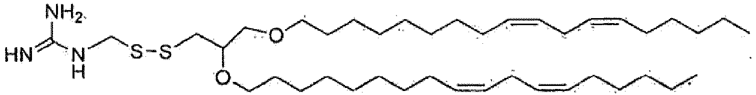 (HGT4005) and pharmaceutically acceptable salts thereof.

Other suitable cationic lipids for use in the compositions and methods of the present invention include cleavable cationic lipids as described in U.S. Provisional Application No. 62/672,194, filed May 16, 2018, and incorporated herein by reference. In certain embodiments, the compositions and methods of the present invention include a cationic lipid that is any of general formulas or any of structures (1a)-(21a) and (1b)-(21b) and (22)-(237) described in U.S. Provisional Application No. 62/672,194. In certain embodiments, the compositions and methods of the present invention include a cationic lipid that has a structure according to Formula (I'), 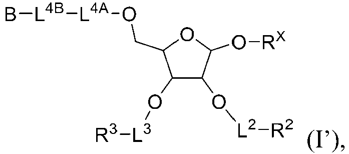 wherein:
R^{X} is independently -H, -L¹-R¹, or -L^{5A}-L^{5B}-B';
each of L¹, L², and L³ is independently a covalent bond, -C(O)-, -C(O)O-, -C(O)S-, or - C(O)NR^{L}-;
each L^{4A} and L^{5A} is independently -C(O)-, -C(O)O-, or -C(O)NR^{L}-;
each L^{4B} and L^{5B} is independently C₁-C₂₀ alkylene; C₂-C₂₀ alkenylene; or C₂-C₂₀ alkynylene;
each B and B' is NR⁴R⁵ or a 5- to 10-membered nitrogen-containing heteroaryl;
each R¹, R², and R³ is independently C₆-C₃₀ alkyl, C₆-C₃₀ alkenyl, or C₆-C₃₀ alkynyl;
each R⁴ and R⁵ is independently hydrogen, C₁-C₁₀ alkyl; C₂-C₁₀ alkenyl; or C₂-C₁₀ alkynyl; and
each R^{L} is independently hydrogen, C₁-C₂₀ alkyl, C₂-C₂₀ alkenyl, or C₂-C₂₀ alkynyl.
In certain embodiments, the compositions and methods of the present invention include a cationic lipid that is Compound (139) of 62/672,194, having a compound structure of: 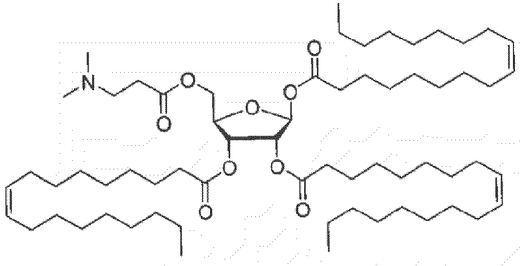 ("18:1 Carbon tail-ribose lipid").

In some embodiments, the compositions and methods of the present invention include the cationic lipid, N-[1-(2,3-dioleyloxy)propyl]-N,N,N-trimethylammonium chloride ("DOTMA"). (Feigner et al. (Proc. Nat'l Acad. Sci. 84, 7413 (1987); U.S. Pat. No. 4,897,355, which is incorporated herein by reference). Other cationic lipids suitable for the compositions and methods of the present invention include, for example, 5-carboxyspermylglycinedioctadecylamide ("DOGS"); 2,3-dioleyloxy-N-[2(spermine-carboxamido)ethyl]-N,N-dimethyl-1-propanaminium ("DOSPA") (Behr et al. Proc. Nat.'l Acad. Sci. 86, 6982 (1989), U.S. Pat. No. 5,171,678; U.S. Pat. No. 5,334,761); 1,2-Dioleoyl-3-Dimethylammonium-Propane ("DODAP"); 1,2-Dioleoyl-3-Trimethylammonium-Propane ("DOTAP").

Additional exemplary cationic lipids suitable for the compositions and methods of the present invention also include: 1,2-distearyloxy-N,N-dimethyl-3-aminopropane ( "DSDMA"); 1,2-dioleyloxy-N,N-dimethyl-3-aminopropane ("DODMA"); 1 ,2-dilinoleyloxy-N,N-dimethyl-3-aminopropane ("DLinDMA"); 1,2-dilinolenyloxy-N,N-dimethyl-3-aminopropane ("DLenDMA"); N-dioleyl-N,N-dimethylammonium chloride ("DODAC"); N,N-distearyl-N,N-dimethylammonium bromide ("DDAB"); N-(1,2-dimyristyloxyprop-3-yl)-N,N-dimethyl-N-hydroxyethyl ammonium bromide ("DMRIE"); 3-dimethylamino-2-(cholest-5-en-3-beta-oxybutan-4-oxy)-1-(cis,cis-9,12-octadecadienoxy)propane ("CLinDMA"); 2-[5'-(cholest-5-en-3-beta-oxy)-3'-oxapentoxy)-3-dimethy 1-1-(cis,cis-9',1-2'-octadecadienoxy)propane ("CpLinDMA"); N,N-dimethyl-3,4-dioleyloxybenzylamine ("DMOBA"); 1,2-N,N'-dioleylcarbamyl-3-dimethylaminopropane ("DOcarbDAP"); 2,3-Dilinoleoyloxy-N,N-dimethylpropylamine("DLinDAP"); 1,2-N,N'-Dilinoleylcarbamyl-3-dimethylaminopropane ("DLincarbDAP"); 1,2-Dilinoleoylcarbamyl-3-dimethylaminopropane ("DLinCDAP"); 2,2-dilinoleyl-4-dimethylaminomethyl-[1,3]-dioxolane ("DLin-K-DMA"); 2-((8-[(3P)-cholest-5-en-3-yloxy]octyl)oxy)-N, N-dimethyl-3-[(9Z, 12Z)-octadeca-9, 12-dien-1 -yloxy]propane-1-amine ("Octyl-CLinDMA"); (2R)-2-((8-[(3beta)-cholest-5-en-3-yloxy]octyl)oxy)-N, N-dimethyl-3-[(9Z, 12Z)-octadeca-9, 12-dien-1-yloxy]propan-1 -amine ("Octyl-CLinDMA (2R)"); (2S)-2-((8-[(3P)-cholest-5-en-3-yloxy]octyl)oxy)-N, fsl-dimethyh3-[(9Z, 12Z)-octadeca-9, 12-dien-1 -yloxy]propan-1 -amine ("Octyl-CLinDMA (2S)"); 2,2-dilinoleyl-4-dimethylaminoethyl-[1,3]-dioxolane ("DLin-K-XTC2-DMA"); and 2-(2,2-di((9Z,12Z)-octadeca-9,12-dien- 1-yl)-1,3-dioxolan-4-yl)-N,N-dimethylethanamine ("DLin-KC2-DMA") (see, WO 2010/042877, which is incorporated herein by reference; Semple et al., Nature Biotech. 28: 172-176 (2010)). (Heyes, J., et al., J Controlled Release 107: 276-287 (2005); Morrissey, DV., et al., Nat. Biotechnol. 23(8): 1003-1007 (2005); International Patent Publication WO 2005/121348). In some embodiments, one or more of the cationic lipids comprise at least one of an imidazole, dialkylamino, or guanidinium moiety.

In some embodiments, one or more cationic lipids suitable for the compositions and methods of the present invention include 2,2-Dilinoleyl-4-dimethylaminoethyl-[1,3]-dioxolane ("XTC"); (3aR,5s,6aS)-N,N-dimethyl-2,2-di((9Z,12Z)-octadeca-9,12-dienyl)tetrahydro-3aH-cyclopenta[d] [1 ,3]dioxol-5-amine ("ALNY-100") and/or 4,7,13-tris(3-oxo-3-(undecylamino)propyl)-N1,N16-diundecyl-4,7,10,13-tetraazahexadecane-1,16-diamide ("NC98-5").

In some embodiments, the compositions of the present invention include one or more cationic lipids that constitute at least about 5%, 10%, 20%, 30%, 35%, 40%, 45%, 50%, 55%, 60%, 65%, or 70%, measured by weight, of the total lipid content in the composition, e.g., a lipid nanoparticle. In some embodiments, the compositions of the present invention include one or more cationic lipids that constitute at least about 5%, 10%, 20%, 30%, 35%, 40%, 45%, 50%, 55%, 60%, 65%, or 70%, measured as a mol %, of the total lipid content in the composition, e.g., a lipid nanoparticle. In some embodiments, the compositions of the present invention include one or more cationic lipids that constitute about 30-70 % (e.g., about 30-65%, about 30-60%, about 30-55%, about 30-50%, about 30-45%, about 30-40%, about 35-50%, about 35-45%, or about 35-40%), measured by weight, of the total lipid content in the composition, e.g., a lipid nanoparticle. In some embodiments, the compositions of the present invention include one or more cationic lipids that constitute about 30-70 % (e.g., about 30-65%, about 30-60%, about 30-55%, about 30-50%, about 30-45%, about 30-40%, about 35-50%, about 35-45%, or about 35-40%), measured as mol %, of the total lipid content in the composition, e.g., a lipid nanoparticle

In some embodiments, sterol-based cationic lipids may be use instead or in addition to cationic lipids described herein. Suitable sterol-based cationic lipids are dialkylamino-, imidazole-, and guanidinium-containing sterol-based cationic lipids. For example, certain embodiments are directed to a composition comprising one or more sterol-based cationic lipids comprising an imidazole, for example, the imidazole cholesterol ester or "ICE" lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate, as represented by structure (I) below. In certain embodiments, a lipid nanoparticle for delivery of RNA (e.g., mRNA) encoding a functional protein may comprise one or more imidazole-based cationic lipids, for example, the imidazole cholesterol ester or "ICE" lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate, as represented by the following structure: 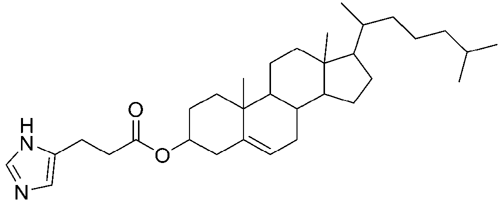 (ICE)

In some embodiments, the percentage of cationic lipid in a liposome may be greater than 10%, greater than 20%, greater than 30%, greater than 40%, greater than 50%, greater than 60%, or greater than 70%. In some embodiments, cationic lipid(s) constitute(s) about 30-50 % (e.g., about 30-45%, about 30-40%, about 35-50%, about 35-45%, or about 35-40%) of the liposome by weight. In some embodiments, the cationic lipid (e.g., ICE lipid) constitutes about 30%, about 35%, about 40 %, about 45%, or about 50% of the liposome by molar ratio.

In preferred embodiments, the one or more cationic lipids comprise cKK-E12 3,6-bi s(4-(bis(2-hydroxydodecyl)amino)butyl)piperazine-2, 5 -dione):

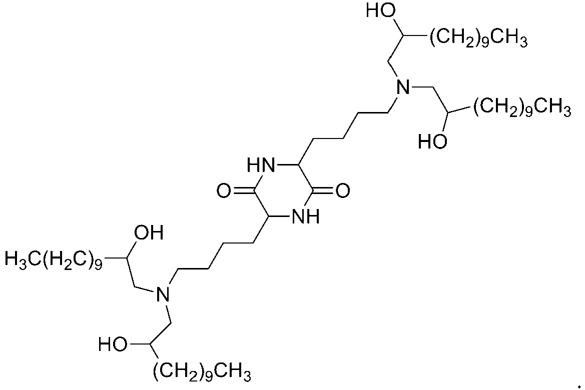

### Non-cationic/Helper Lipids

In some embodiments, provided liposomes contain one or more non-cationic ("helper") lipids. As used herein, the phrase "non-cationic lipid" refers to any neutral, zwitterionic or anionic lipid. As used herein, the phrase "anionic lipid" refers to any of a number of lipid species that carry a net negative charge at a selected H, such as physiological pH. Non-cationic lipids include, but are not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoyl-phosphatidylethanolamine (POPE), dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1-trans PE, 1-stearoyl-2-oleoyl-phosphatidyethanolamine (SOPE), or a mixture thereof.

In some embodiments, such non-cationic lipids may be used alone, but are preferably used in combination with other excipients, for example, cationic lipids. In some embodiments, the non-cationic lipid may comprise a molar ratio of about 5% to about 90%, or about 10 % to about 70% of the total lipid present in a liposome. In some embodiments, a non-cationic lipid is a neutral lipid, i.e., a lipid that does not carry a net charge in the conditions under which the composition is formulated and/or administered. In some embodiments, the percentage of non-cationic lipid in a liposome may be greater than 5%, greater than 10%, greater than 20%, greater than 30%, or greater than 40%.

### Cholesterol-based Lipids

In some embodiments, provided liposomes comprise one or more cholesterol-based lipids. For example, suitable cholesterol-based cationic lipids include, for example, DC-Choi (N,N-dimethyl-N-ethylcarboxamidocholesterol), 1,4-bis(3-N-oleylamino-propyl)piperazine (Gao, et al. Biochem. Biophys. Res. Comm. 179, 280 (1991); Wolf et al. BioTechniques 23, 139 (1997); U.S. Pat. No. 5,744,335), or ICE. In some embodiments, the cholesterol-based lipid may comprise a molar ration of about 2% to about 30%, or about 5% to about 20% of the total lipid present in a liposome. In some embodiments, the percentage of cholesterol-based lipid in the liposome may be greater than 5, %, 10%, greater than 20%, greater than 30%, or greater than 40%.

### PEGylated Lipids

In some embodiments, provided liposomes comprise one or more PEGylated lipids. For example, the use of polyethylene glycol (PEG)-modified phospholipids and derivatized lipids such as derivatized ceramides (PEG-CER), including N-Octanoyl-Sphingosine-1-[Succinyl(Methoxy Polyethylene Glycol)-2000] (C8 PEG-2000 ceramide) is also contemplated by the present invention in combination with one or more of the cationic and, in some embodiments, other lipids together which comprise the liposome. Contemplated PEG-modified lipids include, but are not limited to, a polyethylene glycol chain of up to 2kDa, up to 3 kDa, up to 4kDa or 5 kDa in length covalently attached to a lipid with alkyl chain(s) of C6-C20 length. In some embodiments, a PEG-modified or PEGylated lipid is PEGylated cholesterol or PEG-2K. The addition of such components may prevent complex aggregation and may also provide a means for increasing circulation lifetime and increasing the delivery of the lipid-nucleic acid composition to the target cell, (Klibanov et al. (1990) FEBS Letters, 268 (1): 235-237), or they may be selected to rapidly exchange out of the formulation in vivo (see U.S. Pat. No. 5,885,613). In some embodiments, a PEG-modified or PEGylated lipid is PEGylated cholesterol or PEG-2K. In some embodiments, particularly useful exchangeable lipids are PEG-ceramides having shorter acyl chains (e.g., C14 or C18).

In some embodiments, particularly useful exchangeable lipids are PEG-ceramides having shorter acyl chains (e.g., C14 or C18). The PEG-modified phospholipid and derivitized lipids of the present invention may comprise a molar ratio from about 0% to about 15%, about 0.5% to about 15%, about 1% to about 15%, about 4% to about 10%, or about 2% of the total lipid present in the liposome. PEG-modified phospholipid and derivatized lipids may constitute at least about 5%, 10%, 20%, 30%, 40%, 50%, 60%, or 70% of the total lipids in a suitable lipid solution by weight or by molar. In some embodiments, PEGylated lipid lipid(s) constitute(s) about 30-50 % (e.g., about 30-45%, about 30-40%, about 35-50%, about 35-45%, or about 35-40%) of the total lipids in a suitable lipid solution by weight or by molar.

According to various embodiments, the selection of cationic lipids, non-cationic lipids and/or PEG-modified lipids which comprise the liposome, as well as the relative molar ratio of such lipids to each other, is based upon the characteristics of the selected lipid(s), the nature of the intended target cells, the characteristics of the mRNA to be delivered. Additional considerations include, for example, the saturation of the alkyl chain, as well as the size, charge, pH, pKa, fusogenicity and toxicity of the selected lipid(s). Thus, the molar ratios may be adjusted accordingly.

### Liposome formulations

A suitable liposome for the present invention may include one or more of any of the cationic lipids, non-cationic lipids, cholesterol lipids, PEGylated lipids and/or polymers described herein at various ratios. Typically, a liposome in accordance with the present invention comprises a cationic lipid, a non-cationic lipid, a cholesterol lipid and a PEGylated lipid.

The formulations described herein include a multi-component lipid mixture of varying ratios employing one or more cationic lipids, helper lipids (e.g., non-cationic lipids and/or cholesterol-based lipids) and PEGylated lipids designed to encapsulate mRNA encoding a peptide, polypeptide or protein. Cationic lipids can include (but not exclusively) DOTAP (1,2-dioleyl-3-trimethylammonium propane), DODAP (1,2-dioleyl-3-dimethylammonium propane) , DOTMA (1,2-di-O-octadecenyl-3-trimethylammonium propane), DLinDMA (Heyes, J.; Palmer, L.; Bremner, K.; MacLachlan, I. "Cationic lipid saturation influences intracellular delivery of encapsulated nucleic acids" J. Contr. Rel. 2005, 107, 276-287), DLin-KC2-DMA (Semple, S.C. et al. "Rational Design of Cationic Lipids for siRNA Delivery" Nature Biotech. 2010, 28, 172-176), C12-200 (Love, K.T. et al. "Lipid-like materials for low-dose in vivo gene silencing" PNAS 2010, 107, 1864-1869), cKK-E12 (3,6-bis(4-(bis(2-hydroxydodecyl)amino)butyl)piperazine-2,5-dione), HGT5000, HGT5001, HGT4003, ICE, OF-02, dialkylamino-based, imidazole-based, guanidinium-based, etc. Helper lipids can include (but not exclusively) DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine), DOPG (1,2-dioleoyl-sn-glycero-3-phospho-(1'-rac-glycerol)), cholesterol, etc. The PEGylated lipids can include (but not exclusively) a poly(ethylene) glycol chain of up to 5 kDa in length covalently attached to a lipid with alkyl chain(s) of C₆-C₂₀ length.

As non-limiting examples, a suitable liposome formulation may include a combination selected from cKK-E12, DOPE, cholesterol and DMG-PEG2K; C12-200, DOPE, cholesterol and DMG-PEG2K; HGT4003, DOPE, cholesterol and DMG-PEG2K; or ICE, DOPE, cholesterol and DMG-PEG2K or ICE, DOPE and DMG-PEG2K. Additional combinations of lipids are described in the art, e.g., U.S. Serial No. 62/420,421 (filed on November 10, 2016), U.S. Serial No. 62/421,021 (filed on November 11, 2016), U.S. Serial No. 62/464,327 (filed on February 27, 2017), and PCT Application entitled "Novel ICE-based Lipid Nanoparticle Formulation for Delivery of mRNA," filed on November 10, 2017, the disclosures of which are included here in their full scope by reference.

In various embodiments, cationic lipids (e.g., cKK-E12, C12-200, ICE, and/or HGT4003) constitute about 30-60 % (e.g., about 30-55%, about 30-50%, about 30-45%, about 30-40%, about 35-50%, about 35-45%, or about 35-40%) of the liposome by molar ratio. In some embodiments, the percentage of cationic lipids (e.g., cKK-E12, C12-200, ICE, and/or HGT4003) is or greater than about 30%, about 35%, about 40 %, about 45%, about 50%, about 55%, or about 60% of the liposome by molar ratio.

In some embodiments, the ratio of cationic lipid(s) to non-cationic lipid(s) to cholesterol-based lipid(s) to PEGylated lipid(s) may be between about 30-60:25-35:20-30:1-15, respectively. In some embodiments, the ratio of cationic lipid(s) to non-cationic lipid(s) to cholesterol-based lipid(s) to PEGylated lipid(s) is approximately 40:30:20:10, respectively. In some embodiments, the ratio of cationic lipid(s) to non-cationic lipid(s) to cholesterol-based lipid(s) to PEGylated lipid(s) is approximately 40:30:25:5, respectively. In some embodiments, the ratio of cationic lipid(s) to non-cationic lipid(s) to cholesterol-based lipid(s) to PEGylated lipid(s) is approximately 40:32:25:3, respectively. In some embodiments, the ratio of cationic lipid(s) to non-cationic lipid(s) to cholesterol-based lipid(s) to PEGylated lipid(s) is approximately 50:25:20:5.

### Formation of Liposomes

The liposomes used in the methods of the inventions can be prepared by various techniques which are presently known in the art. For example, multilamellar vesicles (MLV) may be prepared according to conventional techniques, such as by depositing a selected lipid on the inside wall of a suitable container or vessel by dissolving the lipid in an appropriate solvent, and then evaporating the solvent to leave a thin film on the inside of the vessel or by spray drying. An aqueous phase then may be added to the vessel with a vortexing motion which results in the formation of MLVs. Unilamellar vesicles (ULV) can then be formed by homogenization, sonication or extrusion of the multilamellar vesicles. In addition, unilamellar vesicles can be formed by detergent removal techniques.

In certain embodiments, the mRNA is associated on both the surface of the liposome and encapsulated within the same liposome. For example, during preparation of the mRNA encapsulated in a liposome, cationic liposomes may associate with the mRNA through electrostatic interactions. For example, during preparation of the liposomes of the invention, cationic liposomes may associate with the mRNA through electrostatic interactions.

The methods of the invention comprise one or more mRNAs encode the one or more peptides, polypeptides or proteins encapsulated in one or more liposomes. In some embodiments, the one or more mRNA species may be encapsulated in the same liposome. In some embodiments, the one or more mRNA species may be encapsulated in different liposomes. In some embodiments, the mRNA is encapsulated in one or more liposomes, which differ in their lipid composition, molar ratio of lipid components, size, charge (Zeta potential), targeting ligands and/or combinations thereof. In some embodiments, the one or more liposome may have a different composition of cationic lipids, neutral lipid, PEG-modified lipid and/or combinations thereof. In some embodiments the one or more liposomes may have a different molar ratio of cationic lipid, neutral lipid, cholesterol and PEG-modified lipid used to create the liposome.

The process of incorporation of a desired mRNA into a liposome is often referred to as "loading". Exemplary methods are described in Lasic, et al., FEBS Lett., 312: 255-258, 1992, which is incorporated herein by reference. In a typical embodiment, the mRNA of the invention is encapsulated in a liposome using the methods described in WO 2018/089801 (the teachings of which are incorporated herein by reference in their entirety). Briefly, the mRNA is encapsulated by mixing of a solution comprising pre-formed liposomes with mRNA such that liposomes encapsulating mRNA are formed.

Typically, the liposome-incorporated nucleic acids is completely located in the interior space of the liposome within the bilayer membrane of the liposome, although as discussed above, some of the mRNA (e.g., no more than 10% of total mRNA in the liposome composition) may also be associated with the exterior surface of the liposome membrane. The incorporation of a nucleic acid into liposomes is also referred to herein as "encapsulation". Typically, the purpose of incorporating an mRNA into a liposome is to protect the nucleic acid from an environment which may contain enzymes or chemicals that degrade nucleic acids and/or systems or receptors that cause the rapid excretion of the nucleic acids. Accordingly, in some embodiments, a suitable delivery vehicle is capable of enhancing the stability of the mRNA contained therein and/or facilitate the delivery of mRNA to the target cell or tissue.

### Liver-specific targeting of liposomes

Targeting the liposomes to the liver exploits the tolerogenic nature of the liver to induce systemic immune tolerance to foreign peptides, polypeptides or proteins it encounters. Without wishing to be bound by any particular theory, the inventors believe that induction of immune tolerance is mediated by hepatocytes and/or the liver sinusoidal endothelial cells, rather than the resident antigen-presenting cells present in the liver (e.g. Kupffer cells).

The invention therefore provides liposomes which preferentially target mRNA that encodes a peptide, polypeptide or protein for which immune tolerance is desirable to the liver. In preferred embodiments, the liposome specifically targets the one or more mRNAs encode the one or more peptides, polypeptides or proteins to the hepatocytes and/or the sinusoidal endothelial cells.

### Lipid composition

By varying the lipid composition, it is possible to design liposome that preferentially target specific organs in a test subject. For example, DOTMA and DOPE have been used to prepare liposomes with positive as well as negative excess charge, depending on the DOTMA:DOPE ratio. Positively charged mRNA-lipoplexes target predominantly the lungs and less the spleen (Kranz et al., Nature 2016, 534(7607):396-401). By decreasing the cationic lipid content, lipoplexe can be prepared that preferentially target the spleen. Near-neutral or only slightly negative lipoplexes almost exclusively target the spleen.

The spleen is an important lymphoid organ, in which antigen presenting cells are in close proximity to T cells. The spleen therefore provides an ideal microenvironment for efficient priming and amplification of T-cell responses, but is less beneficial in inducing immune tolerance.

In contrast, the liver provides a cellular environment that favours tolerance over an immune response. By preferentially targeting mRNA-encapsulating liposomes to the liver, rather than the spleen or lungs, the inventors found that they can make use of the tolerogenic nature of the liver to induce systemic immunological tolerance to peptides, polypeptides or proteins encoded by the mRNAs of the invention, namely by inducing Treg that are specific to the mRNA-encoded peptides, polypeptides or proteins.

Liposomes comprising a cationic lipid such as cKK-E12, C12-200, HGT4003, HGT5001, HGT5000, DLinKC2DMA, DODAP, DODMA, a non-cationic lipid such as DOPE, a neutral lipid such as cholesterol, and a PEG-modified lipid such as DMG-PEG2K have been shown to preferentially target encapsulated mRNA to the liver (see *e.g.* WO 2012/170930 and WO 2015/061467, which are incorporated herewith by reference). Preferential liver delivery can also be achieved in liposomes comprising a cholesterol-derived cationic lipid such as ICE, a non-cationic lipid such as DOPE, and a PEG-modified lipid such as DMG-PEG2K (WO 2011/068810, which is incorporated herewith by reference).

In preferred embodiments, the liposome comprises cKK-E12, DOPE, cholesterol and DMG-PEG2K.

### Liposome Size

In addition to the lipid composition, the size of a liposome can also determine whether it is preferentially delivered to a particular tissue. For example, DOTMA and DOPE have been used to prepare liposomes of reproducible particle size of 200-400 nm. Liposomes of this size preferentially target the spleen and the lungs (Kranz et al. (2016) Nature 534, 396-401). Liposomes prepared in accordance with the invention are typically sized such that their dimensions are smaller than the fenestrations of the endothelial layer that line hepatic sinusoids in the liver.

Liver sinusoidal endothelial cells are perforated with fenestrations that are 50-250 nm in diameter. Accordingly, a suitable liposome for practising the invention has a size no greater than about 10 - 120 nm (e.g., ranging from about 10 - 100 nm, 10 - 90 nm, 10 - 80 nm, 10 - 70 nm, 10 - 60 nm, or 10 - 50 nm). A particularly suitable liposome for use with the invention has a size of about 80-120 nm. In some embodiments, a suitable liposome has a size of less than about 100 nm. In certain embodiments, the liposome has a size of about 100 nm. In certain embodiments, the liposome has a size of about 50-60 nm. In certain embodiments, the liposome has a size of about 50 nm, 60 nm, 70 nm, 80 nm or 90 nm. Since such liposomes can readily penetrate the endothelial fenestrations, they deliver the encapsulated mRNA to hepatocytes and the liver sinusoidal endothelial cells. The size of a liposome is determined by the length of the largest diameter of the liposome particle.

A variety of alternative methods known in the art are available for sizing of a population of liposomes. One such sizing method is described in U.S. Pat. No. 4,737,323, incorporated herein by reference. Sonicating a liposome suspension either by bath or probe sonication produces a progressive size reduction down to small ULV less than about 0.05 microns in diameter. Homogenization is another method that relies on shearing energy to fragment large liposomes into smaller ones. In a typical homogenization procedure, MLV are recirculated through a standard emulsion homogenizer until selected liposome sizes, typically between about 0.1 and 0.5 microns, are observed. The size of the liposomes may be determined by quasi-electric light scattering (QELS) as described in Bloomfield, Ann. Rev. Biophys. Bioeng., 10:421-150 (1981), incorporated herein by reference. Average liposome diameter may be reduced by sonication of formed liposomes. Intermittent sonication cycles may be alternated with QELS assessment to guide efficient liposome synthesis.

### Exemplary Formulation Protocols

In certain embodiments, the cationic lipid constitutes about 30-60 % of the liposome by molar ratio. In other embodiments, the cationic lipid constitutes about 30%, 40 %, 50%, or 60% of the liposome by molar ratio. In some embodiments, the ratio of cationic lipids:non-cationic lipids:cholesterol lipids:PEGylated lipids is approximately 40:30:20:10 by molar ratio. In some embodiments, the ratio of cationic lipids:non-cationic lipids:cholesterol lipids:PEGylated lipids is approximately 40:30:25:5 by molar ratio. In some embodiments, the ratio of cationic lipids:non-cationic lipids:cholesterol lipids:PEGylated lipids is approximately 40:32:25:3 by molar ratio. In some embodiments, the ratio of cationic lipids:non-cationic lipids:cholesterol lipids:PEGylated lipids is approximately 50:25:20:5 by molar ratio.

### A. cKK-E12

Aliquots of 50 mg/mL ethanolic solutions of cKK-E12, DOPE, cholesterol and DMG-PEG2K are mixed and diluted with ethanol to 3 mL final volume. Separately, an aqueous buffered solution (10 mM citrate/150 mM NaCl, pH 4.5) of MUT mRNA is prepared from a 1 mg/mL stock. The lipid solution was injected rapidly into the aqueous mRNA solution and shaken to yield a final suspension in 20% ethanol. The resulting liposome suspension was filtered, diafiltrated with 1x PBS (pH 7.4), concentrated and stored at 2-8°C. The final concentration, Zave, Dv(50) and Dv(90) of the a peptide, polypeptide or protein encapsulated mRNA were determined.

### B. C12-200

Aliquots of 50 mg/mL ethanolic solutions of C12-200, DOPE, cholesterol and DMG-PEG2K are mixed and diluted with ethanol to 3 mL final volume. Separately, an aqueous buffered solution (10 mM citrate/150 mM NaCl, pH 4.5) of a peptide, polypeptide or protein mRNA is prepared from a 1 mg/mL stock. The lipid solution is injected rapidly into the aqueous mRNA solution and shaken to yield a final suspension in 20% ethanol. The resulting liposome suspension is filtered, diafiltrated with 1x PBS (pH 7.4), concentrated and stored at 2-8°C. The final concentration, Zave, Dv(50) and Dv(90) of the a peptide, polypeptide or protein encapsulated mRNA are determined.

### C. HGT4003

Aliquots of 50 mg/mL ethanolic solutions of HGT4003, DOPE, cholesterol and DMG-PEG2K are mixed and diluted with ethanol to 3 mL final volume. Separately, an aqueous buffered solution (10 mM citrate/150 mM NaCl, pH 4.5) of a peptide, polypeptide or protein mRNA is prepared from a 1 mg/mL stock. The lipid solution is injected rapidly into the aqueous mRNA solution and shaken to yield a final suspension in 20% ethanol. The resulting liposome suspension is filtered, diafiltrated with 1x PBS (pH 7.4), concentrated and stored at 2-8°C. The final concentration, Zave, Dv(50) and Dv(90) of the a peptide, polypeptide or protein encapsulated mRNA are determined.

### D. ICE

Aliquots of 50 mg/mL ethanolic solutions of ICE, DOPE, cholesterol and DMG-PEG2K are mixed and diluted with ethanol to 3 mL final volume. Separately, an aqueous buffered solution (10 mM citrate/150 mM NaCl, pH 4.5) of a peptide, polypeptide or protein mRNA is prepared from a 1 mg/mL stock. The lipid solution is injected rapidly into the aqueous mRNA solution and shaken to yield a final suspension in 20% ethanol. The resulting liposome suspension is filtered, diafiltrated with 1x PBS (pH 7.4), concentrated and stored at 2-8°C. The final concentration, Zave, Dv(50) and Dv(90) of the a peptide, polypeptide or protein encapsulated mRNA are determined.

### E. HGT5001

Aliquots of 50 mg/mL ethanolic solutions of HGT5001, DOPE, cholesterol and DMG-PEG2K are mixed and diluted with ethanol to 3 mL final volume. Separately, an aqueous buffered solution (10 mM citrate/150 mM NaCl, pH 4.5) of a peptide, polypeptide or protein mRNA is prepared from a 1 mg/mL stock. The lipid solution is injected rapidly into the aqueous mRNA solution and shaken to yield a final suspension in 20% ethanol. The resulting liposome suspension is filtered, diafiltrated with 1x PBS (pH 7.4), concentrated and stored at 2-8°C. The final concentration, Zave, Dv(50) and Dv(90) of the a peptide, polypeptide or protein encapsulated mRNA are determined.

### F. HGT5000

Aliquots of 50 mg/mL ethanolic solutions of HGT5000, DOPE, cholesterol and DMG-PEG2K are mixed and diluted with ethanol to 3 mL final volume. Separately, an aqueous buffered solution (10 mM citrate/150 mM NaCl, pH 4.5) of a peptide, polypeptide or protein mRNA is prepared from a 1 mg/mL stock. The lipid solution is injected rapidly into the aqueous mRNA solution and shaken to yield a final suspension in 20% ethanol. The resulting liposome suspension is filtered, diafiltrated with 1x PBS (pH 7.4), concentrated and stored at 2-8°C. The final concentration, Zave, Dv(50) and Dv(90) of the a peptide, polypeptide or protein encapsulated mRNA are determined.

### G. DLinKC2DMA

Aliquots of 50 mg/mL ethanolic solutions of DLinKC2DMA, DOPE, cholesterol and DMG-PEG2K are mixed and diluted with ethanol to 3 mL final volume. Separately, an aqueous buffered solution (10 mM citrate/150 mM NaCl, pH 4.5) of a peptide, polypeptide or protein mRNA is prepared from a 1 mg/mL stock. The lipid solution is injected rapidly into the aqueous mRNA solution and shaken to yield a final suspension in 20% ethanol. The resulting liposome suspension is filtered, diafiltrated with 1x PBS (pH 7.4), concentrated and stored at 2-8°C. The final concentration, Zave, Dv(50) and Dv(90) of the a peptide, polypeptide or protein encapsulated mRNA are determined.

### H. DODAP

Aliquots of 50 mg/mL ethanolic solutions of DODAP, DOPE, cholesterol and DMG-PEG2K are mixed and diluted with ethanol to 3 mL final volume. Separately, an aqueous buffered solution (10 mM citrate/150 mM NaCl, pH 4.5) of a peptide, polypeptide or protein mRNA is prepared from a 1 mg/mL stock. The lipid solution is injected rapidly into the aqueous mRNA solution and shaken to yield a final suspension in 20% ethanol. The resulting liposome suspension is filtered, diafiltrated with 1x PBS (pH 7.4), concentrated and stored at 2-8°C. The final concentration, Zave, Dv(50) and Dv(90) of the a peptide, polypeptide or protein encapsulated mRNA are determined.

### I. DODMA

Aliquots of 50 mg/mL ethanolic solutions of DODMA, DOPE, cholesterol and DMG-PEG2K are mixed and diluted with ethanol to 3 mL final volume. Separately, an aqueous buffered solution (10 mM citrate/150 mM NaCl, pH 4.5) of a peptide, polypeptide or protein mRNA is prepared from a 1 mg/mL stock. The lipid solution is injected rapidly into the aqueous mRNA solution and shaken to yield a final suspension in 20% ethanol. The resulting liposome suspension is filtered, diafiltrated with 1x PBS (pH 7.4), concentrated and stored at 2-8°C. The final concentration, Zave, Dv(50) and Dv(90) of the a peptide, polypeptide or protein encapsulated mRNA are determined.

### mRNA preparation

Messenger RNAs according to the present invention may be synthesized according to any of a variety of known methods. For example, mRNAs according to the present invention may be synthesized via in vitro transcription (IVT). Briefly, IVT is typically performed with a linear or circular DNA template containing a promoter, a pool of ribonucleotide triphosphates, a buffer system that may include DTT and magnesium ions, and an appropriate RNA polymerase (e.g., T3, T7 or SP6 RNA polymerase), DNAse I, pyrophosphatase, and/or RNAse inhibitor. The exact conditions will vary according to the specific application.

In some embodiments, for the preparation of mRNA according to the invention, a DNA template is transcribed in vitro. A suitable DNA template typically has a promoter, for example a T3, T7 or SP6 promoter, for in vitro transcription, followed by desired nucleotide sequence for desired mRNA and a termination signal.

Typically, the mRNA according to the present invention is synthesized as unmodified mRNA. Accordingly, the mRNAs of the invention are synthesized from naturally occurring nucleotides including purines (adenine (A), guanine (G)) or pyrimidines (cytosine (C), uracil (U)).

Typically, mRNA synthesis includes the addition of a "cap" on the N-terminal (5') end, and a "tail" on the C-terminal (3') end. The presence of the cap is important in providing resistance to nucleases found in most eukaryotic cells. The presence of a "tail" serves to protect the mRNA from exonuclease degradation.

Thus, in some embodiments, mRNAs include a 5' cap structure. A 5' cap is typically added as follows: first, an RNA terminal phosphatase removes one of the terminal phosphate groups from the 5' nucleotide, leaving two terminal phosphates; guanosine triphosphate (GTP) is then added to the terminal phosphates via a guanylyl transferase, producing a 5'5'5 triphosphate linkage; and the 7-nitrogen of guanine is then methylated by a methyltransferase. Examples of cap structures include, but are not limited to, m7G(5')ppp (5'(A,G(5')ppp(5')A and G(5')ppp(5')G.

In some embodiments, mRNAs include a 3' poly(A) tail structure. A poly-A tail on the 3' terminus of mRNA typically includes about 10 to 800 adenosine nucleotides (e.g., about 300 to 500 adenosine nucleotides, about 300 to 800 adenosine nucleotides, about 10 to 500 adenosine nucleotides, about 10 to 300 adenosine nucleotides, about 10 to 200 adenosine nucleotides, about 10 to 150 adenosine nucleotides, about 10 to 100 adenosine nucleotides, about 20 to 70 adenosine nucleotides, or about 20 to 60 adenosine nucleotides). Typically, a poly-A tail in an mRNA in accordance with the invention is about 300 to about 800 adenosine nucleotides long (SEQ ID NO: 1). More commonly, the poly-A tail is about 300 adenosine nucleotides long (SEQ ID NO: 2). In some embodiments, the poly(A) tail structure comprises at least 85%, 90%, 95% or 100% adenosine.

In some embodiments, mRNAs include a 3' poly(C) tail structure. A suitable poly-C tail on the 3' terminus of mRNA typically include about 10 to 200 cytosine nucleotides (SEQ ID NO: 3) (e.g., about 10 to 150 cytosine nucleotides, about 10 to 100 cytosine nucleotides, about 20 to 70 cytosine nucleotides, about 20 to 60 cytosine nucleotides, or about 10 to 40 cytosine nucleotides). The poly-C tail may be added to the poly-A tail or may substitute the poly-A tail.

In some embodiments, the mRNA further comprises a 5' untranslated region (5' UTR) comprising a nucleotide sequence and positioned between the 5' cap structure and coding sequence, and/or a 3' untranslated region (3' UTR) comprising a nucleotide sequence and positioned between the coding sequence and the poly(A) tail structure. In some embodiments, a 5' untranslated region includes one or more elements that affect an mRNA's stability or translation, for example, an iron responsive element. In some embodiments, a 5' untranslated region may be between about 50 and 500 nucleotides in length.

In some embodiments, a 3' untranslated region includes one or more of a polyadenylation signal, a binding site for proteins that affect an mRNA's stability of location in a cell, or one or more binding sites for miRNAs. In some embodiments, a 3' untranslated region may be between 50 and 500 nucleotides in length or longer.

### Nucleotide modifications

It has been suggested that the use mRNA which has been prepared with modified nucleotides such as pseudouridine analogues and, in particular 1-methylpseudouridine, is essential for effectively inducing immune tolerance (WO2018/189193). The inventors have demonstrated that an mRNA prepared with unmodified nucleotides are equally effective at inducing immune tolerance to a peptide, polypeptide or protein encoded by said mRNA. Therefore, mRNAs according to the present invention are typically synthesized with unmodified nucleotides. These mRNAs are also referred to as unmodified mRNAs.

Typically, the nucleotides of an mRNA according to the present invention does not include, for example, backbone modifications, sugar modifications or base modifications. Specifically, the mRNAs according to the present invention typically do not contain modified nucleotides analogues or derivatives of purines and pyrimidines, such as e.g. 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queosine, .beta.-D-mannosyl-queosine, wybutoxosine, and phosphoramidates, phosphorothioates, peptide nucleotides, methylphosphonates, 7-deazaguanosine, 5-methylcytosine and inosine.

More specifically, the mRNAs of the invention typically do not contain uracils analogs such as pseudouridine and, in particular 1-methylpseudouridine. Pseudouridine is a C-glycoside isomer of the nucleoside uridine. Examples of pseudouridine analogs include but are not limited to 1-carboxymethyl-pseudouridine, 1-propynyl-pseudouridine, 1-taurinomethyl-pseudouridine, 1 -taurinomethyl-4-thio-pseudouridine, 1-methylpseudouridine , 1-methyl-4-thio-pseudouridine, 4-thio-1-methyl-pseudouridine, 3-methyl-pseudouridine, 2-thio-1 -methyl-pseudouridine, 1-methyl-1-deaza-pseudouridine, 2-thio-1 -methyl- 1-deaza-pseudouridine, dihydropseudouridine, 2-thio-dihydropseudouridine, 2-methoxyuridine, 2-methoxy-4-thio-uridine, 4-methoxy-pseudouridine, 4-methoxy-2-thio-pseudouridine, N -methyl-pseudouridine, 1-methyl-3-(3-amino-3-carboxypropyl)pseudouridine and 2'-0-methyl-pseudouridine.

In some embodiments, it may be advantageous to synthesize an mRNA of the present invention with one or more modified nucleotides. Typically, mRNAs are modified to enhance their stability or reduce their immunogenic properties, in particular when administered to a subject as naked mRNAs or in complexed form. Therefore, providing an mRNA of the present invention may have synergistic effects, resulting in the induction of immune tolerance that exceeds what has been observed with unmodified mRNAs.

Modifications of mRNA can include, for example, modifications of the nucleotides of the RNA. A modified mRNA according to the invention can thus include, for example, backbone modifications, sugar modifications or base modifications. In some embodiments, mRNAs may be synthesized from naturally occurring nucleotides and/or nucleotide analogues (modified nucleotides) including, but not limited to, purines (adenine (A), guanine (G)) or pyrimidines (thymine (T), cytosine (C), uracil (U)), and as modified nucleotides analogues or derivatives of purines and pyrimidines, such as e.g. 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queosine, .beta.-D-mannosyl-queosine, wybutoxosine, and phosphoramidates, phosphorothioates, peptide nucleotides, methylphosphonates, 7-deazaguanosine, 5-methylcytosine and inosine. The preparation of such analogues is known to a person skilled in the art e.g. from the U.S. Pat. No. 4,373,071, U.S. Pat. No. 4,401,796, U.S. Pat. No. 4,415,732, U.S. Pat. No. 4,458,066, U.S. Pat. No. 4,500,707, U.S. Pat. No. 4,668,777, U.S. Pat. No. 4,973,679, U.S. Pat. No. 5,047,524, U.S. Pat. No. 5,132,418, U.S. Pat. No. 5,153,319, U.S. Pat. Nos. 5,262,530 and 5,700,642, the disclosures of which are incorporated by reference in their entirety.

In some embodiments, mRNAs of the present invention may contain RNA backbone modifications. Typically, a backbone modification is a modification in which the phosphates of the backbone of the nucleotides contained in the RNA are modified chemically. Exemplary backbone modifications typically include, but are not limited to, modifications from the group consisting of methylphosphonates, methylphosphoramidates, phosphoramidates, phosphorothioates (e.g. cytidine 5'-O-(1-thiophosphate)), boranophosphates, positively charged guanidinium groups etc., which means by replacing the phosphodiester linkage by other anionic, cationic or neutral groups.

In some embodiments, mRNAs of the present invention may contain sugar modifications. A typical sugar modification is a chemical modification of the sugar of the nucleotides it contains including, but not limited to, sugar modifications chosen from the group consisting of 2'-deoxy-2'-fluoro-oligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

In some embodiments, mRNAs of the present invention may contain modifications of the bases of the nucleotides (base modifications). A modified nucleotide which contains a base modification is also called a base-modified nucleotide. Examples of such base-modified nucleotides include, but are not limited to, 2-amino-6-chloropurine riboside 5'-triphosphate, 2-aminoadenosine 5'-triphosphate, 2-thiocytidine 5'-triphosphate, 2-thiouridine 5'-triphosphate, 4-thiouridine 5'-triphosphate, 5-aminoallylcytidine 5'-triphosphate, 5-aminoallyluridine 5'-triphosphate, 5-bromocytidine 5'-triphosphate, 5-bromouridine 5'-triphosphate, 5-iodocytidine 5'-triphosphate, 5-iodouridine 5'-triphosphate, 5-methylcytidine 5'-triphosphate, 5-methyluridine 5'-triphosphate, 6-azacytidine 5'-triphosphate, 6-azauridine 5'-triphosphate, 6-chloropurine riboside 5'-triphosphate, 7-deazaadenosine 5'-triphosphate, 7-deazaguanosine 5'-triphosphate, 8-azaadenosine 5'-triphosphate, 8-azidoadenosine 5'-triphosphate, benzimidazole riboside 5'-triphosphate, N1-methyladenosine 5'-triphosphate, N1-methylguanosine 5'-triphosphate, N6-methyladenosine 5'-triphosphate, O6-methylguanosine 5'-triphosphate, pseudouridine 5'-triphosphate, puromycin 5'-triphosphate or xanthosine 5'-triphosphate.

### Codon optimization

In some embodiments, the coding regions of the mRNAs of the present invention are codon-optimized relative to the naturally occurring or wild-type coding regions that encode a peptide, polypeptide or protein for which induction of immune tolerance is desired in accordance with the methods disclosed herein. According to an increasing amount of research, mRNAs contain numerous layers of information that overlap the amino acid code. Traditionally, codon optimization has been used to remove rare codons which were thought to be rate-limiting for protein expression. While fast growing bacteria and yeast both exhibit strong codon bias in highly expressed genes, higher eukaryotes exhibit much less codon bias, making it more difficult to discern codons that may be rate-limiting. In addition, it has been found that codon bias *per se* does not necessarily yield high expression but requires other features.

For example, rare codons have been implicated in slowing translation and forming pause sites, which may be required for correct protein folding. Therefore, variations in codon usage may provide a mechanism to fine-tune the temporal pattern of elongation and thus increase the time available for a protein to take on its correct confirmation. Codon optimization can interfere with this fine-tuning mechanism, resulting in less efficient protein translation or an increased amount of incorrectly folded proteins. Similarly, codon optimization may disrupt the normal patterns of cognate and wobble tRNA usage, thereby affecting protein structure and function because wobble-dependent slowing of elongation may likewise have been selected as a mechanism for achieving correct protein folding.

### Cap structure

In some embodiments, mRNAs include a 5' cap structure. A 5' cap is typically added as follows: first, an RNA terminal phosphatase removes one of the terminal phosphate groups from the 5' nucleotide, leaving two terminal phosphates; guanosine triphosphate (GTP) is then added to the terminal phosphates via a guanylyl transferase, producing a 5'5'5 triphosphate linkage; and the 7-nitrogen of guanine is then methylated by a methyltransferase. Examples of cap structures include, but are not limited to, m7G(5')ppp (5'(A,G(5')ppp(5')A and G(5')ppp(5')G.

Naturally occurring cap structures comprise a 7-methyl guanosine that is linked via a triphosphate bridge to the 5'-end of the first transcribed nucleotide, resulting in a dinucleotide cap of m7G(5')ppp(5')N, where N is any nucleoside. In vivo, the cap is added enzymatically. The cap is added in the nucleus and is catalyzed by the enzyme guanylyl transferase. The addition of the cap to the 5' terminal end of RNA occurs immediately after initiation of transcription. The terminal nucleoside is typically a guanosine, and is in the reverse orientation to all the other nucleotides, i.e., G(5')ppp(5')GpNpNp.

A common cap for mRNA produced by in vitro transcription is m7G(5')ppp(5')G, which has been used as the dinucleotide cap in transcription with T7 or SP6 RNA polymerase in vitro to obtain RNAs having a cap structure in their 5'-termini. The prevailing method for the in vitro synthesis of capped mRNA employs a pre-formed dinucleotide of the form m7G(5')ppp(5')G ("m7GpppG") as an initiator of transcription.

To date, a usual form of a synthetic dinucleotide cap used in in vitro translation experiments is the Anti-Reverse Cap Analog ("ARCA") or modified ARCA, which is generally a modified cap analog in which the 2' or 3' OH group is replaced with -OCH3.

Additional cap analogs include, but are not limited to, a chemical structures selected from the group consisting of m7GpppG, m7GpppA, m7GpppC; unmethylated cap analogs (e.g., GpppG); dimethylated cap analog (e.g., m2,7GpppG), trimethylated cap analog (e.g., m2,2,7GpppG), dimethylated symmetrical cap analogs (e.g., m7Gpppm7G), or anti reverse cap analogs (e.g., ARCA; m7,2'OmeGpppG, m72'dGpppG, m7,3'OmeGpppG, m7,3'dGpppG and their tetraphosphate derivatives) (see, e.g., Jemielity, J. et al., "Novel 'anti-reverse' cap analogs with superior translational properties", RNA, 9: 1108-1122 (2003)).

In some embodiments, a suitable cap is a 7-methyl guanylate ("m7G") linked via a triphosphate bridge to the 5'-end of the first transcribed nucleotide, resulting in m7G(5')ppp(5')N, where N is any nucleoside. A preferred embodiment of a m7G cap utilized in embodiments of the invention is m7G(5')ppp(5')G.

In some embodiments, the cap is a Cap0 structure. Cap0 structures lack a 2'-O-methyl residue of the ribose attached to bases 1 and 2. In some embodiments, the cap is a Cap1 structure. Cap1 structures have a 2'-O-methyl residue at base 2. In some embodiments, the cap is a Cap2 structure. Cap2 structures have a 2'-O-methyl residue attached to both bases 2 and 3.

A variety of m7G cap analogs are known in the art, many of which are commercially available. These include the m7GpppG described above, as well as the ARCA 3'-OCH3 and 2'-OCH3 cap analogs (Jemielity, J. et al., RNA, 9: 1108-1122 (2003)). Additional cap analogs for use in embodiments of the invention include N7-benzylated dinucleoside tetraphosphate analogs (described in Grudzien, E. et al., RNA, 10: 1479-1487 (2004)), phosphorothioate cap analogs (described in Grudzien-Nogalska, E., et al., RNA, 13: 1745-1755 (2007)), and cap analogs (including biotinylated cap analogs) described in U.S. Patent Nos. 8,093,367 and 8,304,529, incorporated by reference herein.

### Tail structure

Typically, the presence of a "tail" serves to protect the mRNA from exonuclease degradation. The poly-A tail is thought to stabilize natural messengers and synthetic sense RNA. Therefore, in certain embodiments a long poly-A tail can be added to an mRNA molecule thus rendering the RNA more stable. Poly-A tails can be added using a variety of art-recognized techniques. For example, long poly-A tails can be added to synthetic or in vitro transcribed RNA using poly A polymerase (Yokoe, et al. Nature Biotechnology. 1996; 14: 1252-1256). A transcription vector can also encode long poly-A tails. In addition, poly-A tails can be added by transcription directly from PCR products. Poly-A may also be ligated to the 3' end of a sense RNA with RNA ligase (see, e.g., Molecular Cloning A Laboratory Manual, 2nd Ed., ed. by Sambrook, Fritsch and Maniatis (Cold Spring Harbor Laboratory Press: 1991 edition)).

In some embodiments, mRNAs include a 3' poly(A) tail structure. Typically, the length of the poly-A tail can be at least about 10, 50, 100, 200, 300, 400 or 500 nucleotides in length. In some embodiments, a poly-A tail on the 3' terminus of mRNA typically includes about 10 to 800 adenosine nucleotides (e.g., about 300 to 500 adenosine nucleotides, about 300 to 800 adenosine nucleotides, about 10 to 200 adenosine nucleotides, about 10 to 150 adenosine nucleotides, about 10 to 100 adenosine nucleotides, about 20 to 70 adenosine nucleotides, or about 20 to 60 adenosine nucleotides). In a specific embodiments, an mRNA suitable for use in the invention has a poly-A tail on the 3' terminus that has about 100 to 500 adenosine nucleotides Typically, a poly-A tail in an mRNA in accordance with the invention is about 300 to about 800 adenosine nucleotides long (SEQ ID NO: 4). More commonly, the poly-A tail is about 300 adenosine nucleotides long (SEQ ID NO:5).

In some embodiments, mRNAs include a 3' poly(C) tail structure. A suitable poly-C tail on the 3' terminus of mRNA typically include about 10 to 200 cytosine nucleotides (SEQ ID NO: 3) (e.g., about 10 to 150 cytosine nucleotides, about 10 to 100 cytosine nucleotides, about 20 to 70 cytosine nucleotides, about 20 to 60 cytosine nucleotides, or about 10 to 40 cytosine nucleotides). The poly-C tail may be added to the poly-A tail or may substitute the poly-A tail.

In some embodiments, the length of the poly A or poly C tail is adjusted to control the stability of a modified sense mRNA molecule of the invention and, thus, the transcription of protein. For example, since the length of the poly A tail can influence the half-life of a sense mRNA molecule, the length of the poly A tail can be adjusted to modify the level of resistance of the mRNA to nucleases and thereby control the time course of polynucleotide expression and/or polypeptide production in a target cell.

### 5' and 3' Untranslated Region

**In** some embodiments, mRNAs include a 5' untranslated region (UTR). In some embodiments, mRNAs include a 3' untranslated region. In some embodiments, mRNAs include both a 5' untranslated region and a 3' untranslated region. In some embodiments, a 5' untranslated region includes one or more elements that affect an mRNA's stability or translation, for example, an iron responsive element. In some embodiments, a 5' untranslated region may be between about 50 and 500 nucleotides in length.

In some embodiments, a 3' untranslated region includes one or more of a polyadenylation signal, a binding site for proteins that affect an mRNA's stability of location in a cell, or one or more binding sites for miRNAs. In some embodiments, a 3' untranslated region may be between 50 and 500 nucleotides in length or longer.

Exemplary 3' and 5' untranslated region sequences can be derived from mRNA molecules which are stable (e.g., globin, actin, GAPDH, tubulin, histone, or citric acid cycle enzymes) to increase the stability of the mRNA molecule. For example, a 5' UTR sequence may include a partial sequence of a CMV immediate-early 1 (IE1) gene, or a fragment thereof to improve the nuclease resistance and/or improve the half-life of the polynucleotide. Also contemplated is the inclusion of a sequence encoding human growth hormone (hGH), or a fragment thereof to the 3' end or untranslated region of the polynucleotide (e.g., mRNA) to further stabilize the polynucleotide. Generally, these modifications improve the stability and/or pharmacokinetic properties (e.g., half-life) of the polynucleotide relative to their unmodified counterparts, and include, for example modifications made to improve such polynucleotides' resistance to in vivo nuclease digestion.

In certain embodiments, an mRNA in accordance with the invention includes a coding region flanked by 5' and 3' untranslated regions as represented as X and Y, respectively (*vide infra*)

**X** - Coding Region - **Y**

wherein
X (5' UTR Sequence) is
GGACAGAUCGCCUGGAGACGCCAUCCACGCUGUUUUGACCUCCAUAGAAGACACCG GGACCGAUCCAGCCUCCGCGGCCGGGAACGGUGCAUUGGAACGCGGAUUCCCCGUGC CAAGAGUGACUCACCGUCCUUGACACG (**SEQ ID NO: 6**) or a sequence 70%, 75%, 80%, 85%, 90%, 91%, 92%, 93%, 94%, 95%, 96%, 97%, 98%, 99% or more identical to SEQ ID NO: 6; and where Y (3' UTR Sequence) is
CGGGUGGCAUCCCUGUGACCCCUCCCCAGUGCCUCUCCUGGCCCUGGAAGUUGCCAC UCCAGUGCCCACCAGCCUUGUCCUAAUAAAAUUAAGUUGCAUCAAGCU (**SEQ ID NO**: 7) or a sequence 70%, 75%, 80%, 85%, 90%, 91%, 92%, 93%, 94%, 95%, 96%, 97%, 98%, 99% or more identical to SEQ ID NO: 7, or
GGGUGGCAUCCCUGUGACCCCUCCCCAGUGCCUCUCCUGGCCCUGGAAGUUGCCACU CCAGUGCCCACCAGCCUUGUCCUAAUAAAAUUAAGUUGCAUCAAAGCU (**SEQ ID NO: 8**) or a sequence 70%, 75%, 80%, 85%, 90%, 91%, 92%, 93%, 94%, 95%, 96%, 97%, 98%, 99% or more identical to SEQ ID NO: 8.

### Liver-specific expression of mRNA

In some embodiments, the 5'UTR of the one or more mRNAs comprises a nucleic acid sequence for liver-specific expression. In some embodiments, the sequences that drive liver-specific expression are the 5' UTR sequences derived from RM1 mRNA (Orosomucoid 1), HPX mRNA (Hemoexin), FGA mRNA (Fibrinogen alpha chain), CYP2E12e1 mRNA (cytochrome P450 2E1), C3 mRNA (complement component 3), APOA2 mRNA (Apolipoprotein A-II), ALB mRNA (Albumin) or AGXT mRNA (Alanine-glyoxylate aminotransferase). In a specific embodiment, the mRNA comprises the 5' UTR sequence derived from FGA (Fibrinogen alpha chain) to drive high level protein expression in the liver. In some embodiments, the one or more mRNAs can contain two 5'UTR sequences that drive liver-specific expression of the coding sequence. For example, an mRNA in accordance with the invention may include 5'UTR sequences derived from the mRNAs encoding complement factor 3 (C3) and cytochrome p4502E1 (CYP2E1).

### Suppression of mRNA expression in hematopoietic cells

miRNA are small noncoding RNAs of around 19-25 nucleotides in length that can regulate gene expression by inhibiting translation or by messenger RNA degradation. Typically miRNAs interact with specific binding sites in the 3'UTR region of the mRNA. However, miRNA binding sites can also be located in the 5'UTR and the coding sequence of an mRNA. The introduction one or multiple binding sites for different miRNAs into the 5'UTR, coding sequence or 3'UTR region of the mRNA decreases the longevity, stability, and protein translation of polynucleotides. miRNA binding sites can be incorporated into the 5'UTR, coding sequence or 3'UTR region of the polynucleotides to decrease gene expression in a cell specific manner.

In certain embodiments, the one or more mRNAs comprise a nucleic acid sequence that prevents expression and/or induces degradation of the one or more mRNAs in a haematopoietic cell, optionally wherein the haematopoietic cell is an antigen-presenting cell. Specifically, one or more miRNA binding sites can be incorporated into the 5' UTR, coding region and/or 3' UTR of the mRNAs of the invention to decrease their expression in these cells. In other embodiments, one or more miRNA binding sites can be incorporated into 3' UTR of the mRNAs of the invention to decrease their expression in these cells.

For example, incorporation of miR-142 binding sites into a UGT1A1-expressing lentiviral vector has been shown to reduce expression in hematopoietic cells, and as a consequence, to reduce expression in antigen-presenting cells, leading to the absence of an immune response against the virally expressed UGT1A1 (Schmitt et al., Gastroenterology 2010; 139:999-1007; Gonzalez-Asequinolaza et al., Gastroenterology 2010, 139:726-729; both herein incorporated by reference in its entirety). Similarly, it has been shown that mir-142-3p target sequences can reduce transgene-directed immunogenicity following intramuscular adeno-associated virus 1 vector-mediated gene delivery (Majowicz et al., J Gene Med 2013; 15:219-232). Therefore, without wishing to be bound by any particular theory, the inventors consider that the incorporation of miR-142 binding sites, and in particular binding sites for miR-142-3p and/or miR-142-5p, into an mRNA of the invention is useful to reduce expression of the encoded peptide, polypeptide or protein in hematopoietic cells, and specifically antigen-presenting cells. As a consequence, the presence of these binding sites reduces or abolishes an immune responses to the mRNA-encoded peptide, polypeptide or protein, thereby tipping the scale towards induction of immune tolerance when a subject is exposed to the mRNA. miR-142-3p in particular has been identified as a miRNA that is exclusively expressed in hematopoietic lineage cells, and binding sites for this mRNA may be especially useful in practising the invention. Other miRNAs are known to be specific to hematopoietic cells are miR-142-5p, miR-144, miR-150, miR-155, miR-223, miR-21, miR-24. Incorporating binding sites for these miRNAs may likewise be advantageous when putting the invention into practice.

In some embodiments, an mRNA of the invention comprises a 3' UTR sequences with one or more miRNA binding sites that decrease its expression in hematopoietic lineage cells (in particular in antigen-presenting cells) as well as 5' UTR sequences that drive liver-specific expression.

### Sequence optimization

miR-122 is an abundant miRNA in liver, that is known to regulate hepatic cholesterol and lipid metabolism and has a central role in maintaining liver homeostasis. Other miRNAs that are known to target mRNAs in the liver include miR-33a/b, miR-34a, miR-29, miR-103, miR-107, miR-143 and miR-335 (Rottiers and Naar (2012) Nat Rev Mol Cell Biol 13(4): 239-250).

When preparing mRNAs for use with the invention, liver-specific miRNA binding sites can be removed to ensure that the mRNA is optimally expressed in the liver. In some embodiments, the mRNA of the invention, and in particular its 3'UTR, is optimized to remove potential binding sites for one or more of the following miRNAs: miR-122, miR-29, miR-33a/b, miR-34a, miR-92a, miR-92, miR-103, miR-107, miR-143, miR-335 and miR-483. In a specific embodiment, the 3'UTR region of the mRNA of the invention does not contain a miR-122 binding site.

In certain embodiments, the one or more mRNAs do not comprise a binding site for a liver-specific miRNA. In some embodiments, the liver-specific miRNA is one or more of miR-122, miR-29, miR-33a/b, miR-34a, miR-92a, miR-92, miR-103, miR-107, miR-143, miR-335 and miR-483.

### In vitro transcription

The mRNA of the invention is synthesized by in vitro transcription from a plasmid DNA template encoding the gene, which is followed by the addition of a 5' cap structure (Fechter, P.; Brownlee, G.G. "Recognition of mRNA cap structures by viral and cellular proteins" J. Gen. Virology 2005, 86, 1239-1249) and a 3' poly(A) tail of approximately 100, 200, 250, 300, 400, 500 or 800 nucleotides in length as determined by gel electrophoresis.

### Immune regulators

It has been suggested that immune regulators such as cytokines are required in order to effectively induce immune tolerance in a subject. For example, WO 2018/083111 suggests that co-expression of immune modifiers, such as TGF-β, IL-10 and IL-2 are required to achieve immune tolerance and WO 2016/036902 discloses that phosphatidylserine is essential to induce immune tolerance in a subject.

The inventors have demonstrated that unmodified mRNAs encapsulated in one or more liposomes, which are preferentially directed to the liver, are particularly effective at inducing immune tolerance in a subject without the need for co-administration of an immune regulator. Without wishing to be bound by any particular theory, the inventors believe that the expression of a peptide, polypeptide or protein in hepatocytes and/or liver sinusoidal endothelial cells is sufficient to induce tolerance. Therefore, in one aspect of the invention, the one or more mRNAs encoding the one or more peptides, polypeptides or proteins are the only therapeutic agents for inducing immune tolerance that are administered to the subject. Accordingly, in certain embodiments, the methods according to the present invention do not involve the administration of an immune regulator.

Specifically, the methods according to the present invention do not involve the administration of a cytokine that induces or enhances a Treg phenotype. This includes, *inter alia,* cytokines such as TGF-β, IL-10 and/or IL-2. In another specific embodiment of the invention, the methods according to the present invention do not involve the administration of a molecule that down-modulates the function of macrophages and/or dendritic cells. This includes phospholipids, in particular phosphatidylserine.

In certain aspects of the invention, it may be advantageous to administer the one or more mRNAs, encoding the one or more peptides, polypeptides or proteins, with an mRNA encoding an immune modulator. A suitable immune modulator acts on one or more cells of the immune system. The cell can either be a T-cell, such as a naive CD4+ cells, or an antigen-presenting cell of hematopoietic origin, such as a macrophage and/or a dendritic cell.

In one aspect of the invention, the methods disclosed herein comprise administering to the subject two sets of mRNAs. The first set includes one or more mRNAs encoding the one or more peptides, polypeptides or proteins and the second set includes one or more mRNAs encoding an immune modulator. In certain embodiments the second set of one or more mRNAs encodes one or more cytokines that induce or enhance a Treg phenotype. In certain embodiments, the one or more cytokines are select from TGF-β, IL-10 and IL-2, or a combination thereof. Suitable combinations include (i) TGF-β and IL-10, (ii) TGF-β and IL-2, and (iii) TGF-β, IL-10 and IL-2.

In another aspect of the invention, it may be advantageous to administer the one or more mRNAs encoding the one or more peptides, polypeptides or proteins, for which immune tolerance is desired, in liposomes that comprise a phospholipid that down-modulates the function of macrophages and/or dendritic cells. A suitable phospholipid is phosphatidylserine. Accordingly, in some embodiments, the methods of the invention comprise administering to the subject one or more mRNAs encoding the one or more peptides, polypeptides or proteins encapsulated in a liposomes comprising a phospholipid such as phosphatidylserine.

In a further aspect of the invention, the methods of the invention comprise administering, to a subject in need of immune tolerance induction, two sets of mRNAs encapsulated in liposomes comprising a phospholipid, such as phosphatidylserine. In certain embodiments, the first set of mRNAs include one or more mRNAs encoding the one or more peptides, polypeptides or proteins, and the second set of mRNAs include one or more mRNAs encoding an immune modulator, such as a cytokine that induces or enhances a Treg phenotype.

### Pharmaceutical Compositions

The inventors have identified that one or more mRNAs comprising a 5'UTR, a coding region and a 3'UTR, wherein the one or more coding regions of the one or more mRNAs encode the one or more peptides, polypeptides or proteins, wherein the one or more mRNAs are encapsulated in one or more liposomes, does not require any additional therapeutic agents to induce immune tolerance to one or more peptides, polypeptides or proteins in a subject. Therefore in certain embodiments, the one or more mRNAs encoding the one or more peptides, polypeptides or proteins are the only therapeutic agents for inducing immune tolerance that are administered to the subject. In certain embodiments, the method does not involve the administration of an immune regulator. In certain embodiments, the immune regulator is a cytokine or phosphatidylserine.

Clinical or therapeutic candidate mRNA formulations are selected from the exemplary codon-optimized mRNA sequences having a 5'-cap and a 3'-poly A tail, which is formulated in a suitable lipid combination as described above. Clinical relevant mRNA candidates are characterized by efficient delivery and uptake by the liver, high level of expression and sustained protein production, without detectable adverse effects in the subject to whom the therapeutic is administered, either caused by the pharmacologically active ingredient or by the lipids in the liposome, or by any excipients used in the formulation. In general, high efficiency with low dose administration is favourable for the selection process of a relevant candidate therapeutic.

To facilitate expression of mRNA in vivo, liposomes can be formulated in combination with one or more additional nucleic acids, carriers, targeting ligands or stabilizing reagents, or in pharmacological compositions where it is mixed with suitable excipients. Techniques for formulation and administration of drugs may be found in "Remington's Pharmaceutical Sciences," Mack Publishing Co., Easton, Pa., latest edition.

Provided liposomally-encapsulated mRNAs and compositions containing the same, may be administered and dosed in accordance with current medical practice, taking into account the clinical condition of the subject, the site and method of administration, the scheduling of administration, the subject's age, sex, body weight and other factors relevant to clinicians of ordinary skill in the art. As used herein, the term "therapeutically effective amount" is largely determined based on the total amount of the therapeutic agent contained in the pharmaceutical compositions of the present invention. Generally, a therapeutically effective amount is sufficient to achieve a meaningful benefit to the subject, the mammal, (e.g., inducing immune tolerance to a peptide, polypeptide or protein). For example, a therapeutically effective amount may be an amount sufficient to achieve a desired therapeutic and/or prophylactic effect. Generally, the amount of a therapeutic agent (e.g., mRNA encoding a peptide, polypeptide or protein for inducing immune tolerance) administered to a subject in need thereof will depend upon the characteristics of the subject. Such characteristics include the condition, disease severity, general health, age, sex and body weight of the subject. One of ordinary skill in the art will be readily able to determine appropriate dosages depending on these and other related factors. In addition, both objective and subjective assays may optionally be employed to identify optimal dosage ranges.

In some embodiments, the therapeutically effective dose ranges from about 0.005 mg/kg body weight to 500 mg/kg body weight, e.g., from about 0.005 mg/kg body weight to 400 mg/kg body weight, from about 0.005 mg/kg body weight to 300 mg/kg body weight, from about 0.005 mg/kg body weight to 200 mg/kg body weight, from about 0.005 mg/kg body weight to 100 mg/kg body weight, from about 0.005 mg/kg body weight to 90 mg/kg body weight, from about 0.005 mg/kg body weight to 80 mg/kg body weight, from about 0.005 mg/kg body weight to 70 mg/kg body weight, from about 0.005 mg/kg body weight to 60 mg/kg body weight, from about 0.005 mg/kg body weight to 50 mg/kg body weight, from about 0.005 mg/kg body weight to 40 mg/kg body weight, from about 0.005 mg/kg body weight to 30 mg/kg body weight, from about 0.005 mg/kg body weight to 25 mg/kg body weight, from about 0.005 mg/kg body weight to 20 mg/kg body weight, from about 0.005 mg/kg body weight to 15 mg/kg body weight, from about 0.005 mg/kg body weight to 10 mg/kg body weight.

The "effective dose or effective amount" for the purposes herein may be determined by such relevant considerations as are known to those of ordinary skill in experimental clinical research, pharmacological, clinical and medical arts.

A therapeutic low dose is a dose that is less than the maximal effective dose in the subject but is a dose that shows therapeutic effectiveness. Determining a therapeutic low dose is important in developing a formulation into a drug. A therapeutic low dose may be higher than the minimal effective low dose. A therapeutic low dose may be in the range where the dose is optimally effective without causing any adverse effect.

In some embodiments, an effective therapeutic low dose is administered to the mammal wherein the therapeutic low dose of the pharmaceutical composition comprising one or more mRNAs encoding one or more peptides, polypeptides or proteins is administered at a dosing interval sufficient to induce immune tolerance.

In some embodiments, the one or more encapsulated mRNAs encoding one or more peptides, polypeptides or proteins are administered at a dosing interval of once a day, twice a week, three times a week, once a week, once every two weeks or once a month. In preferred embodiments, the one or more encapsulated mRNAs encoding one or more peptides, polypeptides or proteins are administered at a dosing interval once every three days.

In some embodiments, the only one dose is required to induce immune tolerance. In other embodiments, multiple doses are required to induce immune tolerance. In some embodiments, the one or more encapsulated mRNAs encoding one or more peptides, polypeptides or proteins is administered for one week, two weeks, three weeks, four weeks, five weeks, six weeks, seven weeks or eight weeks. In some embodiments, the dosing interval is once a month. In some embodiments, the dosing interval is once in every two months. In some embodiments, the dosing interval is once every three months, or once every four months or once every five months or once every six months or anywhere in between.

In some embodiments, an additional dose of the one or more encapsulated mRNAs encoding one or more peptides, polypeptides or proteins is administered 6 months to 1 years after the first dose. In some embodiments, an additional dose of the one or more encapsulated mRNAs encoding one or more peptides, polypeptides or proteins is administered at 6 months after the first dose.

In some embodiments the mammal is a human. A suitable therapeutic dose that may be applicable for a human being can be derived based on animal studies. A basic guideline for deriving a human equivalent dose from studies performed in animals can be obtained from the U.S> Food and Drug Administration (FDA) website at https://www.fda.gov/downloads/drugs/guidances/ucm078932.pdf, entitled, "Guidance for Industry Estimating the Maximum Safe Starting Dose in Initial Clinical Trials for Therapeutics in Adult Healthy Volunteers." Based on the guidelines for allometric scaling, a suitable dose of, for example, 0.6 mg/kg in a mouse, would relate to a human equivalent dose of 0.0048 mg/kg. Thus, considering the derived human equivalent dose, a projected human therapeutic dose can be derived based on studies in other animals.

In some embodiments, a pharmaceutical composition comprising a 10-1000 µg dose of the one or more encapsulated mRNAs encoding one or more peptides, polypeptides or proteins is administered to a subject. Typically, a pharmaceutical composition comprising a 50 µg, 75 µg, 100 µg, 200 µg, 300 µg, 400 µg or 800 µg dose of the one or more encapsulated mRNAs encoding one or more peptides, polypeptides or proteins is administered to a subject. In a preferred embodiment, a pharmaceutical composition comprising a dose of 50 µg to 500 µg of the one or more encapsulated mRNAs encoding one or more peptides, polypeptides or proteins (e.g., 75 µg, 150 µg, 350 µg) is administered to a subject. In the most preferred embodiment, a pharmaceutical composition comprising a dose of 100 µg to 250 µg the one or more encapsulated mRNAs encoding one or more peptides, polypeptides or proteins is administered to a subject.

Suitable routes of administration include, for example, oral, rectal, vaginal, transmucosal, pulmonary including intratracheal or inhaled, or intestinal administration; parenteral delivery, including intradermal, intramuscular, subcutaneous, intramedullary injections, as well as intrathecal, direct intraventricular, intravenous, intraperitoneal, or intranasal. The administration results in delivery of the mRNA to a hepatocyte (i.e., liver cell).

In preferred embodiments, the therapeutically effective dose comprising the one or more encapsulated mRNAs encoding one or more peptides, polypeptides or proteins is administered intravenously to the subject.

In some embodiments, the therapeutically effective dose comprising the one or more encapsulated mRNAs encoding one or more peptides, polypeptides or proteins is administered to the subject by intramuscular administration. In particular embodiments, the intramuscular administration is to a muscle selected from the group consisting of skeletal muscle, smooth muscle and cardiac muscle.

Most commonly, the therapeutically effective dose comprising the one or more encapsulated mRNAs encoding one or more peptides, polypeptides or proteins is administered to the subject by intravenous administration.

Alternatively or additionally, liposomally encapsulated mRNAs and compositions of the invention may be administered in a local rather than systemic manner, for example, via injection of the pharmaceutical composition directly into the liver, preferably in a sustained release formulation. Formulations containing provided compositions complexed with therapeutic molecules or ligands can even be surgically administered, for example in association with a polymer or other structure or substance that can allow the compositions to diffuse from the site of implantation to surrounding cells. Alternatively, they can be applied surgically without the use of polymers or supports.

In particular embodiments, the one or more encapsulated mRNAs encoding one or more peptides, polypeptides or proteins is administered intravenously, wherein intravenous administration is associated with delivery of the mRNA to hepatocytes.

A therapeutically effective dose comprising the one or more encapsulated mRNAs encoding one or more peptides, polypeptides or proteins is administered for suitable delivery to the mammal's liver. A therapeutically effective dose comprising the one or more encapsulated mRNAs encoding one or more peptides, polypeptides or proteins is administered for suitable expression in hepatocytes of the administered mammal.

Provided methods of the present invention contemplate single as well as multiple administrations of a therapeutically effective amount of the therapeutic agents (e.g., mRNA encoding peptides, polypeptides or proteins that induce immune tolerance) described herein. Therapeutic agents can be administered at regular intervals, depending on the nature, severity and extent of the subject's condition. In some embodiments, a therapeutically effective amount of the mRNA encoding a peptide, polypeptide or protein of the present invention may be administered intravenously periodically at regular intervals (e.g., once every year, once every six months, once every five months, once every three months, bimonthly (once every two months), monthly (once every month), biweekly (once every two weeks), twice a month, once every 30 days, once every 28 days, once every 14 days, once every 10 days, once every 7 days, weekly, twice a week, daily or continuously).

In some embodiments, provided liposomes and/or compositions are formulated such that they are suitable for extended-release of the mRNA contained therein. Such extended-release compositions may be conveniently administered to a subject at extended dosing intervals. For example, in one embodiment, the compositions of the present invention are administered to a subject twice a day, daily or every other day. In some embodiments, the compositions of the present invention are administered to a subject twice a week, once a week, once every 7 days, once every 10 days, once every 14 days, once every 28 days, once every 30 days, once every two weeks, once every three weeks, once every four weeks, once a month, twice a month, once every six weeks, once every eight weeks, once every other month, once every three months, once every four months, once every six months, once every eight months, once every nine months or annually.

In some embodiments the mRNA is administered concurrently with an additional therapy. In some embodiments, the concurrent therapy is protein replacement therapy. In some embodiments, the protein replacement therapy is Factor VIII. In some embodiments, the protein replacement therapy is insulin.

Also contemplated are compositions and liposomes which are formulated for depot administration (e.g., intramuscularly, subcutaneously, intravitreally) to either deliver or release an mRNA over extended periods of time. Preferably, the extended-release means employed are combined with modifications made to the mRNA to enhance stability.

A therapeutically effective amount is commonly administered in a dosing regimen that may comprise multiple unit doses. For any particular therapeutic protein, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the patient; the time of administration, route of administration, and/or rate of excretion or metabolism of the specific protein employed; the duration of the treatment; and like factors as is well known in the medical arts.

Also contemplated herein are lyophilized pharmaceutical compositions comprising one or more of the liposomes disclosed herein and related methods for the use of such compositions as disclosed for example, in International Patent Application PCT/US12/41663, filed June 8, 2012, the teachings of which are incorporated herein by reference in their entirety. For example, lyophilized pharmaceutical compositions according to the invention may be reconstituted prior to administration or can be reconstituted in vivo. For example, a lyophilized pharmaceutical composition can be formulated in an appropriate dosage form (e.g., an intradermal dosage form such as a disk, rod or membrane) and administered such that the dosage form is rehydrated over time in vivo by the individual's bodily fluids.

In some embodiments, the pharmaceutical composition comprises a lyophilized liposomal delivery vehicle that comprises a cationic lipid, a non-cationic lipid, a PEG-modified lipid and cholesterol. In some embodiments, the pharmaceutical composition has a Dv50 of less than 500nm, 300nm, 200nm, 150nm, 125nm, 120nm, 100nm, 75nm, 50nm, 25nm or smaller upon reconstitution. In some embodiments, the pharmaceutical composition has a Dv90 of less than 750nm, 700nm, 500nm, 300nm, 200nm, 150nm, 125nm, 100nm, 75nm, 50nm, 25nm or smaller upon reconstitution. In some embodiments, the pharmaceutical composition has a polydispersity index value of less than 1, 0.95, 0.9, 0.8, 0.75, 0.7, 0.6, 0.5, 0.4, 0.3, 0.25, 0.2, 0.1, 0.05 or less upon reconstitution. In some embodiments, the pharmaceutical composition has an average particle size of less than 500nm, 400nm, 300nm, 200nm, 175nm, 150nm, 125nm, 100nm, 75nm, 50nm, 25nm or upon reconstitution.

In some embodiments, the lyophilized pharmaceutical composition further comprises one or more lyoprotectants, such as sucrose, trehalose, dextran or inulin. Typically, the lyoprotectant is sucrose. In some embodiments, the pharmaceutical composition is stable for at least 1 month or at least 6 months upon storage at 4°C, or for at least 6 months upon storage at 25°C. In some embodiments, the biologic activity of the mRNA of the reconstituted lyophilized pharmaceutical composition exceeds 75% of the biological activity observed prior to lyophilization of the composition.

Provided liposomes and compositions may be administered to any desired tissue, but the mRNA is expressed in the liver.

According to various embodiments, the timing of expression of delivered mRNAs can be tuned to suit a particular medical need. In some embodiments, the expression of the protein encoded by delivered mRNA is detectable 1, 2, 3, 6, 12, 24, 48, 72, 96 hours, 1 week, 2 weeks, or 1 month after administration of provided liposomes and/or compositions.

In some embodiments the subject is a mammal. In some embodiments, the mammal is an adult. In some embodiments the mammal is an adolescent. In some embodiments the mammal is an infant or a young mammal. In some embodiments, the mammal is a primate. In some embodiments the mammal is a human. In some embodiments the subject is 6 years to 80 years old.

The following further embodiments are provided:
Embodiment 1. A method for inducing immune tolerance to one or more peptides, polypeptides or proteins in a subject in need thereof, wherein said method comprises administering to the subject one or more mRNAs, each mRNA comprising a 5'UTR, a coding region and a 3'UTR, wherein the one or more coding regions of the one or more mRNAs encode the one or more peptides, polypeptides or proteins, wherein said one or more mRNAs are encapsulated in one or more liposomes, wherein upon administration the one or more liposomes are preferentially delivered to the liver of the subject, wherein the nucleotides of the one or more mRNAs are unmodified.
Embodiment 2. The method of Embodiment 1, wherein the one or more mRNAs encoding the one or more peptides, polypeptides or proteins are the only therapeutic agents for inducing immune tolerance that are administered to the subject.
Embodiment 3. The method of Embodiment 1 or 2, wherein each of the one or more mRNAs comprise a nucleic acid sequence that prevents expression and/or induces degradation of the one or more mRNAs in a haematopoietic cell, optionally wherein the haematopoietic cell is an antigen-presenting cell.
Embodiment 4. The method of Embodiment 3, wherein the nucleic acid sequence is in the 3' UTR.
Embodiment 5. The method of Embodiment 3 or 4, wherein the nucleic acid sequence comprises one or more binding sites for miR-142-3p and/or miR-142-5p.
Embodiment 6. The method of any one of the preceding Embodiments, wherein the method does not involve the administration of an immune regulator.
Embodiment 7. The method of Embodiment 6, wherein the immune regulator is a cytokine or phosphatidylserine.
Embodiment 8. The method of any one of the Embodiments, wherein the one or more liposomes comprise one or more cationic lipids, one or more non-cationic lipids, one or more cholesterol-based lipids and one or more PEG-modified lipids.
Embodiment 9. The method of any one of the preceding Embodiments, wherein the one or more cationic lipids are selected from the group consisting of DOTAP (1,2-dioleyl-3-trimethylammonium propane), DODAP (1,2-dioleyl-3-dimethylammonium propane) , DOTMA (1,2-di-0-octadecenyl-3-trimethylammonium propane), DLinKC2DMA, DLin-KC2-DM, C12-200, cKK-E12 (3,6-bi s(4-(bis(2-hydroxydodecyl)amino)butyl)piperazine-2, 5 -dione), HGT5000, HGT5001 , HGT4003, ICE, OF-02 and combinations thereof.
Embodiment 10. The method of any one of the preceding Embodiments, wherein the one or more non-cationic lipids are selected from DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine), DOPG (1,2-dioleoyl-sn-glycero-3-phospho-(1'-rac-glycerol)) or combinations thereof.
**Embodiment 11.** The method of any one of the preceding Embodiments, wherein the one or more cholesterol-based lipids is cholesterol or PEGylated cholesterol.
Embodiment 12. The method of any one of the preceding Embodiments, wherein the one or more PEG-modified lipids comprise a poly(ethylene) glycol chain of up to 5 kDa in length covalently attached to a lipid with alkyl chain(s) of C₆-C₂₀ length.
Embodiment 13. The method of any one of the preceding Embodiments, wherein the cationic lipid constitutes about 30-60 % of the liposome by molar ratio.
Embodiment 14. The method of Embodiment 9, wherein the cationic lipid constitutes about 30%, 40 %, 50%, or 60% of the liposome by molar ratio.
Embodiment 15. The method of any one of the preceding Embodiments, wherein the ratio of cationic lipids:non-cationic lipids:cholesterol lipids:PEGylated lipids is approximately 40:30:20:10 by molar ratio.
Embodiment 16. The method of any one of the preceding Embodiments, wherein the ratio of cationic lipids:non-cationic lipids:cholesterol lipids:PEGylated lipids is approximately 40:30:25:5 by molar ratio.
Embodiment 17. The method of any one of the preceding Embodiments, wherein the ratio of cationic lipids:non-cationic lipids:cholesterol lipids:PEGylated lipids is approximately 40:32:25:3 by molar ratio.
Embodiment 18. The method of any one of the preceding Embodiments, wherein the ratio of cationic lipids:non-cationic lipids:cholesterol lipids:PEGylated lipids is approximately 50:25:20:5 by molar ratio.
Embodiment 19. The method of any one of the preceding Embodiments, wherein the one or more liposomes comprises cKK-E12, C12-200, HGT4003, HGT5001, HGT5000, DLinKC2DMA, DODAP or DODMA as the cationic lipid, DOPE as the non-cationic lipid, cholesterol as the neutral lipid, and DMG-PEG2K as the PEG-modified lipid.
Embodiment 20. The method of Embodiment 19, wherein the one or more liposomes comprises cKK-E12, DOPE, cholesterol and DMG-PEG2K.
Embodiment 21. The method of any one of Embodiments 1-7, wherein the one or more liposomes comprises a cholesterol-derived cationic lipid, a non-cationic lipid, and a PEG-modified lipid.
Embodiment 22. The method of Embodiment 21, wherein the one or more liposomes comprises ICE, DOPE and DMG-PEG2K.
Embodiment 23. The method of any one of the preceding Embodiments, wherein one or more liposomes have a size of about 80 nm to 120 nm, optionally wherein the liposome has a size of about 100 nm or less than 100 nm.
Embodiment 24. The method of any one of the preceding Embodiments, wherein the 5'UTR of the one or more mRNAs comprises a nucleic acid sequence for liver-specific expression.
Embodiment 25. The method of Embodiment 24, wherein the nucleic acid sequence for liver-specific expression is a sequence from the 5' UTR of FGA (Fibrinogen alpha chain) mRNA, complement factor 3 (C3) mRNA or cytochrome p4502E1 (CYP2E1) mRNA.
Embodiment 26. The method any of the preceding Embodiments, wherein the one or more mRNAs do not comprise a binding site for a liver-specific miRNA.
Embodiment 27. The method of Embodiment 26, wherein the liver-specific miRNA is one or more of miR-122, miR-29, miR-33a/b, miR-34a, miR-92a, miR-92, miR-103, miR-107, miR-143, miR-335 and miR-483.
Embodiment 28. The method of any one of the preceding Embodiments, wherein the subject suffers from an autoimmune response mounted against or triggered by the one or more peptides, polypeptides or proteins.
Embodiment 29. The method of any one of the preceding Embodiments, wherein the one or more peptides, polypeptides or proteins are or are derived from a self-antigen listed in Table 1.
Embodiment 30. The method of any one of the preceding Embodiments, wherein the method reduces the levels of autoreactive CD4+ T helper cells and/or CD8+ T cells specific for the one or more peptides, polypeptides or proteins.
Embodiment 31. The method of any one of the preceding Embodiments, wherein the method reduces the levels of B cells that produce autoantibodies specific for the one or more peptides, polypeptides or proteins
Embodiment 32. The method of any one of the preceding Embodiments, wherein the method increases the levels of T regulatory cells (Tregs), in particular CD4+CD25+FOXP3+ Tregs, that are specific for the one or more peptides, polypeptides or proteins.
Embodiment 33. The method of any one of the preceding Embodiments, wherein the subject suffers from an autoimmune disease selected from type I diabetes, celiac disease, multiple sclerosis, rheumatoid arthritis, systemic lupus erythematosus, primary biliary cirrhosis, myasthenia gravis, neuromyelitis optica, or Graves' disease.
Embodiment 34. The method of Embodiment 33, wherein the autoimmune disease is type I diabetes.
Embodiment 35. The method of Embodiment 34, wherein the one or more peptides, polypeptides or proteins are or are derived from proinsulin.
Embodiment 36. The method of Embodiment 34 or 35, wherein the method reduces and/or eliminates the autoimmune response to the subject's β-cells.
Embodiment 37. The method of Embodiment 33, wherein the autoimmune disease is celiac disease.
Embodiment 38. The method of Embodiment 37, wherein the one or more peptides, polypeptides or proteins are or are derived from tTG or ACT1.
Embodiment 39. The method of any one of Embodiments 1-27, wherein the subject suffers from a protein deficiency and the one or more peptides, polypeptides or proteins are or are derived from a replacement protein that is or will be administered to the subject to treat the protein deficiency.
Embodiment 40. The method Embodiment 39, wherein the subject has been treated with and produces antibodies against the replacement protein.
Embodiment 41. The method of Embodiment 39 or 40, wherein the protein deficiency and the corresponding replacement protein are selected from Table 2.
Embodiment 42. The method of Embodiment 39 or 40, wherein the protein deficiency is selected from haemophilia A or B, a lysosomal storage disorder, a metabolic disorder and an α-antitrypsin deficiency.
Embodiment 43. The method of Embodiment 42, wherein the protein deficiency is a metabolic disorder.
Embodiment 44. The method of Embodiment 43, wherein the metabolic disorder and the corresponding replacement protein are selected from Table 3.
Embodiment 45. The method of Embodiment 42, wherein the protein deficiency is haemophilia A and the one or more peptides, polypeptides or proteins are or are derived from Factor VIII.
Embodiment 46. The method of any one of Embodiments 1-27, wherein the subject suffers from an allergy triggered by the one or more peptides, polypeptides or proteins.
Embodiment 47. The method of Embodiment 46, wherein the method reduces or eliminates the subject's allergic response to the one or more peptides, polypeptides or proteins.
Embodiment 48. The method of Embodiment 46-47, wherein the one or more peptides, polypeptides or proteins are or are derived from an allergen listed in Table 4.

### EXAMPLES

### Example 1: Immune tolerance induction via liver-targeted mRNA therapy

Immune regulation in the liver is largely controlled by unique populations of conventional antigen presenting cells, such as macrophages and dendritic cells, but also unconventional antigen presenting cells including Kupffer cells, liver sinusoidal endothelial cells (LSECs), hepatic stellate cells and hepatocytes that express only low levels of MHC-I/MHC-II. The LSECs form a physical barrier between the intraluminal space and the subendothelial space of Dissé, and shield the hepatocytes from the sinusoidal blood (Figure 1).

LSECs regulate the immune response by the selective recruitment of hepatic leukocytes and the activation of both naive CD4+ and CD8+ T cells. The hepatocyte response to an antigen depends on the antigen load as shown in Figure 2. If the initial hepatocellular antigen load is low, then an effector CD8+ T-cell response is initiated, whereas if the antigen load exceeds a certain threshold it leads to CD8+ T-cell exhaustion and silence, and the induction of T cells which express high levels of PD-1 is initiated.

As shown in Figure 3, T-cell priming in hepatocytes is different to conventional antigen presenting cells in the lymph nodes. In the lymph nodes dendritic cell-mediated T-cell priming results in the expansion and activation of T-cells. In contrast, hepatocytes induce antigen-specific activation and proliferation of naive CD8+ T cells, which is independent of co-stimulatory signals, and leads to the premature death of T cells. This death by neglect response is a pivotal mechanism to induce peripheral tolerance to an antigen (Horst et al. (2016) Cellular & Molecular Immunology 13, 277-292).

This unique population of antigen presenting cells in the liver leads to the regulation of local and systematic tolerance to both self and foreign antigens. Without wishing to be bound by any particular theory, the inventors have concluded that directing the expression of a peptide, polypeptide or protein to the hepatocytes and/or liver sinusoidal endothelial results in immune tolerance to the peptide, polypeptide or protein.

Restricting the expression of mRNAs to hepatocytes and liver sinusoidal endothelial cells may be particularly effective at inducing antigen-specific immunologic tolerance. In order to avoid the expression of mRNA in antigen-presenting cells of hematopoietic origin (such as dendritic cells and macrophages), it may therefore be useful to design mRNAs whose expression is restricted to non-hematopoietic cells (such as hepatocytes and liver sinusoidal endothelial cells). This can be achieved through the incorporation of miRNA binding sites into the 3'UTR of the mRNA. miRNA-142 is specifically expressed in hematopoietic stem cell lineages.

mRNAs were designed with the following structure to ensure the specific expression of the peptide, polypeptide or protein in non-hematopoietic cells:
5' viral UTR-coding sequence of a peptide, polypeptide or protein - optionally 4 microRNA 142 binding sites - 3' UTR.

Only non-modified nucleotides were used to prepare the mRNAs by in vitro transcription.

### Example 2: Administration of mRNA encoding proinsulin can induce immune tolerance in patients with Type 1 diabetes

This study is designed to test the effect of the administration of encapsulated mRNA encoding murine proinsulin on the development and progression of type 1 diabetes in glucose intolerant non-obese diabetic (NOD) mice. Type 1 diabetes is characterized by the T-cell mediated destruction of the insulin-producing beta cells of the pancreas. The NOD mice are a good model for the study of type 1 diabetes, because unlike many autoimmune disease models, the mice spontaneously develop the disease. The median age for females to become diabetic is 18 weeks.

### Methods

Encapsulated mRNA encoding murine proinsulin is prepared as described in WO 2018/089801 and administered to ten-week old female NOD mice through intravenous injections three times a week.

In order to assess the ability of encapsulated mRNA encoding murine proinsulin to prevent the development of type 1 diabetes, halt the progression of the disease, and reverse the disease, the encapsulated mRNA encoding the murine proinsulin is administered to NOD mice and disease progression is monitored. Untreated and mock-treated NOD mice act as a controls for the experiment.

The stages of prediabetes and diabetes is determined using blood glucose levels and glucose tolerance tests. The effects of the treatment on the progression of hyperglycaemia and glucose tolerance are monitored throughout the experiment. The level of anti-insulin antibodies is also measured. A population of the mice is sacrificed and the pancreas of each harvested to determine the number of infiltrating CD4⁺ and CD8⁺ lymphocytes present in the islets. In addition, the spleens from the mice are harvested and the reactivity of CD4⁺ and CD8⁺ T-cell lymphocytes, as well as the regulator Treg cells towards proinsulin is determined using an ELISpot assay. An ELISpot assay is also used to determine immune activity in splenocytes.

### Results

NOD mice administered with encapsulated mRNA encoding murine proinsulin have no additional loss of glycemic control and a reduction in the anti-insulin antibody titers relative to control mice. There is no islet infiltration by T-cell CD4⁺ and CD8⁺ lymphocytes, and there is a reduction of the T-cell CD4⁺ and CD8⁺ reactivity towards proinsulin.

NOD mice administered with encapsulated mRNA encoding murine proinsulin after the development of hyperglycemia, but retain functional Langerhans cells are found to revert to a normal glycemic state, with a concomitant reduction in anti-insulin antibody titers as well as a decrease in the CD4⁺ and CD8⁺ T-cell lymphocyte reactivity towards proinsulin. In all cases there is an increase in the Treg cells that are reactive towards proinsulin.

These data indicate that the administration of encapsulated mRNA encoding murine proinsulin is able to both halt the progression of type 1 diabetes as well as reverse the disease in a murine model.

### Example 3: Protein deficiency, Factor IX inhibitors

The development of neutralizing alloantibodies towards an antigen is a significant complication in protein replacement therapy. This study is designed to assess whether administration of encapsulated mRNA encoding human factor IX (FIX) is able to promote immune tolerance induction towards the FIX antigen.

### Methods

Mice are immunized with human Factor IX (FIX). The immunized mice are challenged with the FIX antigen and the establishment of immunity towards FIX is determined by measuring the anti-FIX antibody titers and circulating half-life of FIX in the blood. In addition, the spleens of the mice are harvested, and the reactivity of CD4⁺ and CD8⁺ T-cell lymphocytes and Treg cells towards FIX is determined by an ELISpot assay.

Once mice are immune against FIX, encapsulated mRNA encoding FIX is prepared as described in WO 2018/089801 and administered through 3 times weekly intravenous injections. Mice treated with control mRNA that does not encode a polypeptide act as controls. The immune response towards FIX is determined by measuring the anti-FIX antibody titers and circulating half-life of FIX in the blood. In addition, the spleens of the mice are harvested, and the reactivity of CD4⁺ and CD8⁺ T-cell lymphocytes and Treg cells towards FIX is determined by an ELISpot assay.

### Results

Mice that have been immunized towards FIX and are subsequently administered encapsulated mRNA encoding FIX display a dampened immune response towards FIX in comparison to control mice. They have a decrease in the anti-FIX antibody titers and an elevated circulating half-life of FIX. The CD4⁺ and CD8⁺ T-cell lymphocyte reactivity towards FIX are reduced, while Tregs with reactivity towards FIX is increased.

This example demonstrates that the administration of encapsulated mRNA encoding human factor IX can effectively induce immune tolerance induction towards the FIX antigen.

### EQUIVALENTS

Those skilled in the art will recognize, or be able to ascertain using no more than routine experimentation, many equivalents to the specific embodiments of the invention described herein. The scope of the present invention is not intended to be limited to the above Description, but rather is as set forth in the following claims.

The articles "a" and "an" as used herein in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or the entire group members are present in, employed in, or otherwise relevant to a given product or process. Furthermore, it is to be understood that the invention encompasses all variations, combinations, and permutations in which one or more limitations, elements, clauses, descriptive terms, etc., from one or more of the listed claims is introduced into another claim dependent on the same base claim (or, as relevant, any other claim) unless otherwise indicated or unless it would be evident to one of ordinary skill in the art that a contradiction or inconsistency would arise. Where elements are presented as lists, (e.g., in Markush group or similar format) it is to be understood that each subgroup of the elements is also disclosed, and any element(s) can be removed from the group. It should be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not in every case been specifically set forth in so many words herein. It should also be understood that any embodiment or aspect of the invention can be explicitly excluded from the claims, regardless of whether the specific exclusion is recited in the specification. The publications, websites and other reference materials referenced herein to describe the background of the invention and to provide additional detail regarding its practice are hereby incorporated by reference.

## Claims

1. An mRNA comprising a 5' UTR, a coding region, and a 3' UTR, wherein:
the nucleotides of the mRNA are unmodified or modified;
the mRNA comprises at least one nucleic acid sequence in the 5' UTR, the coding region, and/or the 3' UTR for preventing expression and/or inducing degradation of the mRNA in a haematopoietic cell that comprises one or more binding sites for miR-142, miR-144, miR-150, miR-155, miR-223, miR-21, or miR-24; and
the coding region encodes a self-antigen involved in an auto-immune disease, an allergen, or a replacement protein used to treat a protein deficiency;
wherein the mRNA is encapsulated in a liposome which is delivered to the liver of a subject, and the liposome comprises:
(a) one or more cationic lipids, one or more non-cationic lipids, one or more cholesterol-based lipids and one or more PEG-modified lipids; or
(b) a cholesterol-derived cationic lipid, a non-cationic lipid, and a PEG-modified lipid,
wherein the liposome has a size of 10 nm to 120 nm,
for use for inducing immune tolerance to the self-antigen involved in an auto-immune disease, allergen, or replacement protein used to treat a protein deficiency, in a subject in need thereof.

2. An mRNA comprising a 5' UTR, a coding region, and a 3' UTR, wherein:
the nucleotides of the mRNA are unmodified or modified;
the 5' UTR comprises at least one nucleic acid sequence for liver-specific expression that is a nucleic acid sequence from the 5' UTR of RM1 mRNA (Orosomucoid 1), HPXmRNA (Hemopexin), FGA mRNA (Fibrinogen alpha chain), CYP2E1 mRNA (cytochrome P450 2E1), C3 mRNA (complement component 3), APOA2 mRNA (Apolipoprotein A-II), ALB mRNA (Albumin), or AGXT mRNA (Alanine-glyoxylate aminotransferase); and
the coding region encodes a self-antigen involved in an auto-immune disease, an allergen, or a replacement protein used to treat a protein deficiency;
wherein the mRNA is encapsulated in a liposome which is delivered to the liver of a subject, and the liposome comprises:
(a) one or more cationic lipids, one or more non-cationic lipids, one or more cholesterol-based lipids and one or more PEG-modified lipids; or
(b) a cholesterol-derived cationic lipid, a non-cationic lipid, and a PEG-modified lipid,
wherein the liposome has a size of 10 nm to 120 nm,
for use for inducing immune tolerance to the self-antigen involved in an auto-immune disease, allergen, or replacement protein used to treat a protein deficiency, in a subject in need thereof.

3. The mRNA for use according to claim 1 or 2, wherein the mRNA encoding the self-antigen involved in an auto-immune disease, allergen, or replacement protein used to treat a protein deficiency is the only therapeutic agent for inducing immune tolerance to the self-antigen involved in an auto-immune disease, allergen, or replacement protein used to treat a protein deficiency, that is administered to the subject.

4. The mRNA for use according to any one of claims 1-3, wherein:
the at least one nucleic acid sequence for preventing expression and/or inducing degradation of the mRNA in a haematopoietic cell comprises one or more binding sites for miR-142-3p and/or miR-142-5p, optionally wherein the haematopoietic cell is an antigen-presenting cell; and/or
the at least one nucleic acid sequence for liver-specific expression is a nucleic acid sequence from the 5' UTR of FGA mRNA, C3 mRNA, or CYP2E1 mRNA.

5. The mRNA for use according to any one of claims 1-4, wherein the mRNA does not comprise a binding site for a liver-specific miRNA.

6. The mRNA for use according to claim 5, wherein the liver-specific miRNA is one or more of miR-122, miR-29, miR-33a/b, miR-34a, miR-92a, miR-92, miR-103, miR-107, miR-143, miR-335, and miR-483.

7. The mRNA for use according to any one of the preceding claims, wherein:
(a) the one or more cationic lipids are selected from the group consisting of DOTAP (1,2-dioleyl-3-trimethylammonium propane), DODAP (1,2-dioleyl-3-dimethylammonium propane), DOTMA (1,2-di-0-octadecenyl-3-trimethylammonium propane), DLinKC2DMA, DLin-KC2-DM, C12-200, cKK-E12 (3,6-bis(4-(bis(2-hydroxydodecyl)amino)butyl) piperazine-2,5-dione), HGT5000, HGT5001, HGT4003, ICE, OF-02, and combinations thereof;
(b) the one or more non-cationic lipids are selected from DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine), DPPE (1,2-dipalmitoyl-sn-glycero-3-phosphoethanolamine), DMPE (1,2-dimyristoyl-sn-glycero-3-phosphoethanolamine), DOPG (1,2-dioleoyl-sn-glycero-3-phospho-(1'-rac-glycerol)), and combinations thereof;
(c) the one or more cholesterol-based lipids is cholesterol or PEGylated cholesterol; and/or
(d) the one or more PEG-modified lipids comprise a poly(ethylene) glycol chain of up to 5 kDa in length covalently attached to a lipid with alkyl chain(s) of C₆-C₂₀ length.

8. The mRNA for use according to any one of the preceding claims, wherein:
(a) the cationic lipid constitutes about 30-60% of the liposome by molar ratio, optionally wherein the cationic lipid constitutes about 30%, 40%, 50%, or 60% of the liposome by molar ratio;
(b) the ratio of cationic lipids:non-cationic lipids:cholesterol lipids:PEGylated lipids is approximately 40:30:20:10 by molar ratio;
(c) the ratio of cationic lipids:non-cationic lipids:cholesterol lipids:PEGylated lipids is approximately 40:30:25:5 by molar ratio;
(d) the ratio of cationic lipids:non-cationic lipids:cholesterol lipids:PEGylated lipids is approximately 40:32:25:3 by molar ratio;
(e) the ratio of cationic lipids:non-cationic lipids:cholesterol lipids:PEGylated lipids is approximately 50:25:20:5 by molar ratio; and/or
(f) the liposome comprises cKK-E12, C12-200, OF-02, HGT4003, HGT5001, HGT5000, DLinKC2DMA, DODAP or DODMA as the cationic lipid, DOPE as the non-cationic lipid, cholesterol as the cholesterol-based lipid, and DMG-PEG2K as the PEG-modified lipid; optionally wherein the liposome comprises cKK-E12, DOPE, cholesterol and DMG-PEG2K.

9. The mRNA for use according to any one of claims 1-6, wherein the liposome comprises ICE, DOPE and DMG-PEG2K.

10. The mRNA for use according to any one of the preceding claims, wherein the liposome has a size of about 80 nm to 120 nm, optionally wherein the liposome has a size of about 100 nm or less than 100 nm.

11. The mRNA for use according to any one of the preceding claims, wherein the coding region encodes a self-antigen listed in Table 1.

12. The mRNA for use according to any one of the preceding claims, wherein:
(a) coding region encodes a peptide, polypeptide, or protein that is proinsulin; or
(b) coding region encodes a peptide, polypeptide, or protein that is tTG or ACT1.

13. The mRNA for use according to any one of claims 1-10, wherein the coding region encodes a replacement protein for treatment of the protein deficiency in the subject.

14. The mRNA for use according to claim 13, wherein:
(a) the protein deficiency and the corresponding replacement protein are selected from Table 2; or
(b) the protein deficiency is selected from haemophilia A or B, a lysosomal storage disorder, a metabolic disorder and an α-antitrypsin deficiency, optionally wherein:
(i) the protein deficiency is a metabolic disorder, optionally wherein the metabolic disorder and the corresponding replacement protein are selected from Table 3; or
(ii) the protein deficiency is haemophilia A and the coding region encodes a peptide, polypeptide, or protein that is Factor VIII.

15. The mRNA for use according to any one of claims 1-10, wherein the coding region encodes a peptide, polypeptide, or protein that is an allergen listed in Table 4.
